(19) Europäisches Patentamt
European Patent Office
Office européen des brevets

(11) EP 2 285 282 B1

(12) EUROPEAN PATENT SPECIFICATION

(45) Date of publication and mention of the grant of the patent:
**10.05.2017 Bulletin 2017/19**

(21) Application number: **09724958.5**

(22) Date of filing: **18.03.2009**

(51) Int Cl.:
***A61B 5/00*** *(2006.01)* *A61B 6/00* *(2006.01)*

(86) International application number:
**PCT/US2009/001694**

(87) International publication number:
**WO 2009/120281 (01.10.2009 Gazette 2009/40)**

(54) **SPATIAL ORIENTATION METHOD OF AN IMMOBILIZED SUBJECT**

RÄUMLICHES ORIENTIERUNGSVERFAHREN EINER IMMOBILISIERTEN PERSON

PROCÉDÉ D ORIENTATION SPATIALE D UN SUJET IMMOBILISÉ

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **24.03.2008 US 38789**
**22.08.2008 US 196300**
**08.09.2008 US 94997**
**16.01.2009 US 354830**
**13.03.2009 US 381599**

(43) Date of publication of application:
**23.02.2011 Bulletin 2011/08**

(73) Proprietor: **Bruker BioSpin Corporation**
**Billerica, MA 01821-3991 (US)**

(72) Inventors:
- **FEKE, Gilbert**
**Durham**
**CT 06422 (US)**
- **PAPINENI, Rao**
**Branford**
**CT 06405 (US)**
- **CHEN, Shoupu**
**Rochester**
**NY 14623 (US)**
- **WOOD, Douglas**
**North Haven**
**CT 06473 (US)**
- **MCLAUGHLIN, William**
**Guilford**
**CT 06437 (US)**

(74) Representative: **Wagner, Karl H.**
**Wagner & Geyer**
**Gewürzmühlstrasse 5**
**80538 Munich (DE)**

(56) References cited:
**EP-A- 1 304 070**
**WO-A-2004/089204**
**US-A1- 2003 063 788**
**US-A1- 2005 080 332**
**US-A1- 2005 085 710**
**US-A1- 2005 147 325**
**US-A1- 2007 015 991**
**US-A1- 2007 153 969**
**US-A1- 2007 237 290**
**US-A1- 2007 291 895**
**US-A1- 2007 297 566**

EP 2 285 282 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### FIELD OF THE INVENTION

The invention relates generally to the field of imaging systems, and more particularly to multi-modal imaging of living subjects. More specifically, the invention relates to (A) adjusting the physical, spatial orientation of an immobilized subject in a multi-modal imaging system so as substantially to reproduce or match the physical, spatial orientation of a reference subject, wherein the reference subject is either (a) the same or (b) a different subject, either (1) during a prior imaging session for a later imaging session, or, in the case where a plurality of subjects is imaged in one imaging session, (2) during a contemporaneous imaging session; and (B) adjusting the virtual, spatial orientation of an immobilized subject in a set of multi-modal images.

### BACKGROUND OF THE INVENTION

Electronic imaging systems are well known for enabling molecular imaging. An exemplary electronic imaging system 10, shown in Figure 1 and diagrammatically illustrated in Figure 2, is the KODAK Image Station 2000MM Multi-modal Imaging System. System 10 includes a light source 12, an optical compartment 14 which can include a mirror 16, a lens and camera system 18, and a communication and computer control system 20 which can include a display device 22, for example, a computer monitor. Lens and camera system 18 can include an emission filter wheel for fluorescent imaging. Light source 12 can include an excitation filter selector for fluorescent excitation or bright field color imaging. In operation, an image of an object is captured using lens and camera system 18. System 18 converts the light image into an electronic image, which can be digitized. The digitized image can be displayed on the display device, stored in memory, transmitted to a remote location, processed to enhance the image, and/or used to print a permanent copy of the image.

A system for creating a tomographic image is disclosed in U.S. Patent Application Publication 2007/0238957 by Yared. A system is disclosed by Yared that includes an X-ray source, an X-ray detector, a light source, and a light detector, wherein these components are radially disposed about an imaging chamber. More specifically these components are mounted on a gantry that is rotatable about the imaging chamber. The system includes code comprising instructions to create a three dimensional optical absorption map of a target volume based at least in part on the detected X-ray radiation and to use in the optical absorption map in optical tomographic reconstruction to create the tomographic image. In addition or alternatively, Yared's system includes code comprising instructions to create a surface model of at least a portion of the object based at least in part on the detected X-ray radiation and to use the surface model in optical tomographic reconstruction to create the tomographic image. The system further may include code comprising instructions to create a three-dimensional anatomical data set using the detected X-ray radiation and to register the anatomical data set with the tomographic image to create a composite image.

US Patent No. 6,868,172 (Boland et al) is directed to a method for registering images in radiography applications.

### SUMMARY OF THE INVENTION

The present invention provides an improved, simpler solution for combining anatomical imaging with molecular imaging. The invention does not require a complex tomographic imaging system, nor radial disposition of an X-ray source, an X-ray detector, a light source, and a light detector about an imaging chamber, nor mounting of these components on a gantry rotatable about the imaging chamber. Furthermore, the present invention typically is not necessary for tomographic imaging systems wherein the spatial orientation of the subject does not affect the resulting data since in tomography the spatial orientation is not projected into a two-dimensional planar representation but instead may float in a three-dimensional representation. However, the technical features of the invention relating to a region of interest template would be useful in a tomographic system for longitudinal studies or sequentially different subject studies, in which case the region of interest would the three-dimensional. In comparison, the present invention is advantageous for planar imaging systems because in such systems the spatial orientation, such as the cranio-caudal rotation angle of the subject, may affect the resulting data. Furthermore, the present invention is more generally applied to all modes of molecular imaging, including optical imaging and imaging of ionizing radiation, such as from radio-isotopic probes, by means of a phosphor screen.

Applicants have recognized a need for substantially reproducing the spatial orientation of an immobilized subject, such as a small animal, in a multi-modal imaging system used to take time-spaced images of the subject. For example, in known imaging methods a small animal used in a longitudinal multi-modal molecular imaging study typically has been loaded into an animal chamber, such as a right circular cylindrical tube, for a first time and imaged for the first time. The animal then is unloaded from the animal chamber, later loaded back into the animal chamber for at least a second time, and imaged for at least the second time. Thus, a first-time set of multi-modal molecular images and at least a second-time set of multi-modal molecular images are provided. If the physical, spatial orientation of the animal, for example the cranio-caudal rotation angle of the animal, with respect to the tube and/or the imaging system is different between the first time and the at least second time, then the at least second-time set of multi-modal molecular images may be affected by the difference in the physical, spatial orientation compared to the first-time set of multi-modal molecular images. This difference may result in artifacts, such as relative attenuation or enhancement of a molecular signal, upon comparison to the first-time set of multi-modal molecular images.

Figure 33A illustrates an example of relative attenuation or enhancement of fluorescence molecular signals for different cranio-caudal rotation angles, typical for known methods. A graph of the fluorescence intensity vs. cranio-caudal rotation angle is provided for several organs in the body of a mouse, including the bladder, kidney, stomach, and intestines. Figure 33B illustrates an example of relative attenuation or enhancement of radio-isotopic molecular signals for different cranio-caudal rotation angles, also typical for known methods. A graph of radio-isotopic signal vs. cranio-caudal rotation angle is provided for simulated radionuclide-labeled tissue in the body of a mouse. Continuing with regard to the example of the known method described above, if the physical, spatial orientation of the animal is different between the first time and the at least second time, then the first-time set of multi-modal molecular images and the at least second-time set of multi-modal molecular images cannot be precisely co-registered. Lack of co-registration can degrade the quantitation provided by a simple regions-of-interest analysis wherein a single regions-of-interest template is applied to both the first-time set of multi-modal molecular images and the at least second-time set of multi-modal molecular images. Similar problems arise with known methods when a plurality of animals is loaded serially into a field of view.

For example, when a plurality of small animals is used in known methods for a multi-modal molecular imaging study, the animals are loaded into animal chambers, such as right circular cylindrical tubes, whereby the loading may be performed serially at a given spatial location within the field of view of the multi-modal imaging system, or may be performed in parallel across a plurality of spatial locations in the field of view of the multi-modal imaging system. In such an example, the physical, spatial orientations of the animals, for example the cranio-caudal rotation angles, may differ among the plurality of animals. As a result, each set of multi-modal molecular images for each animal may be affected by the difference in the physical, spatial orientation, thereby resulting in artifacts, such as relative attenuation or enhancement of a molecular signal, in one set of multi-modal molecular images compared to another set of multi-modal molecular images.

If small animals are loaded in parallel across a plurality of spatial locations in the field of view in known methods of using the multi-modal imaging system, then regions of interest defined for one animal may not be spatially translatable to the other animals by the simple difference between the spatial locations of the animals due to differences in the physical, spatial orientations, for example the cranio-caudal rotation angles, of the animals at their locations. As a result, degraded quantitation may be provided by a simple regions-of-interest analysis wherein an array-like regions-of-interest template (i.e., multiple copies of a set of regions of interest across the field of view) is applied to the set of multi-modal molecular images.

The problems of known methods caused by different physical, spatial orientations of test animals during different imaging sessions are solved or substantially reduced by implementation of the method and apparatus of the present invention as defined in claim 1 and in claim 5. A first embodiment of the inventive method substantially reproduces the physical, spatial orientation of an immobilized subject in an X-ray imaging system including a computer, from a prior imaging session for a later imaging session. The method includes steps of: performing a physical, spatial orientation of the immobilized subject for a first time in the imaging system; using the computer, acquiring an X-ray anatomical image of the immobilized subject for the first time in the imaging system; performing a test physical, spatial orientation of the immobilized subject for a next time in the imaging system; using the computer, acquiring a test X-ray anatomical image of the immobilized subject for the next time in the imaging system; using the computer, comparing the test X-ray anatomical image for the next time and the X-ray anatomical image for the first time, including a calculation of the difference therebetween; physically, spatially reorienting the immobilized subject to improve the comparison, if the comparison is not satisfactory to demonstrate reproduction of the physical, spatial orientation for the first time; repeating the steps of performing a test physical, spatial orientation, acquiring a test X-ray anatomical image, comparing the test X-ray anatomical image and physically, spatially reorienting the immobilized subject until the comparison is satisfied; and using the computer, acquiring an X-ray anatomical image of the immobilized subject for the next time in the multi-modal imaging system.

A second embodiment of the inventive method reproduces the physical, spatial orientation of an immobilized subject in an X-ray imaging system including a computer from one subject for another subject. The method includes steps of: performing a physical, spatial orientation of a first immobilized subject in the multi-modal imaging system, using the computer, acquiring an X-ray anatomical image of the first immobilized subject in the imaging system; performing a physical, spatial orientation of a next immobilized subject in the imaging system; using the computer, acquiring a test X-ray anatomical image of the next immobilized subject in the imaging system; using the computer, comparing the test X-ray anatomical image of the next immobilized subject and the X-ray anatomical image of the first immobilized subject, including a calculation of the difference therebetween; physically, spatially reorienting the next immobilized subject to improve the comparison, if the comparison is not satisfactory to demonstrate reproduction of the physical, spatial orientation of the first immobilized subject; repeating the steps of performing a physical, spatial orientation of a next immobilized subject, acquiring a test X-ray anatomical image, comparing and physically, spatially reorienting until the comparison is satisfied; and acquiring an X-ray anatomical image of the next immobilized subject in the multi-modal imaging system.

A third embodiment of the inventive method reproduces the physical, spatial orientation of a plurality of immobilized subjects in an X-ray imaging system including a computer. The method includes steps of: performing a test physical, spatial orientation of the plurality of immobilized subjects in the imaging system; using the computer, acquiring a test X-ray anatomical image of the plurality of immobilized subjects in the imaging system; using the computer, dividing the test X-ray anatomical image of the plurality of immobilized subjects into X-ray anatomical image sections corresponding to each subject; using the computer, comparing the test X-ray anatomical image section corresponding to each subject to the test X-ray anatomical image section of a reference subject selected from the test X-ray anatomical images of the plurality of immobilized subjects, including a calculation of the difference between X-ray anatomical image sections; physically, spatially reorienting each immobilized subject, except the reference subject to improve the comparison, if the comparison is not satisfactory to demonstrate reproduction of the reference subject; repeating the steps of performing, acquiring, dividing, comparing and physically, spatially reorienting until comparison is satisfied; and using the computer, acquiring an X-ray anatomical image of the plurality of immobilized subjects in the multi-modal imaging system.

A fourth embodiment of the inventive method registers and analyzes multi-modal molecular images of an immobilized subject in a multi-modal imaging system including a computer, for a plurality of times. The method includes steps of; performing a physical, spatial orientation of the immobilized subject for a first time in the multi-modal imaging system; using the computer, acquiring an X-ray anatomical image of the immobilized subject for the first time in the multi-modal imaging system; using the computer, acquiring a set of multi-modal molecular images of the immobilized subject for the first time using a set of modes of the multi-modal imaging system, wherein the set of multi-modal molecular images may include at least one image acquired using at least one mode included in the set of modes; using the computer, creating regions-of-interest templates identifying the regions of interest in the set of multi-modal molecular images for the first time; using the computer, applying the regions-of-interest templates to measure the molecular signals in the regions of interest in the set of multi-modal molecular images of the immobilized subject for the first time; using the computer, acquiring an X-ray anatomical image of the immobilized subject for a next time in the multi-modal imaging system; using the computer, acquiring a set of multi-modal molecular images of the immobilized subject for the next time using a set of modes of the multi-modal imaging system, wherein the set of multi-modal molecular images may include at least one image acquired using at least one mode included in the set of modes; using the computer, comparing the X-ray anatomical image for the next time and the X-ray anatomical image for the first time, including a calculation of the difference between; using the computer, registering the X-ray anatomical image for the next time to the X-ray anatomical image for the first time by virtually, spatially reorienting the X-ray anatomical image for the next time to improve the comparison, if the comparison is not satisfactory to demonstrate registration to the X-ray anatomical image for the first time; using the computer, registering the set of multi-modal molecular images for the next time to the set of multi-modal molecular images for the first time, by applying the same spatial transformation parameters as were applied to the X-ray anatomical image for the next time to the set of multi-modal molecular images for the next time; and using the computer, applying the regions-of-interest templates to measure the molecular signals in the regions of interest in the set of multi-modal molecular images of the immobilized subject for the next time.

A fifth embodiment of the inventive method reproduces the physical, spatial orientation of one or more immobilized subjects in an X-ray imaging system including a computer. The method includes steps of: performing a reference series of physical, spatial orientations of the immobilized subject(s) in the imaging system; using the computer, acquiring a reference X-ray anatomical image of each subject for each physical, spatial orientation of the reference series; using the computer, using the reference X-ray anatomical images to calculate a first plurality of correspondences for achieving desired physical, spatial orientations of the subjects of the reference series for X-ray images; performing a test series of physical, spatial orientations of immobilized subject(s) in the imaging system; using the computer, acquiring a test X-ray anatomical image of the immobilized subject(s) for each physical, spatial orientation of the test series; and using the computer, using the test X-ray anatomical images to calculate a second plurality of correspondences for selecting reproduced desired physical, spatial orientations of the subjects of the test series for X-ray images.

A sixth embodiment of the inventive method adjusts a physical, spatial orientation of at least one immobilized subject in an X-ray imaging system including a computer, so as substantially to reproduce the physical, spatial orientation of another, reference immobilized subject. The method includes steps of: performing a physical, spatial orientation of the reference subject; using the computer, acquiring an X-ray anatomical image of the reference subject; performing a physical, spatial orientation of the at least one subject; using the computer, acquiring an X-ray anatomical image of the at least one subject; using the computer, analyzing the combination of the X-ray anatomical image of the reference subject and the X-ray anatomical image of the at least one subject; and following the analyzing, physically, spatially reorienting the at least one subject so as substantially to reproduce the physical, spatial orientation of the reference subject.

## BRIEF DESCRIPTION OF THE DRAWINGS

The foregoing and other objects, features, and advantages of the invention will be apparent from the following more particular description of the embodiments of the invention, as illustrated in the accompanying drawings. The elements of the drawings are not necessarily to scale relative to each other.

FIG. 1 shows a perspective view of an exemplary electronic imaging system.
FIG. 2 shows a diagrammatic view of the electronic imaging system of FIG. 1.
FIG. 3A shows a diagrammatic side view of an imaging system useful in accordance with the present invention.
FIG. 3B shows a diagrammatic front view of the imaging system of FIG. 3A.
FIG. 4 shows a perspective view of the imaging system of FIGS. 3A and 3B.
FIG. 5A shows a diagrammatic partial view of a mouse in a sample chamber on a sample object stage of the imaging system of FIGS. 3A and 3B when either (a) a first-time X-ray anatomical image is acquired in accordance with the present invention, or (b) a next-time X-ray anatomical image is virtually, spatially reoriented in accordance with the invention.
FIG. 5B shows a diagrammatic partial view of the mouse in the sample chamber on the sample object stage of the imaging system of FIGS. 3A and 3B when a first-time set of multi-modal molecular images is acquired in accordance with the present invention.
FIG. 6 shows a workflow diagram in accordance with a method of the present invention.
FIG. 7A shows a diagrammatic partial view of the mouse in the sample chamber on the sample object stage of the imaging system of FIGS. 3A and 3B when a next-time test X-ray anatomical image is acquired in accordance with the present invention.
FIG. 7B shows a diagrammatic partial view of the mouse in the sample chamber on the sample object stage of the imaging system of FIGS. 3A and 3B when a next-time X-ray anatomical image after physical, spatial reorientation is acquired in accordance with the present invention.
FIG. 7C shows a diagrammatic partial view of the mouse in the sample chamber on the sample object stage of the imaging system of FIGS. 3A and 3B when a next-time set of multi-modal molecular images (a) is acquired in accordance with the present invention or (b) has been virtually, spatially reoriented in accordance with the present invention.
FIG. 8 shows a workflow diagram in accordance with a method of the present invention.
FIG. 9 shows a flow diagram of statistical method used in step 260 of FIG. 8 in accordance with the present invention.
FIG. 10 shows a flow diagram of another embodiment of a method used in step 260 of FIG. 8 in accordance with the present invention.
FIG. 11 shows a diagrammatic partial view of a plurality of subject mice in a corresponding plurality of sample chambers on the sample object stage of the imaging system of FIGS. 3A and 3B being imaged serially in accordance with the present invention.
FIG. 12 shows a workflow diagram in accordance with a method of the present invention.
FIG. 13 shows a workflow diagram in accordance with a method of the present invention.
FIG. 14 shows a flow diagram of statistical method used in step 660 of FIG. 13 in accordance with the present invention.
FIG. 15 shows a flow diagram of another embodiment of a method used in step 660 of FIG. 13 in accordance with the present invention.
FIG. 16 shows a diagrammatic partial view of a plurality of subject mice in a corresponding plurality of sample chambers on the sample object stage of the imaging system of FIGS. 3A and 3B being imaged in parallel in accordance with the present invention.
FIG. 17 shows several multi-subject images acquired in accordance with the present invention.
FIG. 18 shows a workflow diagram in accordance with a method of the present invention.
FIG. 19 shows a flow diagram of statistical method used in step 1030 of FIG. 18 in accordance with the present invention.
FIG. 20 shows a flow diagram of another embodiment of a method used in step 1030 of FIG. 18 in accordance with the present invention.
FIG. 21 is a graphical representation of the first-time molecular signals measured in regions of interest in accordance with the present invention.
FIG. 22 shows a workflow diagram in accordance with a method of the present invention.
FIG. 23 is a graphical representation of the next-time molecular signals measured in regions of interest in accordance with the present invention.

FIG. 24 is a graphical representation of the next-time molecular signals measured in regions of interest from a virtually, spatially reoriented image in accordance with the present invention.

FIG. 25 shows a workflow diagram in accordance with a method of the present invention.

FIG. 26 shows a flow diagram of statistical method used in step 3320 of FIG. 31 in accordance with the present invention.

FIG. 27 shows a flow diagram of another embodiment of a method used in step 3320 of FIG. 25 in accordance with the present invention.

FIG. 28 shows use of an exogenous X-ray anatomical image contrast agent to provide contrast with soft tissue.

FIG. 29 shows use of an exogenous X-ray anatomical image contrast device to provide contrast with soft tissue.

FIG. 30 shows an alternative flow diagram of statistical method used in step 260 of FIG. 8 in accordance with the present invention.

FIG. 31 shows an alternative flow diagram of statistical method used in step 660 of FIG. 13 in accordance with the present invention; and

FIG. 32 shows an alternative flow diagram of statistical method used in step 1030 of FIG. 18 in accordance with the present invention.

FIG. 33A shows a graph of fluorescence intensity vs. cranio-caudal rotation angle for several organs in the body of a mouse.

FIG. 33B shows a graph of radio-isotopic signal vs. cranio-caudal rotation angle for simulated radionuclide-labeled tissue in the body of a mouse.

FIG. 34 shows a series of reference X-ray images of a mouse incrementally rotated through various cranio-caudal rotation angles.

FIG. 35A shows the series of reference X-ray images of a mouse of FIG. 34 with a gradient filter in applied, and the location of line profiles.

FIG. 35B shows the series of reference X-ray images of a mouse of FIG. 34 with a different gradient filter applied which is opposite the gradient filter applied in FIG.35A, and the location of line profiles.

FIG. 36 shows line profiles of the series of images shown in FIGS. 35A and B, wherein the abscissae of the line profiles of the series of images from FIG. 35B have been reversed.

FIG. 37 shows a graph of the maximum of the cross-correlation of the line profiles shown in FIG. 36 vs. cranio-caudal rotation angle.

FIG. 38A shows the series of test X-ray images of a mouse of FIG. 34, shifted one image to the right, with a gradient filter in applied, and the location of line profiles.

FIG. 38B shows the series of test X-ray images of a mouse of FIG. 34, shifted one image to the right, with a different gradient filter applied which is opposite the gradient filter applied in FIG. 38A, and the location of line profiles.

FIG. 39 shows line profiles of the series of images shown in FIGS. 3845A and B, wherein the abscissae of the line profiles of the series of images from FIG. 38B have been reversed.

FIG. 40 shows a graph of the maximum of the cross-correlation of the line profiles shown in FIG. 39 vs. cranio-caudal rotation angle.

FIGS. 41A and 41B show a workflow diagram in accordance with a method of the present invention.

FIGS. 42A and 42B show a workflow diagram in accordance with another method of the present invention.

FIG. 43 shows a series of reference X-ray anatomical images of a mouse incrementally rotated through various cranio-caudal rotation angles.

FIG. 44 shows X-ray density images corresponding to the images of FIG. 43.

FIG. 45 shows a series of binary threshold images corresponding to the images of FIG. 44.

FIG. 46 shows a series of gradient images corresponding to the images of FIG. 43.

FIG. 47 shows the images of FIG. 45 imagewise multiplied by the images of FIG. 46.

FIG. 48 shows the imagewise absolute value of the images of Figure 47.

FIG. 49 shows a graph of normalized absolute values versus orientation of the subject.

FIGS. 50A and 50B show a work flow diagram for producing the images of FIGS. 43 to 49.

## DETAILED DESCRIPTION OF THE INVENTION

The invention has been described in detail with particular reference to certain preferred embodiments thereof, but it will be understood that variations and modifications can be effected within the scope of the invention. The following is a detailed description of the preferred embodiments of the invention, reference being made to the drawings in which the same reference numerals identify the same elements of structure in each of the several figures.

Reference is made to commonly assigned, copending provisional U.S. Patent Application Serial No. 61/131,948 filed June 13, 2008 by Feke et al., and entitled TORSIONAL SUPPORT APPARATUS FOR CRANIOCAUDAL ROTATION OF ANIMALS.

As shown in Figure 3A, imaging system 100 includes an X-ray source 102 and a sample object stage 104. Imaging system 100 further comprises epi-illumination, for example, using fiber optics 106, which directs conditioned light (of appropriate wavelength and divergence) toward sample object stage 104 to provide bright-field or fluorescent imaging. Sample object stage 104 is disposed within a sample environment 108, which allows access to the object being imaged. Preferably, a radiographic phosphor screen, not shown, is positioned between stage 104 and camera and lens system 18 to transduce projected X-rays into visible light for capture by system 18.

Commonly assigned U.S. Patent 6,444,988 by Vizard, entitled: ELECTRONIC IMAGING SCREEN WITH OPTICAL INTERFERENCE COATING discloses such a screen. The screen may be movable into and out of the X-ray beam, as disclosed in the previously mentioned U.S. Patent Applications Serial No. 11/221,530 and 12/354,830. Preferably, sample environment 108 is light-tight and fitted with light-locked gas ports for environmental control. Such environmental control might be desirable for controlled X-ray imaging or for support of particular specimens. Environmental control enables practical X-ray contrast below 8 KeV (air absorption) and aids in life support for biological specimens. Imaging system 100 can include an access means or member 110 to provide convenient, safe and light-tight access to sample environment 108. Access means are well known to those skilled in the art and can include a door, opening, labyrinth, and the like. Additionally, sample environment 108 is preferably adapted to provide atmospheric control for sample maintenance or soft X-ray transmission (e.g., temperature/humidity/alternative gases and the like). The inventions disclosed in the previously mentioned U.S. Patent Applications of Harder et al. and Vizard et al., are examples of electronic imaging systems capable of multi-modal imaging that are useful in accordance with the present invention.

Figures 5A and 5B show diagrammatic partial views of a cylindrical sample chamber or tube 118 and a sample object stage 104 of the imaging system 100 of Figures 3A and 3B. A subject mouse 112 is administered immobilizing anesthesia through a respiratory device 114 connected to an outside source via a tube 116 that enters the chamber 118 via the light-locked gas ports. A first-time X-ray anatomical image 120 of Figure 5A and a first-time set of multi-modal molecular images 122 of Figure 5B are acquired of the immobilized subject mouse 112.

As shown in the flow chart of Figure 6, a first-time physical, spatial orientation of immobilized subject 112 is performed at step 200, followed by acquisition of first-time X-ray anatomical image 120 at step 210 and first-time set of multi-modal molecular images 122 at step 220. A set of modes of multi-modal imaging system 100 is used at step 220. These modes may include at least one of bright-field mode, dark-field mode, and radio isotopic mode. The first set of multi-modal molecular images may include at least one image acquired using at least one mode included in the first set of modes. First-time set of multi-modal molecular images 122 is acquired using the same camera conditions, such as zoom and focus, as the first X-ray anatomical image 120. Thus, co-registration between image 120 and images 122 is achieved by virtue of the fact the physical, spatial orientation of subject 112 does not change between capture of images 122 and image 120.

Now referring to Figure 7A, a next-time test X-ray anatomical image 124 is acquired of immobilized subject 112. The next-time test X-ray anatomical image 124 may be an image taken after the subject has been removed from and then returned to chamber 118 and/or system 100. Image 124 may be an image of subject 112 taken after a long period of time, for example 24 hours, since image 120 was captured. Or, image 124 may be taken after some occurrence has caused subject 112 to change its position hence changing its physical, spatial orientation with respect to chamber 118 and/or system 100.

Figure 7B illustrates the acquisition a next-time X-ray anatomical image 130 of subject 112 in sample tube 118 of system 100 after physical, spatial reorientation of the subject has been performed. The physical, spatial reorientation may be performed by manual means, or robotic means controlled by the communication and computer control system 20, for example via a rotational mechanism 126 and an X-Y translation mechanism 128 as shown in Figures 5A and B; and 7A, B and C.

Figure 7C illustrates the acquisition a next-time set of multi-modal molecular images 132 of subject 112 in sample tube 118 of system 100 after either (a) physical, spatial reorientation or (b) virtual, spatial reorientation of the subject has been performed.

As shown in the workflow chart in Figure 8, a next-time test physical, spatial orientation of immobilized subject 112 in system 100 is performed at step 230, followed by acquisition of image 124 at step 240, then comparison of image 124 to image 120 against matching criteria designed to match the next-time test physical, spatial orientation to the first-time physical, spatial orientation at step 250. The comparison may be made by a calculation of the difference between image 124 and image 120, or the comparison may be according to the digital image processing method for image registration described in commonly assigned U.S. Patent 7,263,243 of Chen et al. The comparison may be manual or automated. The comparison may be performed based on endogenous X-ray anatomical image contrast, such as from skeletal and/or soft tissue, or exogenous X-ray anatomical image contrast, such as injected, implanted, and/or otherwise attached radio-opaque imaging agents or devices. If the analysis of the output of the comparison at step 250 is satisfactory, corresponding to the "YES" branch of step 250, the next-time set of multi-modal molecular images 132 may be acquired in step 270. The set of imaging modes may include at least one of bright-field mode, dark-field mode, and radio isotopic mode, and the next-time set of multi-modal molecular images 132 may include at least one image acquired using at least one mode included in the set of modes. The next-time set of multi-modal molecular images 132 may be co-registered with the next-time test X-ray anatomical image 124, thereby resulting in the next-time set of multi-modal molecular images 132 being additionally co-registered with the first-time set of multi-modal molecular images 122 and the first-time X-ray anatomical image 120. If the output of the comparison is unsatisfactory, corresponding to the "NO" branch of step 250, immobilized subject 112 is physically, spatially reoriented in step 260 to improve the comparison. The physical, spatial reorientation may be determined by spatially mapping the results determined from the digital image processing method for image registration described in the previously mentioned U.S. Patent of Chen et al., to the subject 112, or the physical, spatial reorientation may be made by trial-and-error. The physical, spatial reorientation may be performed by manual means, or by robotic means controlled by the communication and computer control system 20 as previously discussed. Steps 240, 250, and 260 are repeated until the output of the comparison is satisfactory, thereby corresponding to the "YES" branch of step 250, and proceeding accordingly as described above.

In an embodiment the following statistical method is used for step 260 of Figure 8. Referring to the workflow shown in Figure 9, a physical, spatial reorientation of subject 112 to achieve a match between image 120 and image 124 is accomplished by steps of applying vector quantization to image 120 and image 124; converting these X-ray anatomical images to vectorized X-ray anatomical images having corresponding local intensity information as derived respectively from the X-ray anatomical images at step 300; obtaining a joint statistical representation of the X-ray anatomical images by employing the vectorized X-ray anatomical images at step 310; computing a cost function using the joint statistical representation of the X-ray anatomical images at step 320; selecting a reference image (the first-time X-ray anatomical image) from the plurality of X-ray anatomical images at step 330; and evaluating the cost function at step 340. If the predetermined cost function criterion is unsatisfied as shown in the "NO" branch of step 340, the subject is physically, spatially reoriented according to its virtual, spatial correspondence to the reference image at step 350, and flow goes back to step 300 of Figure 9. If the predetermined cost function criterion is satisfied as shown in the "YES" branch of step 340, the physical, spatial reorientation is complete and the next-time set of multi-modal molecular images can be acquired at step 270 of Figure 8. Persons skilled in the art understand that step 260 can be implemented with or without vector quantization in step 300.

In another embodiment the following method is used for step 260 of Figure 8. Referring to the workflow shown in Figure 30, a physical, spatial reorientation of subject 112 to achieve a match between image 120 and image 124 is accomplished by steps of selecting a reference image (image 120) at step 4000; applying an image registration algorithm (e.g. described in the previously mentioned U.S. Patent of Chen et al.) to image 120 and image 124 at step 4010; obtaining a minimal cost function value from the image registration process at step 4020; obtaining a virtual spatial displacement map corresponding to the minimal cost function from the image registration process at step 4030; and evaluating the cost function at step 4040. If the predetermined cost function criterion is unsatisfied as shown in the "NO" branch of step 4040, the subject is physically, spatially reoriented according to the virtual spatial displacement map at step 4050, and flow goes back to step 4000 of Figure 30. If the predetermined cost function criterion is satisfied as shown in the "YES" branch of step 4040, the physical, spatial reorientation is complete and the next-time set of multi-modal molecular images can be acquired at step 270 of Figure 8. The virtual spatial displacement map can be computed based on the virtual spatial transformation described by Chen et al.

In another embodiment the following method is used for step 260 of Figure 8. Referring to the work flow shown in Figure 10, image 120 is compared to image 124; a calculation of the image difference between the two X-ray anatomical images is made at step 400; and a comparison of the image difference to a null (zero) image is made at step 410. Where the comparison of the image difference to a null (zero) image is not satisfactory as shown in the "NO" branch of step 420, subject 112 is physically, spatially reoriented according to its virtual, spatial correspondence to the reference image at step 430, and the flow goes back to step 400 of Figure 10. If the comparison of the image difference to a null (zero) image is satisfactory as shown in "YES" branch of step 420, the physical, spatial reorientation is complete and the next-time set of multi-modal molecular images can be acquired at step 270 of Figure 8.

In a second embodiment of the present invention the physical, spatial reorientations of the subjects involves comparing different animals as shown in Figure 11. A plurality of small animals such as subject mice 500 a, b, c, and d are used in a multi-modal molecular imaging study and are loaded into animal chambers, such as right circular cylindrical tubes 510 a, b, c, and d, respectively, whereby the loading and imaging may be conducted serially. A first-subject X-ray anatomical image 520a of subject mouse "a" is acquired along with a first-subject set of multi-modal molecular images 530a using the multi-modal imaging system 100. As previously described the acquisition of a first-subject set of multi-modal molecular images 530a may be made using a set of modes of system 100. As seen in Figure 11, set 530a includes two multi-modal molecular images, a left image 531 a captured using a first molecular imaging mode and a right image 531b captured using a second molecular imaging mode. The set of modes may include at least one of bright-field mode, dark-field mode, and radio isotopic mode. The first-subject set of multi-modal molecular images 530a may include at least one image acquired using at least one mode included in the first set of modes. Because the first-subject set of multi-modal molecular images 530a is acquired using the same camera conditions, such as zoom and focus, as the first-subject X-ray anatomical image 520a, co-registration between the first-subject X-ray anatomical image and the first-subject set of multi-modal molecular images is achieved by virtue of the fact the physical, spatial orientation does not change between capture of images 530a and image 520a for subject mouse "a".

Now referring to the workflow shown in Figure 12, a first-subject physical spatial orientation of an immobilized subject 500a, subject mouse "a", in system 100 is performed at step 600, followed by the acquisition of a first-subject X-ray anatomical image 520a at step 610 and the acquisition of a first-subject set of multi-modal molecular images 530a of the immobilized subject 500a using a set of modes of the multi-modal imaging system 100 at step 620. The set of modes may include at least one of bright-field mode, dark-field mode, and radio isotopic mode. The first set of multi-modal molecular images may include at least one image acquired using at least one mode included in the first set of modes. Because the first-subject set of multi-modal molecular images 530a is acquired using the same camera conditions, such as zoom and focus, as the first-subject X-ray anatomical image 520a, co-registration between the images is achieved in the manner previously described. Referring again to Figure 11, the next-subject mice 500b, c, and d (subject mouse "b", subject mouse "c", and subject mouse "d") are loaded serially to a plurality of next-subject physical, spatial orientations in the field of view of system 100. A next-subject test X-ray anatomical image 525b, c, and d, respectively, is acquired for each of the next-subject mice "b", "c", and "d".

As shown in the workflow chart in Figure 13, a next-subject test physical, spatial orientation is performed for each immobilized subject mouse 500b, c, and d (subject mouse "b", subject mouse "c", and subject mouse "d") at step 630, followed by acquisition of a next-subject test X-ray anatomical image 525b, c, and d for each of the next-subject mice "b", "c" and "d", respectively, in system 100 at step 640; then comparison of the next-subject test X-ray anatomical image 525b, c, and d to image 520a against matching criteria designed to match the next-subject test physical, spatial orientation to the first-subject physical, spatial orientation at step 650. The comparison may be made by a calculation of the difference between image 525b, c, and d and image 520a, or the comparison may be according to the digital image processing method for image registration described in the U.S. Patent by Chen et al. The comparison may be manual or automated. The comparison may be performed based on endogenous X-ray anatomical image contrast, such as from skeletal and/or soft tissue, or exogenous X-ray anatomical image contrast, such as injected, implanted, and/or otherwise attached radio-opaque imaging agents or devices. If the analysis of the output of the comparison at step 650 is satisfactory, corresponding to the "YES" branch of step 650, the next-subject set of multi-modal molecular images 540b, c, and d may be acquired, step 670. As seen in Figure 11, sets 540b, c, and d each include two multi-modal molecular images, left images 541 a, 542a, and 543a captured using a first molecular imaging mode and right images 541b, 542b, and 543b captured using a second molecular imaging mode. The set of modes may include at least one of bright-field mode, dark-field mode, and radio isotopic mode. The at least next-subject set of multi-modal molecular images 540b, c, and d may include at least one image acquired using at least one mode included in the set of modes, and whereby the next-subject set of multi-modal molecular images 540b, c, and d is co-registered with the next-subject test X-ray anatomical image 525b, c, and d, thereby resulting in the next-subject set of multi-modal molecular images 540b, c, and d to be additionally co-registered with the first-subject set of multi-modal molecular images 530a and the first-subject X-ray anatomical image 520a. If the output of the comparison is unsatisfactory, corresponding to the "NO" branch of step 650, the immobilized subjects mice 500b, c, and d are physically, spatially reoriented at step 660 to improve the comparison. The reorientation may be determined by spatially mapping the results determined from the digital image processing method for image registration described in the U.S. Patent of Chen et al., to the physical subject 500b, c, and d, or the reorientation may be made by trial-and-error. The physical, spatial reorientation may be performed by manual means, or by robotic means controlled by the communication/computer control system 20 as previously discussed. Steps 640, 650, and 660 are repeated until the output of the comparison is satisfactory, as shown by images 535 b, c and d of Figure 11, thereby corresponding to the "YES" branch of step 650, and proceeding accordingly as described above.

In another embodiment the following statistical method is used for step 660 of Figure 13. Referring to the workflow shown in Figure 14, the comparison of image 520a to image 525b, c, and d is accomplished by steps of applying vector quantization to image 520a and image 525b, c, and d; converting these X-ray anatomical images to vectorized X-ray anatomical images having corresponding local intensity information as derived respectively from the X-ray anatomical images at step 700; obtaining a joint statistical representation of the X-ray anatomical images by employing the vectorized X-ray anatomical images at step 710; computing a cost function using the joint statistical representation of the X-ray anatomical images at step 720; selecting a reference image (the first-subject X-ray anatomical image) from the plurality of X-ray anatomical images at step 730; and evaluating the cost function at step 740. If the predetermined cost function criterion is unsatisfied as shown in the "NO" branch of step 740, the next subject is physically, spatially reoriented according to its virtual, spatial correspondence to the reference image at step 750, and flow goes back to step 700 of Figure 14. If the predetermined cost function criterion is satisfied as shown in the "YES" branch of step 740, the physical, spatial reorientation is complete and the next-subject set of multi-modal molecular images can be acquired at step 670 of Figure 13. Persons skilled in the art understand that step 660 can be implemented with or without vector quantization in step 700. The spatial displacement map can be computed based on the virtual spatial transformation described by Chen et al.

In yet another embodiment the following method is used for step 660 of Figure 13. Referring to the workflow shown in Figure 31, the physical, spatial reorientation of the next subject to achieve a match between image 520a and image 525b, c and d is accomplished by steps of selecting a reference image (image 520a) at step 5000; applying an image registration algorithm (e.g. described in the U.S. Patent of Chen et al.) to image 520 and image 525b, c, and d at step 5010; obtaining a minimal cost function value from the image registration process at step 5020; obtaining a virtual spatial displacement map corresponding to the minimal cost function from the image registration process at step 5030; and evaluating the cost function at step 5040. If the predetermined cost function criterion is unsatisfied as shown in the "NO" branch of step 5040, the next subject is physically, spatially reoriented according to the virtual spatial displacement map at step 5050, and flow goes back to step 5000 of Figure 31. If the predetermined cost function criterion is satisfied as shown in the "YES" branch of step 5040, the physical, spatial reorientation is complete and the next-subject set of multi-modal molecular images can be acquired at step 670 of Figure 13.

In another embodiment the following method is used for step 660 of Figure 13. Referring to the workflow shown in Figure 15, image 520a is compared to image 525 b, c and d; a calculation of the image difference between the two X-ray anatomical images is made at step 800; and a comparison of the image difference to a null (zero) image is made at step 810. Where the comparison of the image difference to a null (zero) image is not satisfactory as shown in the "NO" branch of step 820, the subject is physically, spatially reoriented according to its virtual, spatial correspondence to the reference image at step 830, and the flow goes back to step 800 of Figure 15. If the comparison of the image difference to a null (zero) image is satisfactory as shown in "YES" branch of step 820, the physical, spatial reorientation is complete and the next-subject set of multi-modal molecular images can be acquired at step 670 of Figure 13.

In yet another embodiment of the present invention the physical, spatial reorientations of the physical subjects involve comparing different animals as shown in Figure 16. A plurality of small animals such as subject mice 900a, b, c, and d are used in a multi-modal molecular imaging study and are loaded into animal chambers 910a, b, c, and d, respectively, whereby the loading and imaging may be in parallel.

As shown in the images of Figure 17 and the workflow chart in Figure 18, a multi-subject test physical, spatial orientation is performed at step 1000, followed by acquisition of a test multi-subject X-ray anatomical image 920 of the multi-subject mice "a", "b", "c" and "d" in system 100 at step 1010. Image 920 is divided into image sections 925a, b, c, and d of subject mice "a", "b", "c", and "d", respectively. The image sections 925b, c, and d of subject mice "b", "c", and "d", respectively, are compared to the image section 925a of subject mouse "a" using the matching criteria designed to match the physical, spatial orientation of the subject mice "b", "c", and "d" to the physical, spatial orientation of subject mouse "a" at step 1020. The comparison may be made by a calculation of the difference between the image section 925b, c, and d of each subject mouse "b", "c", and "d", respectively, and the image section 925a of subject mouse "a". Or the comparison may be according to the digital image processing method for image registration described in the U.S. Patent of Chen et al. The comparison may be manual or automated. The comparison may be performed based on endogenous X-ray anatomical image contrast, such as from skeletal and/or soft tissue, or exogenous X-ray anatomical image contrast, such as injected, implanted, and/or otherwise attached radio-opaque imaging agents or devices. If the analysis of the output of the comparison at step 1020 is satisfactory, corresponding to the "YES" branch of step 1020, a set of multi-subject multi-modal molecular images 940 may be acquired, step 1040. As seen in Figure 17, set 940 includes two multi-modal molecular images, an upper image 941 a captured using a first molecular imaging mode and a lower image 941b captured using a second molecular imaging mode. The set of modes may include at least one of bright-field mode, dark-field mode, and radio isotopic mode. The set of multi-subject multi-modal molecular images 940 may include at least one image acquired using at least one mode included in the set of modes, wherein the set of multi-subject multi-modal molecular images 940 is co-registered with the test multi-subject X-ray anatomical image 920. If the output of the comparison is unsatisfactory, corresponding to the "NO" branch of step 1020, the immobilized subject mice 900b, c, and d are physically, spatially reoriented at step 1030 to improve the comparison, whereby the physical, spatial reorientation may be determined by spatially mapping the results determined from the digital image processing method for image registration described by Chen et al. to the physical subjects 900b, c, and d, or the reorientation may be made by trial-and-error. The reorientation may be performed by manual means, or by robotic means controlled by the communication and computer control system 20 in the manner previously discussed but via rotational mechanisms 926b, c, and d and X-Y translation mechanisms 928b, c, and d as shown in Figure 16. Steps 1010, 1015, 1020, and 1030 are repeated, until the output of the comparison is satisfactory, thereby corresponding to the "YES" branch of step 1020 and a set of multi-subject multi-modal molecular images 940 is acquired, which is co-registered with a set of multi-subject X-ray anatomical images 930.

In another embodiment the following statistical method is used for step 1030 of Figure 18. Referring to the workflow shown in Figure 19, the comparison of subject mouse "a" in X-ray anatomical image section 925a to the mice "b", "c", and "d" in X-ray anatomical image sections 925b, c, and d is comprised of the steps of applying vector quantization to the X-ray anatomical image sections 925a, b, c, and d; converting the X-ray anatomical image sections to vectorized X-ray anatomical image sections having corresponding local intensity information as derived respectively from the X-ray anatomical image sections 925a, b, c, and d at step 2000; obtaining a joint statistical representation of the X-ray anatomical image sections by employing the vectorized image sections at step 2010; computing cost functions using the joint statistical representation of the X-ray anatomical image sections of subject mice "a", "b", "c", and "d" at step 2020; selecting a reference X-ray anatomical image section (the image section of mouse "a") from the X-ray anatomical image sections at step 2030; and evaluating the cost functions for each of subject mice "b", "c", and "d" at step 2040. If the predetermined cost function criterion is unsatisfied as shown in the "NO" branch of step 2040, subject mice "b", "c", and/or "d" are physically, spatially reoriented according to their virtual, spatial correspondence to the reference X-ray anatomical image section for subject mouse "a" at step 2050, and the flow goes back to step 2000 of Figure 19. If the predetermined cost function criterion is satisfied as shown in the "YES" branch of step 2040, the physical, spatial reorientation is complete and a set of multi-subject multi-modal molecular images 940 can be acquired at step 1040 of Figure 18. Persons skilled in the art understand that step 1030 can be implemented with or without vector quantization in step 2000.

In another embodiment the following method is used for step 1030 of Figure 18. Referring to the workflow shown in Figure 32, the comparison of subject mouse "a" in X-ray anatomical image section 925a to the mice "b", "c", and "d" in X-ray anatomical image sections 925b, c, and d is comprised of the steps of selecting a reference image section (X-ray anatomical image section of mouse a) at step 6000; applying an image registration algorithm (e.g. as described by Chen et al.) to X-ray anatomical image sections of mice a, b, c, and d at step 6010; obtaining minimal cost function values from the image registration process at step 6020; obtaining virtual spatial displacement maps corresponding to the minimal cost functions from the image registration process at step 6030; and evaluating the cost functions at step 6040. If the predetermined cost function criterion is unsatisfied as shown in the "NO" branch of step 6040, the subject mice "b", "c" and/or "d" are physically, spatially reoriented automatically or manually according to the virtual spatial displacement maps at step 6050, and flow goes back to step 6000 of Figure 32. If the predetermined cost function criterion is satisfied as shown in the "YES" branch of step 6040, the physical, spatial reorientation is complete and a set of multi-subject multi-modal molecular images 940 can be acquired at step 1040 of Figure 18. The spatial displacement map can be computed based on the virtual spatial transformation described by Chen et al.

In another embodiment the following method is used for step 1030 of Figure 18. Referring to the workflow shown in Figure 20, the X-ray anatomical image section 925a of subject mouse "a" is compared to the X-ray anatomical image sections 925b, c, and d of subject mice "b", "c", and "d", respectively, and a calculation of the X-ray anatomical image section differences between the images is made at step 3000; and a comparison of the image differences to a null (zero) image section is made at step 3010. Where the comparison of the X-ray anatomical image section differences to a null (zero) image is not satisfactory as shown in the "NO" branch of step 3020, the subject is physically, spatially reoriented according to its virtual spatial correspondence to the reference image at step 3030, and the flow goes back to step 3000 of Figure 20. If the comparison of the X-ray anatomical image section differences to a null (zero) image is satisfactory as shown in "YES" branch of step 3020, the physical, spatial reorientation is complete and a set of multi-subject multi-modal molecular images can be acquired at step 1040.

In another embodiment the problem of registering multi-modal molecular images is solved by virtually, spatially reorienting both the X-ray anatomical images and the multi-modal molecular images of the subject(s) using the same virtual spatial transformation parameters to achieve the desired registration in the resulting images. More specifically referring to Figures 5A and 5B and the workflow shown in Figure 22, a first-time physical, spatial orientation is performed at step 3030, followed by acquisition of a first-time X-ray anatomical image 120 at step 3040 and a first-time set of multi-modal molecular images 122 at step 3050 of subject 112 using system 100. Regions-of-interest templates 3100 and 3110 are created using techniques familiar to those skilled in the art and include region of interest 3105 and regions of interest 3115a and 3115b in the first-time set of multi-modal molecular images 122 at step 3060. Molecular signals are measured in the regions of interest 3105, 3115a and b at step 3070. Figure 21 shows a graphical representation of the first-time molecular signals measured in regions of interest 3105, 3115a and 3115 b.

As shown in Figures 7A and 7C and the workflow shown in Figure 25, a next-time physical, spatial orientation is performed step 3300. A next-time X-ray anatomical image 3200 and a next-time set of multi-modal molecular images 3220 are acquired of subject 112 using the imaging system 100 at step 3310. The next-time X-ray anatomical image 3200 is registered to the first-time X-ray anatomical image 120 at step 3320. The image registration at step 3320 may be performed by using the calculation of the difference between the next-time test X-ray anatomical image 3200 and the first-time X-ray anatomical image 120, or the image registration at step 3320 may be according to the digital image processing method for image registration described by Chen et al. The image registration may be manual or automated. The image registration may be performed based on endogenous X-ray anatomical image contrast, such as from skeletal and/or soft tissue, or exogenous X-ray anatomical image contrast, such as injected, implanted, and/or otherwise attached radio-opaque imaging agents or devices. Once the next-time X-ray anatomical image 3200 is registered to the first-time X-ray anatomical image 120, the same spatial transformation parameters that were required to perform the image registration at step 3320 are applied to the next-time set of multi-modal molecular images as subsequently described with regard to Figure 26, thereby creating a virtually, spatially reoriented next-time set of multi-modal molecular images at step 3330. Next the regions-of-interest templates 3100 and 3110 are applied to the virtually, spatially reoriented next-time set of multi-modal molecular images 3220 at step 3340, and the next-time signals measured in regions of interest 3105, 3115a and b are measured step 3350. At step 3360 the signals are then compared to the signals measured at step 3070. Figures 23 and 24 shows graphical representations of the next-time molecular signals measured in regions of interest 3105, 3115a and 3115b, excluding and including steps 3320 and 3330, respectively, to demonstrate the advantage of the present invention.

In the embodiment described above the following statistical method is used for the image registration step 3320 of Figure 25. Referring to the workflow shown in Figure 26, the registration of the first-time X-ray anatomical image 3210 to the next-time X-ray anatomical image 3200, is accomplished by steps of applying vector quantization to the first-time X-ray anatomical image 3210 and the next-time X-ray anatomical image 3200; converting these X-ray anatomical images to vectorized X-ray anatomical images having corresponding local intensity information as derived respectively from the X-ray anatomical images at step 3400; obtaining a joint statistical representation of the X-ray anatomical images by employing the vectorized X-ray anatomical images at step 3410; computing a cost function using the joint statistical representation of the X-ray anatomical images at step 3420; selecting a reference image (the first-time X-ray anatomical image) from the plurality of X-ray anatomical images at step 3430; and evaluating the cost function at step 3440. If the predetermined cost function criterion is unsatisfied as shown in the "NO" branch of step 3440, the next-time X-ray anatomical image 3200 is virtually, spatially reoriented at step 3450 and the flow goes back to step 3400. If the predetermined cost function criterion is satisfied as shown in the "YES" branch of step 3440, the virtual, spatial reorientation is complete, a virtually, spatially reoriented next-time X-ray anatomical image 3210 is produced, and the flow goes back to step 3330 of Figure 25.

In another embodiment the following method is used for the image registration step 3320 of Figure 25. Referring to the workflow shown in Figure 27, using the first-time X-ray anatomical image 120 and the next-time X-ray anatomical image 3200 a calculation of the image difference between the two images is made at step 3500; and a comparison of the image difference to a null (zero) image is made at step 3510. Where the comparison of the image difference to a null (zero) image is not satisfactory as shown in the "NO" branch of step 3520 the next-time X-ray anatomical image 3200 is virtually, spatially reoriented at step 3530 and the flow returns to step 3500. If the comparison of the image difference to a null (zero) image is satisfactory as shown in "YES" branch of step 3520, the virtual, spatial reorientation is complete, a virtually, spatially reoriented next-time X-ray anatomical image 3210 is produced, and the flow goes to step 3330 of Figure 25.

It should be understood that the method described as registration of multi-modal molecular images and shown in Figures 5A, 5B, 7A, 7C and 21 to 27 may be applied to any of the following scenarios; imaging a single subject at different times, imaging multiple subjects serially, and imaging multiple subjects in parallel.

The method of virtual, spatial reorientation of multi-modal molecular images is better suited for reproducing the spatial orientation when the molecular signals are closer to the surface of the subject and not significantly affected by the optical effects of tissue such as absorption and scattering, while the method of physical, spatial reorientation of the subject(s) is better suited for reproducing the spatial orientation when the molecular signals are deeper within the subject and are significantly affected by the optical effects of tissue such as absorption and scattering. However, it will be understood that the method of virtual, spatial orientation of multi-modal molecular images may be useful for reproducing the spatial orientation when the molecular signals are deeper within the subject, and that the method of physical, spatial reorientation of the subject(s) may be useful for reproducing the spatial orientation when the molecular signals are closer to the surface of the subject.

An example of use of an exogenous X-ray anatomical image contrast agent to facilitate the reproduction of spatial orientation is shown in Figure 28. A radio opaque imaging agent 3600 is injected into the subject. The imaging agent provides contrast associated with the soft tissue, such as the organs and/or vasculature, of the subject. Such radio opaque imaging agents include barium, palladium, gold, and iodine. An example of use of an exogenous X-ray anatomical image contrast device to facilitate the reproduction of spatial orientation is shown in Figure 29. Solid metal objects or pieces of metal foil 3610a and b are inserted and/or attached to the subject.

Another method for reproducing the spatial orientation of immobilized subjects in a multi-modal imaging system is shown in Figures 34 to 41. First, as shown in Figures 34, 41A and 41B, a series of reference physical, spatial orientations of the immobilized subject(s) in the multi-modal imaging system is performed, whereby a reference X-ray anatomical image of the immobilized subject(s) is acquired for each physical, spatial orientation, step 7000. Figure 34 shows a series of reference X-ray anatomical images of an immobilized mouse in which the spatial orientation, in this case the cranio-caudal rotation angle, has been incremented by approximately 30 degrees from image to image over 360 degrees. Next, a gradient image and an opposite-gradient image for each reference X-ray anatomical image are calculated, step 7010 of Figure 41A. Methods for calculating a gradient image are known in the art; such methods involve application of an edge-detection kernel, for example a Prewitt kernel, Sobel kernel, or variations thereof, to the image. For example, the series of gradient images shown in Figure 35A was obtained by taking the X-ray anatomical images shown in Figure 34 and applying the following 7x7 left-to-right edge-detection kernel:

| | | | | | | |
|---|---|---|---|---|---|---|
| +1 | +1 | +1 | +1 | -1 | -1 | -1 |
| +1 | +1 | +1 | +1 | -1 | -1 | -1 |
| +1 | +1 | +1 | +1 | -1 | -1 | -1 |
| +1 | +1 | +1 | -6 | -1 | -1 | -1 |
| +1 | +1 | +1 | +1 | -1 | -1 | -1 |
| +1 | +1 | +1 | +1 | -1 | -1 | -1 |
| +1 | +1 | +1 | +1 | -1 | -1 | -1 |

This kernel is appropriate because the direction of the cranio-caudal axis is from the top to bottom in the images, so the edges of interest (e.g., the edges of the pubis bones) will be detected by a left-to-right edge-detection kernel. The series of opposite-gradient images shown in Figure 35B was obtained by taking the X-ray anatomical images shown in Figure 34 and applying the following 7x7 right-to-left edge-detection kernel:

| | | | | | | |
|---|---|---|---|---|---|---|
| -1 | -1 | -1 | +1 | +1 | +1 | +1 |
| -1 | -1 | -1 | +1 | +1 | +1 | +1 |
| -1 | -1 | -1 | +1 | +1 | +1 | +1 |
| -1 | -1 | -1 | -6 | +1 | +1 | +1 |
| -1 | -1 | -1 | +1 | +1 | +1 | +1 |
| -1 | -1 | -1 | +1 | +1 | +1 | +1 |
| -1 | -1 | -1 | +1 | +1 | +1 | +1 |

Next, a line profile for each gradient image and opposite-gradient image is captured, step 7020 of Figure 41A. For example, the location of such a line profile is shown in Figures 35A and B, where the line profile intersects the pubis bones of the mouse. A plurality of line profiles may also be appropriate. Next, the abscissae of the line profiles from the opposite-gradient images are reversed, step 7030. For example, the series of line profiles shown in Figure 36 include the gradient image line profiles (solid curves) plotted with the abscissae based on the gradient image left-to-right coordinates, and the opposite-gradient image line profiles (dashed curves) plotted with the abscissae reversed from the opposite-gradient image left-to-right coordinates. Next, for each reference physical, spatial orientation, the cross-correlation of the line profile from the gradient image and the abscissa-reversed line profile from the opposite-gradient image is calculated, step 7040. Alternatively, those skilled in the art would recognize that it is mathematically equivalent to forego the calculation of the opposite-gradient images and simply to take the line profiles from the gradient images, reverse their abscissae, negate their ordinates, and calculate the cross-correlations of the results with the original line profiles. Next, for each reference physical, spatial orientation, the maximum of the resulting cross-correlations are determined and plotted vs. physical, spatial orientation (e.g., cranio-caudal rotation angle), for example as shown in Figure 37, step 7050. Next, the peak positions in the plot of cross-correlation maximum vs. reference physical, spatial orientation are assigned to prone and supine physical, spatial orientations, step 7060. For the plot shown in Figure 37, the prone physical, spatial orientations are assigned to 0 degrees and 360 degrees, and the supine physical, spatial orientation is assigned to 180 degrees. This assignment is enabled by the fact that the peaks in the plot of cross-correlation maximum vs. physical, spatial orientation are indicative of the physical, spatial orientations that exhibit maximal bilateral symmetry. The assignment to prone and supine physical, spatial orientations presumes prior knowledge of the approximate physical, spatial relationship of the subject to the imaging system in the series of X-ray anatomical images to be able to distinguish the prone physical, spatial orientations from the supine physical, spatial orientations. Next, the reference physical, spatial orientations corresponding to prone and supine physical, spatial orientations are used as references for achieving an arbitrary physical, spatial orientation, step 7070.

For example, Figure 33A shows that the optimal physical, spatial orientation for detecting a fluorescence molecular signal from the right kidney is at -150 degrees (or, equivalently, 210 degrees), where 0 degrees is defined as the prone position and clockwise rotation is defined as being a negative rotation, so upon determination of the physical, spatial orientation (e.g., cranio-caudal rotation angle) corresponding to the prone physical, spatial orientation, the subject would be rotated -150 degrees to obtain the optimal physical, spatial orientation for detecting a fluorescence molecular signal from the right kidney.

Next, reference sets of multi-modal molecular images of the immobilized subjects using a set of modes of the multi-modal imaging system are acquired, whereby the sets of multi-modal molecular images include at least one image acquired using at least one mode included in the set of modes, step 7080 of Figure 41A. Next, for example at a later time for the same subject, or upon substitution of a different subject, a series of test physical, spatial orientations of the immobilized subject(s) in the multi-modal imaging system is performed, whereby a test X-ray anatomical image of the immobilized subject(s) is acquired for each physical, spatial orientation, step 7090 of Figure 41B. For example, a series of test X-ray anatomical images of an immobilized mouse whereby the physical, spatial orientation, in this case the cranio-caudal rotation angle, has been incremented by 30 degrees would be as shown in Figure 34, but shifted one image to the right due to a happenstance 30 degree difference in the initial animal positioning, as illustrated.

Next, a gradient image and an opposite-gradient image for each test X-ray anatomical image is calculated, step 7100. For example, the series of gradient images shown in Figure 38A were obtained by taking the X-ray anatomical images shown in Figure 34, shifted one image to the right due to a happenstance 30 degree difference in the initial animal positioning, and applying the 7x7 left-to-right edge-detection kernel as described previously; and the series of opposite-gradient images shown in Figure 38B were obtained by taking the X-ray anatomical images shown in Figure 34, shifted one image to the right due to a happenstance 30 degree difference in the initial animal positioning, and applying the 7x7 right-to-left edge-detection kernel as described previously.

Next, a line profile for each gradient image and opposite-gradient image is captured, step 7110. For example, the location of such a line profile is shown in Figures 38A and B, whereby the line profile intersects the pubis bones of the mouse. A plurality of line profiles may also be appropriate. Next, the abscissae of the line profiles from the opposite-gradient images are reversed, step 7120. For example, the series of line profiles shown in Figure 39 include the gradient image line profiles (solid curves) plotted with the abscissae based on the gradient image left-to-right coordinates, and the opposite-gradient image line profiles (dashed curves) plotted with the abscissae reversed from the opposite-gradient image left-to-right coordinates.

Next, for each test physical, spatial orientation, the cross-correlation of the line profile from the gradient image and the abscissa-reversed line profile from the opposite-gradient image is calculated, step 7130. Alternatively, those skilled in the art would recognize that it is mathematically equivalent to forego the calculation of the opposite-gradient images and simply to take the line profiles from the gradient images, reverse their abscissae, negate their ordinates, and calculate the cross-correlations of the results with the original line profiles.

Next, for each test physical, spatial orientation, the maximum of the resulting cross-correlations are determined and plotted vs. physical, spatial orientation (e.g., cranio-caudal rotation angle), for example as shown in Figure 40, step 7140. Next, the peak positions in the plot of cross-correlation maximum vs. test spatial orientation are assigned to prone and supine physical, spatial orientations, step 7150. For the plot shown in Figure 40, the prone physical, spatial orientation is assigned to 150 degrees, and the supine physical, spatial orientation is assigned to 330 degrees. This assignment is enabled by the fact that the peaks in the plot of cross-correlation maximum vs. physical, spatial orientation are indicative of the physical, spatial orientations that exhibit maximal bilateral symmetry. The assignment to prone and supine physical, spatial orientations presumes prior knowledge of the approximate physical, spatial relationship of the subject to the imaging system in the series of X-ray anatomical images to be able to distinguish the prone physical, spatial orientations from the supine physical, spatial orientations.

Next, the test physical, spatial orientations corresponding to prone and supine physical, spatial orientations are used as references for achieving an arbitrary physical, spatial orientation, i.e., reproducing the arbitrary physical, spatial orientation achieved previously, for example -150 degree rotation from the prone physical, spatial orientation, step 7160.

Finally, sets of multi-modal molecular images of the immobilized subjects using a set of modes of the multi-modal imaging system are acquired, whereby the sets of multi-modal molecular images include at least one image acquired using at least one mode included in the set of modes, step 7170. Hence, the sets of multi-modal molecular images may be fairly compared to the reference sets of multi-modal molecular images by virtue of the reproduction of the physical, spatial orientation.

Although one or more line profiles may be used to assess the degree of bilateral symmetry of the X-ray anatomical images as described above, one may alternatively use a method involving analysis of gradient orientation histograms to assess the degree of bilateral symmetry of the X-ray anatomical images, for example as described in "Symmetry detection using gradient information" by C. Sun, Pattern Recognition Letters 16 (1995) 987-996, and "Fast Reflectional Symmetry Detection Using Orientation Histograms" by C. Sun and D. Si, Real-Time Imaging 5, 63-74,1999. An embodiment using this method is described in Figures 42A and B. In this method, first a series of reference physical, spatial orientations of the immobilized subject(s) in the multi-modal imaging system is performed, whereby a reference X-ray anatomical image of the immobilized subject(s) is acquired for each physical, spatial orientation, step 8000. Next, a gradient image and an orthogonal-gradient image for each reference X-ray anatomical image are calculated, step 8010.

Methods for calculating a gradient image are known in the art; such methods involve application of an edge-detection kernel, for example a Prewitt kernel, Sobel kernel, or variations thereof, to the image.

For example, the following 7x7 edge-detection kernel may be applied to calculate the gradient image:

| | | | | | | |
|---|---|---|---|---|---|---|
| +1 | +1 | +1 | +1 | -1 | -1 | -1 |
| +1 | +1 | +1 | +1 | -1 | -1 | -1 |
| +1 | +1 | +1 | +1 | -1 | -1 | -1 |
| +1 | +1 | +1 | -6 | -1 | -1 | -1 |
| +1 | +1 | +1 | +1 | -1 | -1 | -1 |
| +1 | +1 | +1 | +1 | -1 | -1 | -1 |
| +1 | +1 | +1 | +1 | -1 | -1 | -1 |

The following 7x7 edge-detection kernel may be applied to calculate the orthogonal-gradient image:

| | | | | | | |
|---|---|---|---|---|---|---|
| +1 | +1 | +1 | +1 | +1 | +1 | +1 |
| +1 | +1 | +1 | +1 | +1 | +1 | +1 |
| +1 | +1 | +1 | +1 | +1 | +1 | +1 |
| +1 | +1 | +1 | -6 | +1 | +1 | +1 |
| -1 | -1 | -1 | -1 | -1 | -1 | -1 |
| -1 | -1 | -1 | -1 | -1 | -1 | -1 |
| -1 | -1 | -1 | -1 | -1 | -1 | -1 |

Next, a gradient orientation image is calculated for each pair of gradient image and orthogonal-gradient image by calculating the inverse tangent of the pair, step 8020.

Next, the gradient orientation histogram is calculated for each gradient orientation image, step 8030.

Next, each gradient orientation histogram is analyzed to calculate the degree of the bilateral symmetry of the corresponding reference X-ray anatomical image which is plotted vs. reference spatial orientation (e.g., cranio-caudal rotation angle), step 8040.

Next, peak positions are assigned in the plot of degree of bilateral symmetry vs. reference physical, spatial orientation to prone and supine physical, spatial orientations, step 8050.

Next, reference physical, spatial orientations corresponding to prone and supine physical, spatial orientations are used as references for achieving an arbitrary physical, spatial orientation, step 8060. For example, Figure 33A shows that the optimal physical, spatial orientation for detecting a fluorescence molecular signal from the right kidney is at -150 degrees (or, equivalently, 210 degrees), where 0 degrees is defined as the prone position and clockwise rotation is defined as being a negative rotation, so upon determination of the physical, spatial orientation (e.g., cranio-caudal rotation angle) corresponding to the prone physical, spatial orientation, the subject would be rotated -150 degrees to obtain the optimal physical, spatial orientation for detecting a fluorescence molecular signal from the right kidney.

Next, reference sets of multi-modal molecular images of the immobilized subjects are acquired using a set of modes of the multi-modal imaging system, whereby the sets of multi-modal molecular images include at least one image acquired using at least one mode included in the set of modes, step 8070.

Next, a series of test physical, spatial orientations of the immobilized subjects in the multi-modal imaging system is performed, whereby a test X-ray anatomical image of the immobilized subject(s) is acquired for each physical, spatial orientation, step 8080 in Figure 42B.

Next, a gradient image and an orthogonal-gradient image for each test X-ray anatomical image is calculated, step 8090.

Next, a gradient orientation image is calculated for each pair of gradient image and orthogonal-gradient image by calculating the inverse tangent of the pair, step 8100. Next, the gradient orientation histogram is calculated for each gradient orientation image, step 8110.

Next, each gradient orientation histogram is analyzed to calculate the degree of the bilateral symmetry of the corresponding test X-ray anatomical image which is plotted vs. test physical, spatial orientation (e.g., cranio-caudal rotation angle), step 8120.

Next, peak positions are assigned in the plot of degree of bilateral symmetry vs. test physical, spatial orientation to prone and supine physical, spatial orientations, step 8130.

Next, test physical, spatial orientations corresponding to prone and supine physical, spatial orientations are used as references for achieving an arbitrary physical, spatial orientation, step 8140.

Finally, sets of multi-modal molecular images of the immobilized subjects are acquired using a set of modes of the multi-modal imaging system, whereby the sets of multi-modal molecular images include at least one image acquired using at least one mode included in the set of modes, step 8150. Hence, the sets of multi-modal molecular images may be fairly compared to the reference sets of multi-modal molecular images by virtue of the reproduction of the physical, spatial orientation.

Other methods for assessing the degree of bilateral symmetry of X-ray anatomical images are described in the art and are applicable to this invention; for example, "Optimal Detection of Symmetry Axis in Digital Chest X-ray Images" by C. Vinhais and A. Campilho, F. J. Perales et al. (Eds.): IbPRIA 2003, LNCS 2652, pp. 1082-1089, 2003, and references cited therein.

Another method for reproducing the physical, spatial orientation of immobilized subjects in a multi-modal imaging system is shown in Figures 43 to 50. First, as shown in Figures 43, 50A and 50B, a series of reference physical, spatial orientations of the immobilized subject(s) in the multi-modal imaging system is performed, whereby a reference X-ray anatomical image of the immobilized subject(s) is acquired for each physical, spatial orientation, step 9000. Figure 43 shows a series of reference X-ray anatomical images of an immobilized mouse in which the physical, spatial orientation, in this case the cranio-caudal rotation angle, has been incremented by approximately 5 degrees from image to image.

Next, an X-ray density image is calculated for each reference X-ray anatomical image, step 9010. Figure 44 shows X-ray density images corresponding to the images in Figure 43. The calculation of the X-ray density images, i.e., conversion of the image intensity scale to an X-ray density scale, is achieved using methods well-known to those of ordinary skill in the art.

Next, pixels with X-ray density less than a predetermined threshold are set to zero (i.e., discarded), and pixels with X-ray density greater than or equal to the predetermined threshold are set to one (i.e., retained), in other words a binary thresholding operation, step 9020. The predetermined threshold is designed to substantially discard pixels corresponding to soft-tissue (e.g., muscle tissue, intestines, etc.) and to substantially retain pixels corresponding to skeletal tissue. For example, a threshold value of approximately 0.9 has been empirically found to suffice for mice weighing 20-25 grams, and was used to obtain the series of binary thresholded images shown in Figure 45 based on the series of X-ray density images of Figure 44. Hence, the reason for conversion of the original images to X-ray density scale at step 9010 is to provide calibrated images for binary thresholding and thereby remove all image intensity scale dependence on factors such as X-ray source intensity, phosphor screen speed, exposure time, and sensor speed.

Next, a gradient image for each reference X-ray anatomical image is calculated, step 9030. Methods for calculating a gradient image are known in the art; such methods involve application of an edge-detection kernel, for example a Prewitt kernel, Sobel kernel, or variations thereof, to the image. For example, the series of gradient images shown in Figure 46 was obtained by taking the X-ray anatomical images shown in Figure 43 and applying the following 7x7 left-to-right edge-detection kernel:

| | | | | | | |
|---|---|---|---|---|---|---|
| +1 | +1 | +1 | +1 | -1 | -1 | -1 |
| +1 | +1 | +1 | +1 | -1 | -1 | -1 |
| +1 | +1 | +1 | +1 | -1 | -1 | -1 |
| +1 | +1 | +1 | -6 | -1 | -1 | -1 |
| +1 | +1 | +1 | +1 | -1 | -1 | -1 |
| +1 | +1 | +1 | +1 | -1 | -1 | -1 |
| +1 | +1 | +1 | +1 | -1 | -1 | -1 |

This kernel is appropriate because the direction of the cranio-caudal axis is from the top to bottom in the images, so the edges of interest will be detected by a left-to-right edge-detection kernel. Alternatively, a right-to-left edge detection kernel would serve equivalently.

Next, the results of step 9020 of Figure 50A are imagewise (that is, pixel by pixel) multiplied by the results of step 9030 in step 9040. For example, Figure 47 shows the series of images of Figure 45 imagewise multiplied by the series of images of Figure 46. The purpose of step 9040 is to use the results of the binary thresholding operation of step 9020 to mask the gradient images of step 9030, hence isolating and retaining the gradient values due to the skeletal features and discarding the gradient values due to soft-tissue, especially the boundary of the animal.

Next, the imagewise absolute values of the results of step 9040 are calculated, step 9050. For example, Figure 48 shows the imagewise absolute value of the series of images of Figure 47. The calculation of the imagewise absolute values is necessary to assess the magnitude of the gradient values. Alternatively, any even function may be performed on the output of step 9040. Alternatively, the calculation of the imagewise absolute values or any even function could be performed on the results of step 9030 instead of the results of step 9040, and then those results could be used as the input to step 9040 instead of the results of step 9030.

Next, the sum within a predetermined region of interest is calculated for the results of step 9050 in step 9060. The predetermined region of interest is chosen so as to include sufficient skeletal features to assess the overall skeletal alignment of the animal with respect to the cranio-caudal rotation axis: when the cranio-caudal rotation angle of the animal is other than those corresponding to prone or supine physical, spatial orientations, then many dominant skeletal features such as the spine and femurs are askew with respect to the cranio-caudal rotation axis due to the projection of the X-ray shadow of the natural geometry of these features onto the phosphor screen; however, when the cranio-caudal rotation angle of the animal corresponds to prone or supine physical, spatial orientations, then many dominant skeletal features such as the spine and femurs appear aligned to the cranio-caudal rotation axis. The predetermined region of interest may include the entire animal as shown in Figure 43 to 48, or may alternatively include only a portion of the animal (such as below the head, or around and below the pelvis).

Next, the peak positions in the plot of the results of step 9060 vs. reference physical, spatial orientation are assigned to prone and supine physical, spatial orientations, step 9070 of Figure 50A. For example, such a plot is shown in Figure 49, showing the peak corresponding to the prone physical, spatial orientation. The plot shows a relative peak height above background of 15%, which is sufficient to identify the peak position.

Next, the reference physical, spatial orientations corresponding to prone and supine physical, spatial orientations are used as references for achieving an arbitrary physical, spatial orientation, step 9080.

Next, reference sets of multi-modal molecular images of the immobilized subjects using a set of modes of the multi-modal imaging system are acquired, whereby the sets of multi-modal molecular images include at least one image acquired using at least one mode included in the set of modes, step 9090.

Next, a series of test physical, spatial orientations of the immobilized subject(s) in the multi-modal imaging system is performed, whereby a test X-ray anatomical image of the immobilized subject(s) is acquired for each physical, spatial orientation, step 9100 in Figure 50B.

Next, an X-ray density image is calculated for each test X-ray anatomical image, step 9110.

Next, pixels with X-ray density less than the predetermined threshold used in step 9020 are set to zero (i.e., discarded), and pixels with X-ray density greater than or equal to the predetermined threshold used in step 9020 are set to one (i.e., retained), in other words a binary thresholding operation, step 9120.

Next, a gradient image for each test X-ray anatomical image is calculated, step 9130.

Next, the results of step 9120 are imagewise multiplied by the results of step 9130, step 9140.

Next, the imagewise absolute values of the results of step 9140 are calculated, step 9150.

Next, the sum within a predetermined region of interest is calculated for the results of step 9150, step 9160.

Next, the peak positions in the plot of the results of step 9160 vs. test physical, spatial orientation are assigned to prone and supine orientations, step 9170.

Next, the test physical, spatial orientations corresponding to prone and supine physical, spatial orientations are used as references for achieving an arbitrary physical, spatial orientation, step 9180.

Finally, sets of multi-modal molecular images of the immobilized subjects using a set of modes of the multi-modal imaging system are acquired, whereby the sets of multi-modal molecular images include at least one image acquired using at least one mode included in the set of modes, step 9190.

**PARTS LIST**

| | |
|---|---|
| 10 | electronic imaging system |
| 12 | light source |
| 14 | optical compartment |
| 16 | mirror |
| 18 | lens and camera system |
| 20 | communication and computer control system |

| | |
|---|---|
| 22 | display device, computer monitor |
| 100 | imaging system |
| 102 | X-ray source |
| 104 | sample object stage |
| 106 | fiber optics |
| 108 | sample environment |
| 110 | access means or member |
| 112 | subject mouse |
| 114 | respiratory device |
| 116 | tube |
| 118 | cylindrical sample chamber or tube |
| 120 | first-time X-ray anatomical image |
| 122 | first-time set of multi-modal molecular images |
| 124 | next-time test X-ray anatomical image |
| 126 | rotational mechanism |
| 128 | translation mechanism |
| 130 | next-time X-ray anatomical image after physical, spatial reorientation |
| 132 | next-time set of multi-modal molecular images |
| 200 - 420 | process steps |
| 500a, b, c, d | subject mouse |
| 510a, b, c, d | cylindrical sample tube |
| 520a | first-subject X-ray anatomical image |
| 525b, c, d | next-subject test X-ray anatomical image |
| 530a | first-subject set of multi-modal molecular images |
| 531a, b | images |
| 535b, c, d | next-subject X-ray anatomical image after physical, spatial reorientation |
| 540b, c, d | next-subject set of multi-modal molecular images |
| 541a | next-subject multi-modal molecular images captured using a first molecular imaging mode |
| 541b | next-subject multi-modal molecular images captured using a second molecular imaging mode |
| 542a | next-subject multi-modal molecular images captured using a first molecular imaging mode |
| 542b | next-subject multi-modal molecular images captured using a second molecular imaging mode |
| 543a | next-subject multi-modal molecular images captured using a first molecular imaging mode |
| 543b | next-subject multi-modal molecular images captured using a second molecular imaging mode |
| 600 - 830 | process steps |
| 900a, b, c, d | subject mice |
| 910a, b, c, d | animal chambers |
| 920 | test multi-subject X-ray anatomical image |
| 925a, b, c, d | image sections |
| 926a, b, c, d | rotational mechanism |
| 928a, b, c, d | translation mechanism |
| 930 | multi-subject X-ray anatomical image after physical, spatial reorientation |
| 940 | set of multi-subject multi-modal molecular images |
| 941a | multi-subject multi-modal molecular images captured using a first molecular imaging mode |
| 941b | multi-subject multi-modal molecular images captured using a second molecular imaging mode |
| 1000 - 3070 | process steps |
| 3100 | regions-of-interest template |
| 3105 | region of interest |
| 3110 | regions-of-interest template |
| 3115a, b | regions of interest |
| 3200 | next-time X-ray anatomical image |
| 3210 | virtually, spatially reoriented next-time X-ray anatomical image |
| 3220 | virtually, spatially reoriented next-time set of multi-modal molecular images |
| 3300 - 3530 | process steps |
| 3600 | exogenous X-ray anatomical image contrast agent |
| 3610a, b | exogenous X-ray anatomical image contrast devices |
| 4000 - 9190 | process steps |

## Claims

1. A method of reproducing the physical, spatial orientation of an immobilized subject in an X-ray imaging system (100) including a computer (20) from one subject for another subject, comprising:

    performing a physical, spatial orientation (600) of a first immobilized subject (500a) in the multi-modal imaging system (100),
    using the computer (20), acquiring (610) an X-ray anatomical image of the first immobilized subject (500a) in the imaging system (100);
    performing a physical, spatial orientation (630) of a next immobilized subject (500b) in the imaging system (100);
    using the computer (20), acquiring (640) a test X-ray anatomical image of the next immobilized subject (500b) in the imaging system (100);
    using the computer (20), comparing (650) the test X-ray anatomical image of the next immobilized subject (500b) and the X-ray anatomical image of the first immobilized subject (500a), including a calculation of the difference therebetween;
    physically, spatially reorienting (660) the next immobilized subject (500b) to improve the comparison, if the comparison is not satisfactory to demonstrate reproduction of the physical, spatial orientation of the first immobilized subject (500a);
    repeating the steps of performing a physical, spatial orientation (630) of a next immobilized subject (500b), acquiring (640) a test X-ray anatomical image, comparing (650) and physically, spatially reorienting (660) until the comparison is satisfied; and
    acquiring (640) an X-ray anatomical image of the next immobilized subject (500b) in the multi-modal imaging system (100).

2. The method of claim 1, where the first and next X-ray anatomical images are digital and the step of reorienting (660) comprises:

    applying vector quantization (700) to the digital X-ray anatomical images of the first (500a) and next (500b) subjects to convert the digital X-ray anatomical images to vectorized X-ray anatomical images having corresponding local intensity information as derived respectively from the digital X-ray anatomical images;
    obtaining (710) a joint statistical representation of the digital X-ray anatomical images by employing the vectorized X-ray anatomical images;
    computing (720) a cost function using the joint statistical representation of the digital X-ray anatomical images;
    selecting (730) the first X-ray anatomical image as a reference X-ray anatomical image from the digital X-ray anatomical images;
    evaluating (740) the cost function;
    physically, spatially reorienting (750) the next subject (500b) according to its virtual spatial correspondence to the reference X-ray anatomical image, where a predetermined cost function criterion is unsatisfied; and
    repeating the steps of applying (700), obtaining (710), computing (720), selecting (730), evaluating (740) and physically, spatially reorienting (750), where the predetermined cost function criterion is unsatisfied, for the next-subject (500b) that has been previously physically, spatially reoriented (750) in order to align the X-ray anatomical image of the next-subject (500b) to the reference X-ray anatomical image.

3. The method of claim 1, where the step of physically, spatially reorienting (660) comprises:

    calculating the difference (800) between the test X-ray anatomical image of the next subject (500b) and the X-ray anatomical image of the first subject (500a);
    comparing the difference (810, 820) to a null (zero) image;
    physically, spatially reorienting (830) the next subject (500b) according to its virtual spatial correspondence to the X-ray anatomical image of the first subject (500a); and
    repeating the steps of calculating the difference (800), comparing the difference (810, 820), and physically, spatially reorienting (830) the next subject (500b) until the null (zero) image difference criterion is unsatisfied.

4. The method of claim 1, where the step of physically, spatially reorienting (660) comprises:

    selecting the first-subject X-ray anatomical image as a reference image;
    applying an image registration algorithm to the first-subject X-Ray anatomical image and the next-subject test X-Ray anatomical image;

obtaining a minimal cost function value from applying the image registration algorithm;
obtaining a virtual spatial displacement map corresponding to the minimal cost function from applying the image registration algorithm;
evaluating the cost function;
physically, spatially reorienting the subject according to the virtual spatial displacement map, where a predetermined cost function criterion is unsatisfied; and
repeating the steps of selecting, applying, obtaining a minimal cost function value, obtaining a virtual spatial displacement map, evaluating and physically, spatially reorienting, where the predetermined cost function criterion is unsatisfied, for the next subject that has been previously physically, spatially reoriented in order to align the next-subject X-ray anatomical image with the reference X-ray anatomical image.

**5.** A method of adjusting a physical, spatial orientation of at least one immobilized subject in an X-ray imaging system (100) including a computer (20), so as substantially to reproduce the physical, spatial orientation of another, reference immobilized subject, comprising:

performing a physical, spatial orientation (1000) of the reference subject;
using the computer (20), acquiring (1010) an X-ray anatomical image of the reference subject;
performing a physical, spatial orientation (1000) of the at least one subject;
using the computer (20), acquiring (1010) an X-ray anatomical image of the at least one subject;
using the computer (20), analyzing (1020) the combination of the X-ray anatomical image of the reference subject and the X-ray anatomical image of the at least one subject; and
following the analyzing, physically, spatially reorienting (1030) the at least one subject so as substantially to reproduce the physical, spatial orientation of the reference subject.

**6.** A method according to claim 5, wherein the X-ray imaging system (100) is furthermore a multi-modal system, the method also comprises acquiring a set of multi-modal molecular images of the at least one immobilized subject, having such a substantially reproduced physical spatial orientation, for comparison with a corresponding set of multi-modal molecular images of the reference immobilized subject, further comprising:

acquiring (1040) a set of multi-modal molecular images of the reference subject;
following the reorienting, acquiring (1040) a set of multi-modal molecular images of the at least one subject; and
comparing the multi-modal molecular images of the at least one subject with the multi-modal molecular images of the reference subject.

## Patentansprüche

**1.** Verfahren zum Wiedergeben der physikalisch räumlichen Orientierung eines immobilisierten Subjektes in einem Röntgenbildgebungssystem (100), welches einen Computer (20) aufweist, von einem Subjekt für ein anderes Subjekt, welches Folgendes aufweist:

Ausführen einer physikalisch räumlichen Orientierung (600) eines ersten immobilisierten Subjektes (500a) in dem multimodalen Abbildungssystem (100),
Aufnehmen (610) eines anatomischen Röntgenbildes des ersten immobilisierten Subjektes (500a) in dem Bildgebungssystem (100) unter Verwendung des Computers (20);
Ausführung einer physikalisch räumlichen Orientierung (630) eines nächsten immobilisierten Subjektes (500b) in dem Bildgebungssystem (100);
Aufnehmen (640) eines anatomischen Röntgentestbildes des nächsten immobilisierten Subjektes (500a) in dem Bildgebungssystem (100) unter Verwendung des Computers (20);
Vergleichen (650) des anatomischen Röntgentestbildes des nächsten immobilisierten Subjektes (500b) und des anatomischen Röntgenbildes des ersten immobilisierten Subjektes (500a), einschließlich einer Berechnung der Differenz dazwischen unter Verwendung des Computers (20);
physikalisch räumliches Reorientieren (660) des nächsten immobilisierten Subjektes (500b), um den Vergleich zu verbessern, wenn der Vergleich nicht zufriedenstellend ist, um die Wiedergabe der physikalisch räumlichen Orientierung des ersten immobilisierten Subjektes (500a) zu zeigen;
Wiederholen der Schritte des Ausführens einer physikalisch räumlichen Orientierung (630) eines nächsten immobilisierten Subjektes (500b), des Aufnehmens (640) eines anatomischen Röntgentestbildes, des Vergleichens (650) und des physikalisch räumlichen Reorientierens (660) bis der Vergleich zufriedenstellend ist; und

Aufnehmen (640) eines anatomischen Röntgenbildes des nächsten immobilisierten Subjektes (500b) in dem multimodalen Bildgebungssystem (100).

**2.** Verfahren nach Anspruch 1, wobei das erste und das nächste anatomische Röntgenbild digital sind, und wobei der Schritt des Reorientierens (660) Folgendes aufweist:

Anwenden einer Vektorquantisierung (700) auf die digitalen anatomischen Röntgenbilder des ersten Subjektes (500a) und des nächsten Subjektes (500b), um die digitalen anatomischen Röntgenbilder in vektorisierte anatomische Röntgenbilder mit entsprechender lokaler Intensitätsinformation umzuwandeln, wie jeweils aus dem digitalen anatomischen Röntgenbildern abgeleitet;
Gewinnen (710) einer gemeinsamen statistischen Darstellung der digitalen anatomischen Röntgenbilder durch Einsetzen der vektorisierten anatomischen Röntgenbilder;
Berechnen (720) einer Kostenfunktion unter Verwendung der gemeinsamen statistischen Darstellung der digitalen anatomischen Röntgenbilder;
Auswählen (730) des ersten anatomischen Röntgenbildes aus den digitalen anatomischen Röntgenbildern als ein anatomisches Referenz-Röntgenbild;
Bewerten (740) der Kostenfunktion;
physikalisch räumliches Reorientieren (750) des nächsten Subjektes (500b) gemäß seiner virtuellen räumlichen Entsprechung zu dem anatomischen Röntgenreferenzbild, wenn ein vorbestimmtes Kostenfunktionskriterium nicht erfüllt ist; und
Wiederholen der Schritte des Anwendens (700), Gewinnens (710), Berechnens (720), Auswählens (730), Bewertens (740) und physikalisch räumlichen Reorientierens (750), wenn das vorbestimmte Kostenfunktionskriterium nicht erfüllt ist, und zwar für das nächste Subjekt (500b), welches zuvor physikalisch räumlich reorientiert worden ist (750), um das anatomische Röntgenbild des nächsten Subjektes (500b) mit dem anatomischen Röntgenreferenzbild auszurichten.

**3.** Verfahren nach Anspruch 1, wobei der Schritt des physikalisch räumlichen Reorientierens (660) Folgendes aufweist:

Berechnen der Differenz (800) zwischen dem anatomischen Röntgentestbild des nächsten Subjektes (500b) und dem anatomischen Röntgenbild des ersten Subjektes (500a);
Vergleichen der Differenz (810, 820) mit einem Null-Bild (Null);
physikalisch räumliches Reorientieren (830) des nächsten Subjektes (500b) gemäß seiner virtuellen räumlichen Entsprechung zu dem anatomischen Röntgenbild des ersten Subjektes (500a); und
Wiederholen der Schritte des Berechnens der Differenz (800), des Vergleiches der Differenz (810, 820) und des physikalisch räumlichen Reorientierens (830) des nächsten Subjektes (500b) bis das Kriterium einer Null-Bilddifferenz (Null) nicht erfüllt ist.

**4.** Verfahren nach Anspruch 1, wobei der Schritt des physikalisch räumlichen Reorientierens (660) Folgendes aufweist:

Auswählen des anatomischen Röntgenbildes des ersten Subjektes als ein Referenzbild;
Anwenden eines Bildregistrierungs- bzw. Bildübereinanderlage-Algorithmus auf das anatomische Röntgenbild des ersten Subjektes und auf das anatomische Röntgentestbild des nächsten Subjektes;
Gewinnen eines minimalen Kostenfunktionswertes durch Anwenden des Bildregistrierungsalgorithmus;
Gewinnen einer Karte bzw. eines Kennfeldes zur virtuellen räumlichen Verschiebung entsprechend der minimalen Kostenfunktion vom Anwenden des Bildregistrierungsalgorithmus;
Bewerten der Kostenfunktion;
physikalisch räumliches Reorientieren des Subjektes gemäß der Karte der virtuellen räumlichen Verschiebung, wenn ein vorbestimmtes Kostenfunktionskriterium nicht erfüllt ist; und
Wiederholen der Schritte des Auswählens, Anwendens, Gewinnens eines minimalen Kostenfunktionswertes, Erhaltens einer Karte der virtuellen räumlichen Verschiebung, des Bewertens und physikalisch räumlichen Reorientierens, wenn das vorbestimmte Kostenfunktionskriterium nicht erfüllt ist, für das nächste Subjekt, welches zuvor physikalisch räumlich reorientiert worden ist, um das anatomische Röntgenbild des nächsten Subjektes mit dem anatomischen Röntgenreferenzbild auszurichten.

**5.** Verfahren zum Einstellen einer physikalisch räumlichen Orientierung von mindestens einem immobilisierten Subjekt in einem Röntgenbildgebungssystem (100), welches einen Computer (20) aufweist, um im Wesentlichen die physikalisch räumliche Orientierung eines weiteren immobilisierten Referenzsubjektes wiederzugeben, welches Folgendes aufweist:

Ausführen einer physikalisch räumlichen Orientierung (1000) des Referenzsubjektes;
Aufnehmen (1010) eines anatomischen Röntgenbildes des Referenzsubjektes unter Verwendung des Computers (20);
Ausführen einer physikalisch räumlichen Orientierung (1000) des mindestens einen Subjektes;
Aufnehmen (1010) eines anatomischen Röntgenbildes des mindestens einen Subjektes unter Verwendung des Computers (20);
Analysieren (1020) der Kombination des anatomischen Röntgenbildes des Referenzsubjektes und des anatomischen Röntgenbildes des mindestens einen Subjektes unter Verwendung des Computers (20); und
folgend auf das Analysieren physikalisch räumliches Reorientieren (1030) des mindestens einen Subjektes, um im Wesentlichen die physikalisch räumliche Orientierung des Referenzsubjektes wiederzugeben.

**6.** Verfahren nach Anspruch 5, wobei das Röntgenbildgebungssystem (100) ferner ein multimodales System ist, wobei das Verfahren auch aufweist, einen Satz von multimodalen Molekularbildern des mindestens einen immobilisierten Subjektes aufzunehmen, welches eine im Wesentlichen reproduzierte physikalisch räumliche Orientierung hat, und zwar zum Vergleich mit einem entsprechenden Satz von multimodalen Molekularbildern des immobilisierten Referenzsubjektes, wobei das Verfahren weiter Folgendes aufweist:

Aufnehmen (1040) eines Satzes von multimodalen Molekularbildern des Referenzsubjektes;
folgend auf die Reorientierung, Aufnehmen (1040) eines Satzes von multimodalen Molekularbildern des mindestens einen Subjektes; und
Vergleichen der multimodalen Molekularbilder des mindestens einen Subjektes mit den multimodalen Molekularbildern des Referenzsubjektes.

## Revendications

**1.** Procédé pour reproduire l’orientation spatiale physique d’un sujet immobilisé dans un système d’imagerie à rayons X (100) comprenant un ordinateur (20), à partir d’un sujet donné pour un autre sujet, comprenant :

réaliser une orientation spatiale physique (600) d’un premier sujet immobilisé (500a) dans le système d’imagerie multimodal (100) ;
utiliser l’ordinateur (20), acquérir (610) une image anatomique de rayons X du premier sujet immobilisé (500a) dans le système d’imagerie (100) ;
réaliser une orientation spatiale physique (630) d’un sujet immobilisé suivant (500b) dans le système d’imagerie (100) ;
utiliser l’ordinateur (20), acquérir (640) une image anatomique de rayons X de test du sujet immobilisé suivant (500b) dans le système d’imagerie (100) ;
utiliser l’ordinateur (20), comparer (650) l’image anatomique de rayons X de test du sujet immobilisé suivant (500b) et l’image anatomique de rayons X du premier sujet immobilisé (500a), comprenant un calcul de la différence entre les deux ;
réorienter spatialement physiquement (660) le sujet immobilisé suivant (500b) pour améliorer la comparaison, si la comparaison n’est pas satisfaisante pour démontrer une reproduction de l’orientation spatiale physique du premier sujet immobilisé (500a) ;
répéter les étapes consistant à réaliser une orientation spatiale physique (630) d’un sujet immobilisé suivant (500b), acquérir (640) une image anatomique de rayons X de test, comparer (650) et réorienter spatialement physiquement (660), jusqu’à ce que la comparaison soit satisfaisante ; et
acquérir (640) une image anatomique de rayons X du sujet immobilisé suivant (500b) dans le système d’imagerie multimodal (100).

**2.** Procédé selon la revendication 1, dans lequel la première image anatomique de rayons X et la suivante sont numériques et l’étape de réorientation (660) comprend :

appliquer une quantification vectorielle (700) aux images anatomiques de rayons X numériques du premier sujet (500a) et du sujet suivant (500b) pour convertir les images anatomiques de rayons X numériques en images anatomiques de rayons X vectorisées ayant des informations d’intensité locale correspondantes dérivées respectivement des images anatomiques de rayons X numériques ;
obtenir (710) une représentation statistique conjointe des images anatomiques de rayons X numériques en utilisant les images anatomiques de rayons X vectorisées ;

calculer (720) une fonction de coût en utilisant la représentation statistique conjointe des images anatomiques de rayons X numériques ;
sélectionner (730) la première image anatomique de rayons X comme image anatomique de rayons X de référence parmi les images anatomiques de rayons X numériques ;
évaluer (740) la fonction de coût ;
réorienter spatialement physiquement (750) le sujet suivant (500b) en fonction de sa correspondance spatiale virtuelle avec l'image anatomique de rayons X de référence, un critère de fonction de coût prédéterminé étant non satisfait ; et
répéter les étapes consistant à appliquer (700), obtenir (710), calculer (720), sélectionner (730), évaluer (740) et réorienter spatialement physiquement (750), le critère de fonction de coût prédéterminé étant non satisfait, pour le sujet suivant (500b) qui a été précédemment réorienté spatialement physiquement (750) afin d'aligner l'image anatomique de rayons X du sujet suivant (500b) sur l'image anatomique de rayons X de référence.

**3.** Procédé selon la revendication 1, dans lequel l'étape consistant à réorienter spatialement physiquement (660) comprend :

calculer la différence (800) entre l'image anatomique de rayons X de test du sujet suivant (500b) et l'image anatomique de rayons X du premier sujet (500a) ;
comparer la différence (810, 820) à une image nulle (zéro) ;
réorienter spatialement physiquement (830) le sujet suivant (500b) en fonction de sa correspondance spatiale virtuelle avec l'image anatomique de rayons X du premier sujet (500a) ; et
répéter les étapes consistant à calculer la différence (800), comparer la différence (810, 820), et réorienter physiquement spatialement (830) le sujet suivant (500b) jusqu'à ce que le critère de différence d'image nulle (zéro) soit non satisfait.

**4.** Procédé selon la revendication 1, dans lequel l'étape consistant à réorienter spatialement physiquement (660) comprend :

sélectionner la première image anatomique de rayons X du premier sujet comme image de référence ;
appliquer un algorithme de recalage d'image à l'image anatomique de rayons X du premier sujet et à l'image anatomique de rayons X de test du sujet suivant ;
obtenir une valeur de fonction de coût minimale à partir de l'application de l'algorithme de recalage d'image ;
obtenir une carte de déplacement spatial virtuel correspondant à la fonction de coût minimale à partir de l'application de l'algorithme de recalage d'image ;
évaluer la fonction de coût ;
réorienter spatialement physiquement le sujet en fonction de la carte de déplacement spatial virtuel, un critère de fonction de coût prédéterminé étant non satisfait ; et
répéter les étapes consistant à sélectionner, appliquer, obtenir une valeur de fonction de coût minimale, obtenir une carte de déplacement spatial virtuel, évaluer et réorienter spatialement physiquement, le critère de fonction de coût prédéterminé étant non satisfait, pour le sujet suivant qui a été précédemment réorienté spatialement physiquement afin d'aligner l'image anatomique de rayons X du sujet suivant sur l'image anatomique de rayons X de référence.

**5.** Procédé d'ajustement d'une orientation spatiale physique d'au moins un sujet immobilisé dans un système d'imagerie à rayons X (100) comprenant un ordinateur (20), de façon à sensiblement reproduire l'orientation spatiale physique d'un autre sujet immobilisé de référence, comprenant :

réaliser une orientation spatiale physique (1000) du sujet de référence ;
utiliser l'ordinateur (20), acquérir (1010) une image anatomique de rayons X du sujet de référence ;
réaliser une orientation spatiale physique (1000) dudit au moins un sujet ;
utiliser l'ordinateur (20), acquérir (1010) une image anatomique de rayons X dudit au moins un sujet ;
utiliser l'ordinateur (20), analyser (1020) la combinaison de l'image anatomique de rayons X du sujet de référence et de l'image anatomique de rayons X dudit au moins un sujet ; et
à la suite de l'analyse, réorienter spatialement, physiquement (1030) ledit au moins un sujet de façon à reproduire sensiblement l'orientation spatiale physique du sujet de référence.

**6.** Procédé selon la revendication 5, dans lequel le système d'imagerie à rayons X (100) est en outre un système multimodal, le procédé comprend aussi l'acquisition d'un ensemble d'images moléculaires multimodales dudit au moins un sujet immobilisé, ayant une orientation spatiale physique sensiblement reproduite de la sorte, pour comparaison avec un ensemble correspondant d'images moléculaires multimodales du sujet immobilisé de référence, comprenant en outre :

acquérir (1040) un ensemble d'images moléculaires multimodales du sujet de référence ;
à la suite de la réorientation, acquérir (1040) un ensemble d'images moléculaires multimodales dudit au moins un sujet ; et
comparer les images moléculaires multimodales dudit au moins un sujet aux images moléculaires multimodales du sujet de référence.

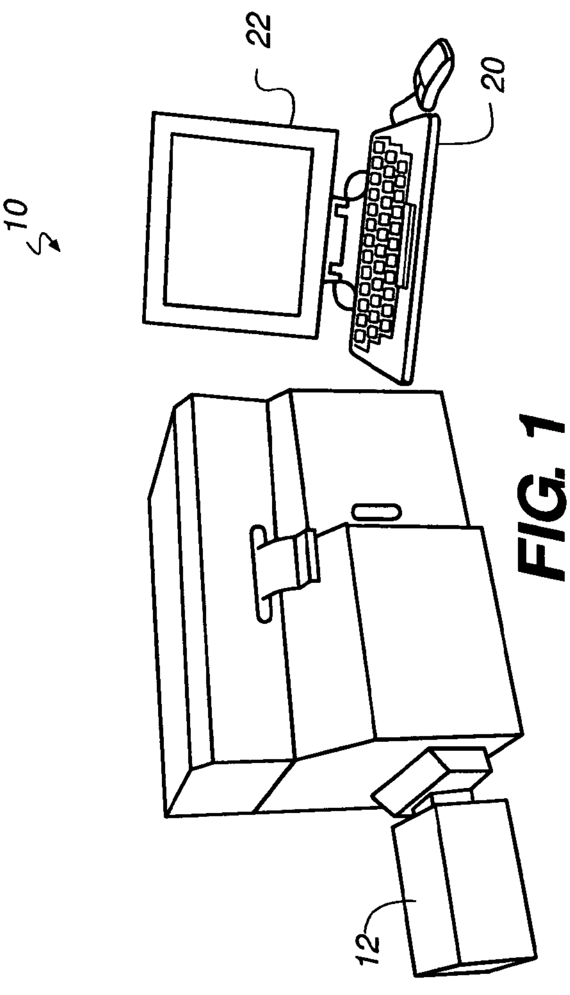


FIG. 1
(PRIOR ART)

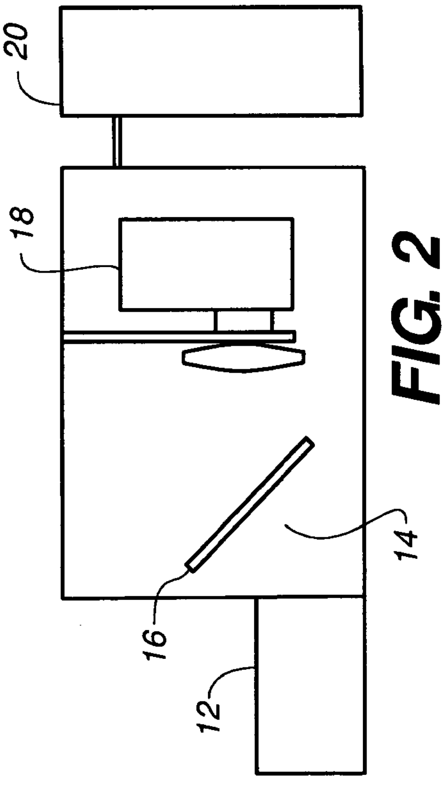


FIG. 2
(PRIOR ART)

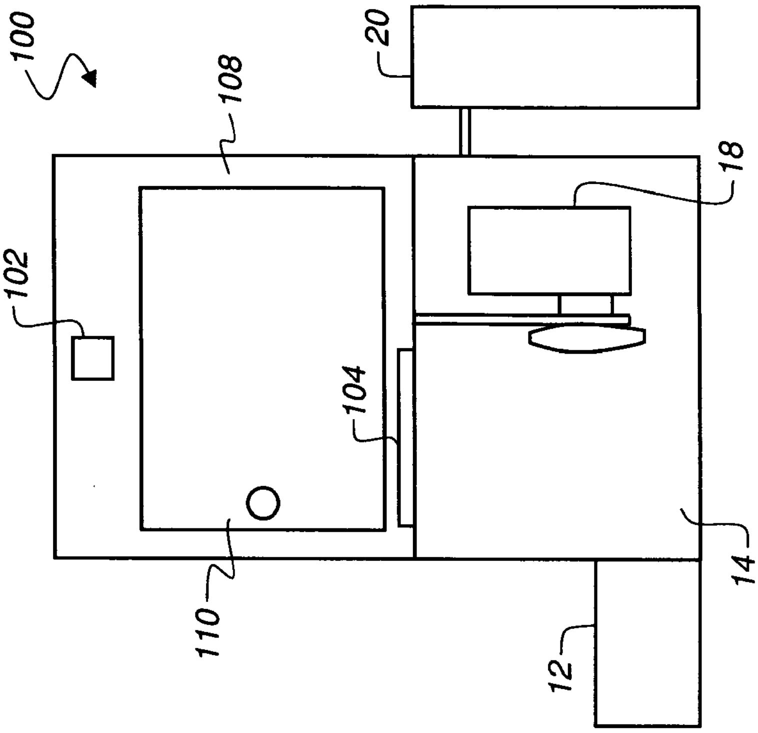


FIG. 3B

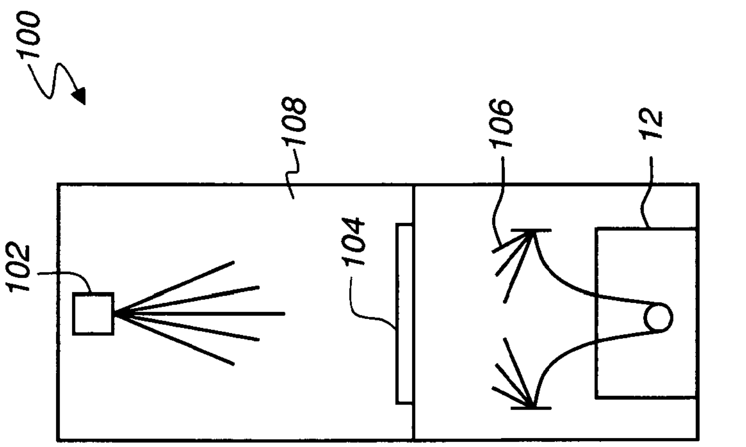


FIG. 3A

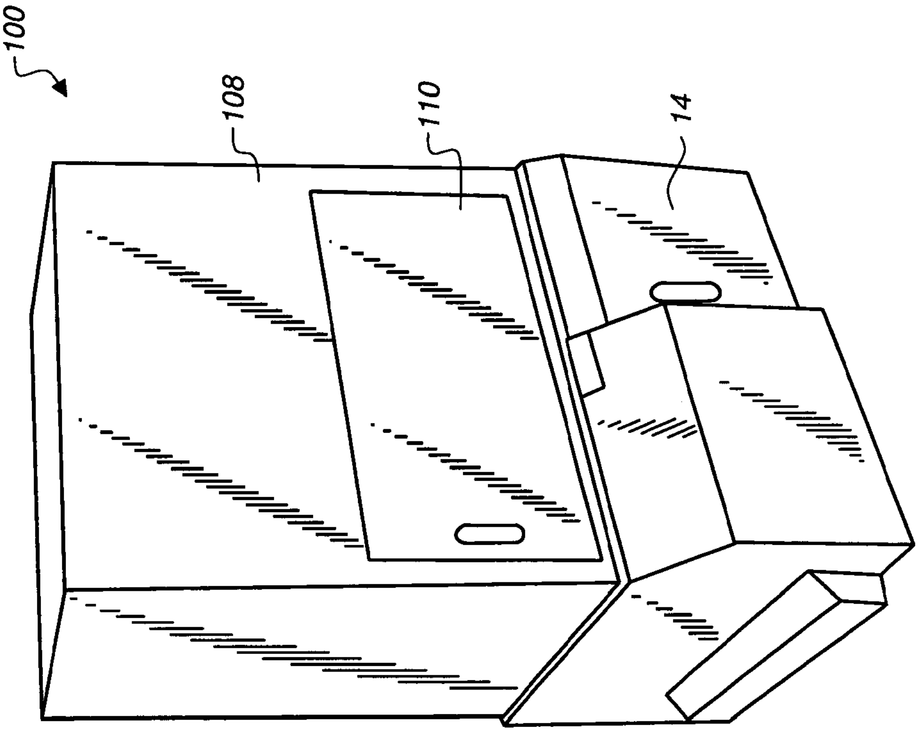


FIG. 4

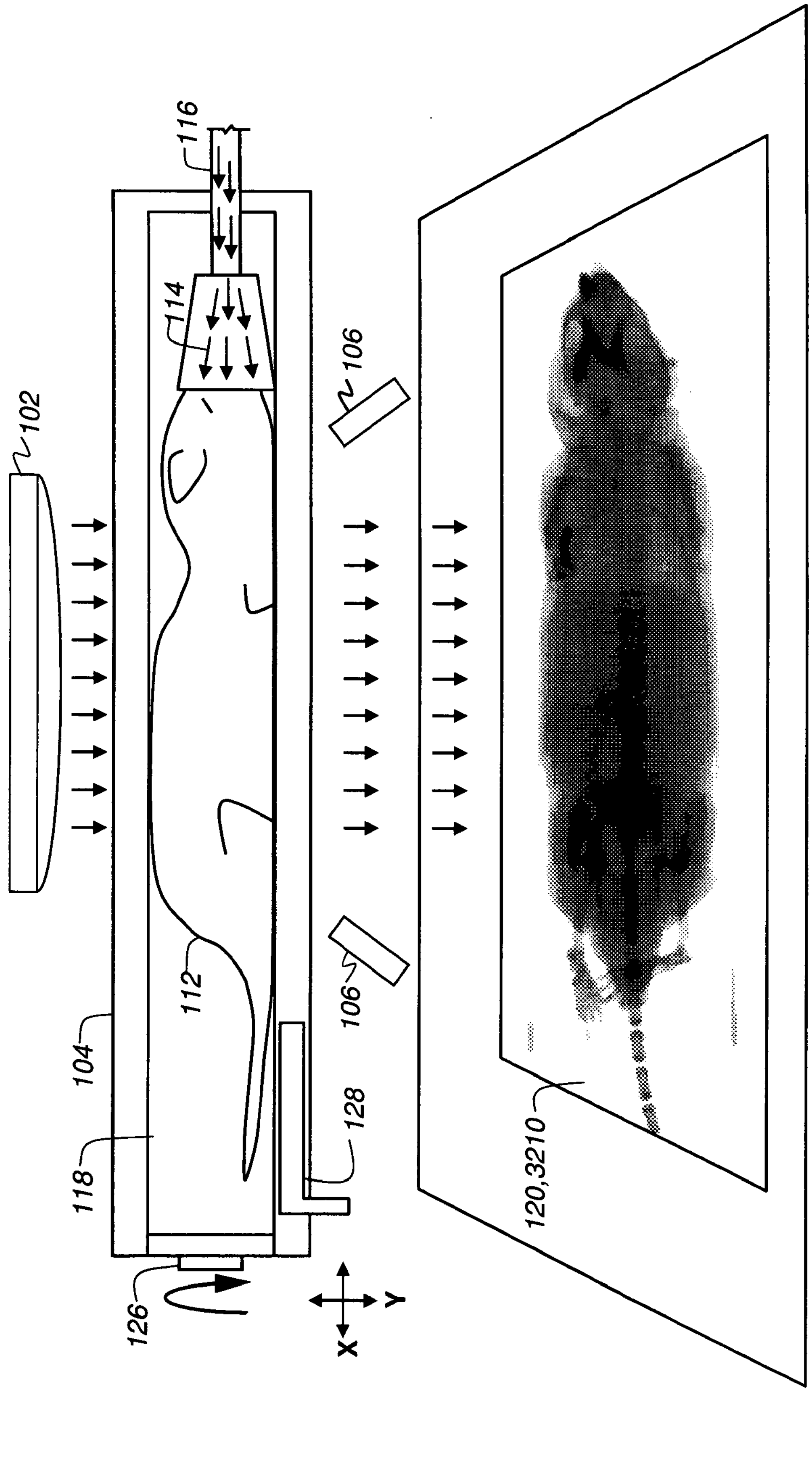


FIRST-TME X-RAY ANATOMICAL IMAGE; OR VIRTUALLY, SPATIALLY REORIENTED NEXT-TIME X-RAY ANATOMICAL IMAGE (AFTER SPATIAL TRANSFORMATION OF X-RAY ANATOMICAL IMAGE 3200)

**FIG. 5A**

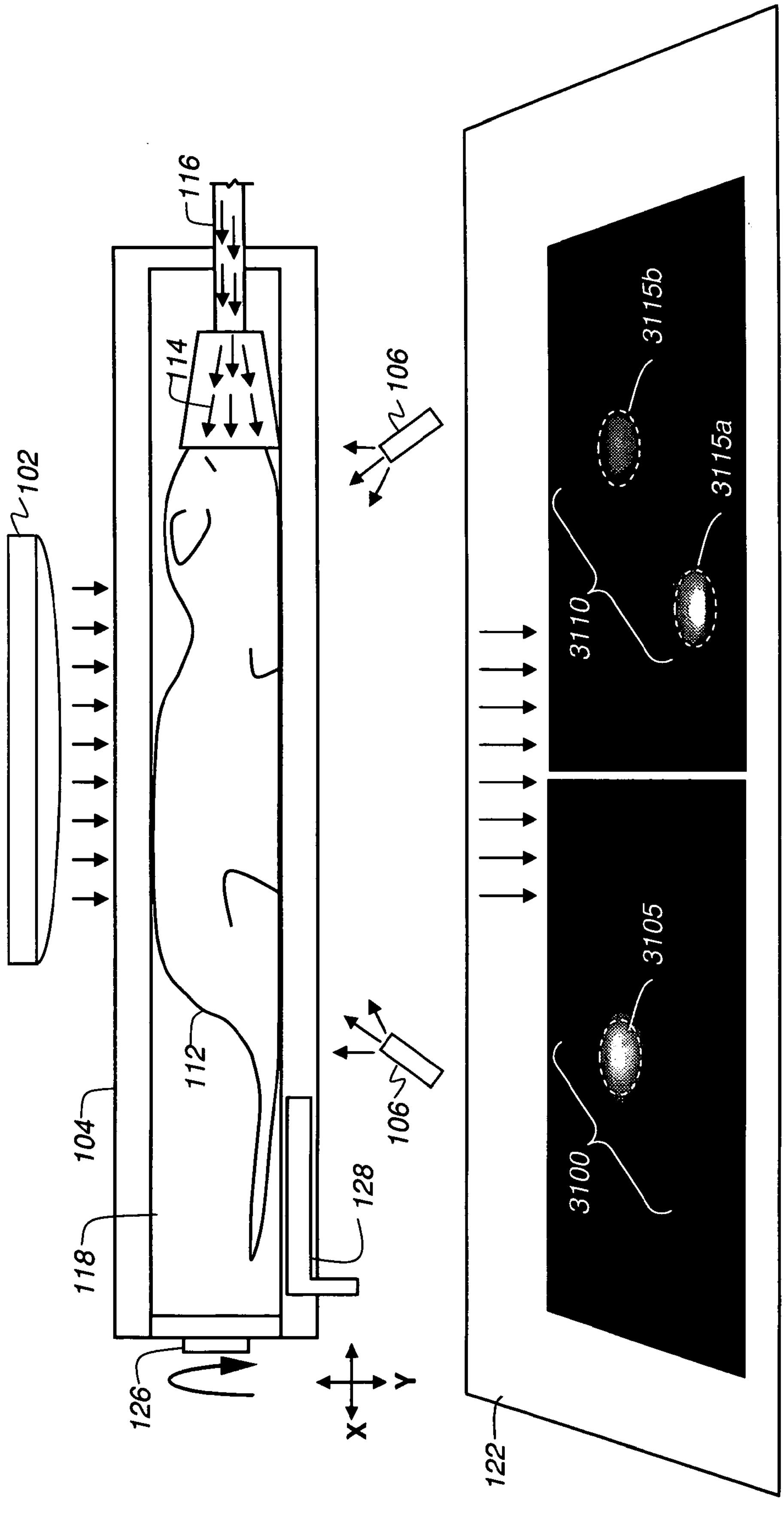


FIRST-TIME SET OF MULTIMODAL MOLECULAR IMAGES

**FIG. 5B**

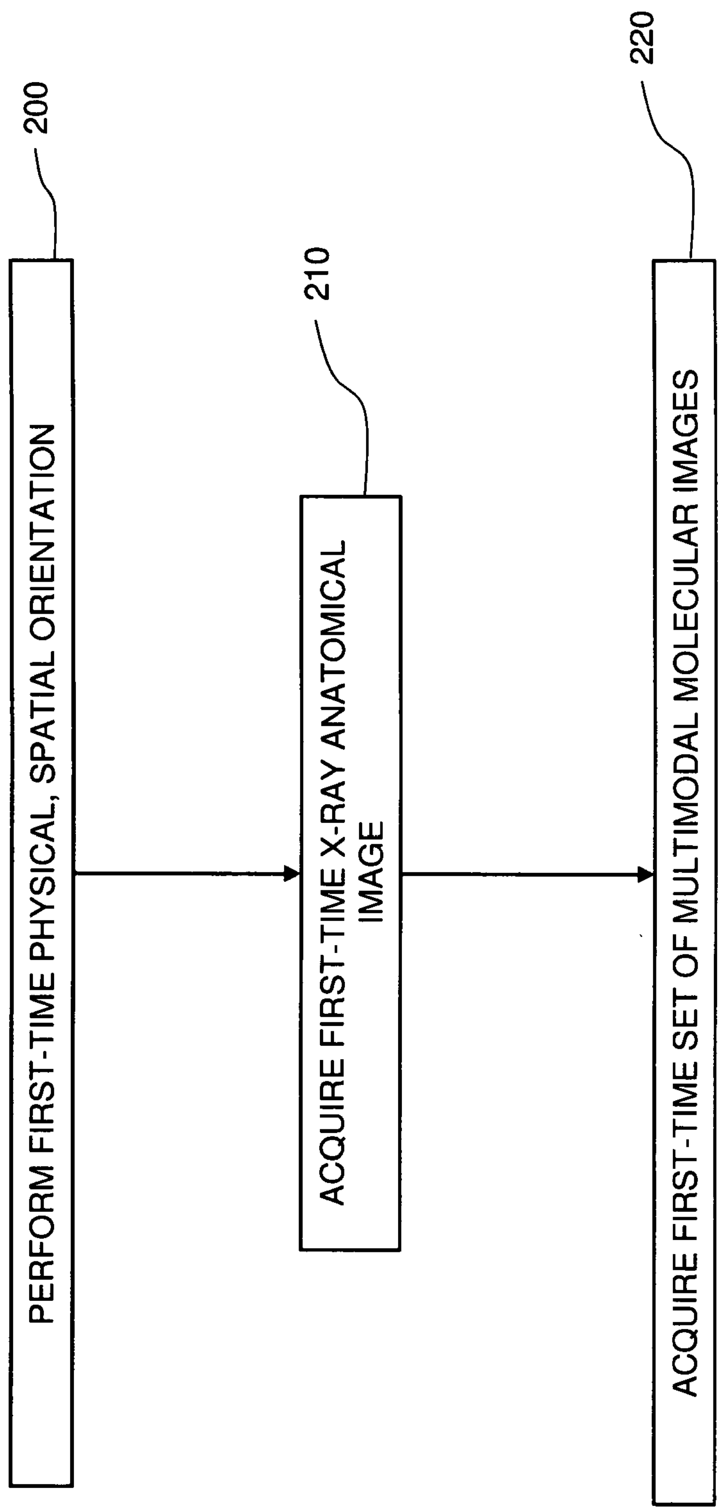
200
PERFORM FIRST-TIME PHYSICAL, SPATIAL ORIENTATION
210
ACQUIRE FIRST-TIME X-RAY ANATOMICAL IMAGE
220
ACQUIRE FIRST-TIME SET OF MULTIMODAL MOLECULAR IMAGES

FIG. 6

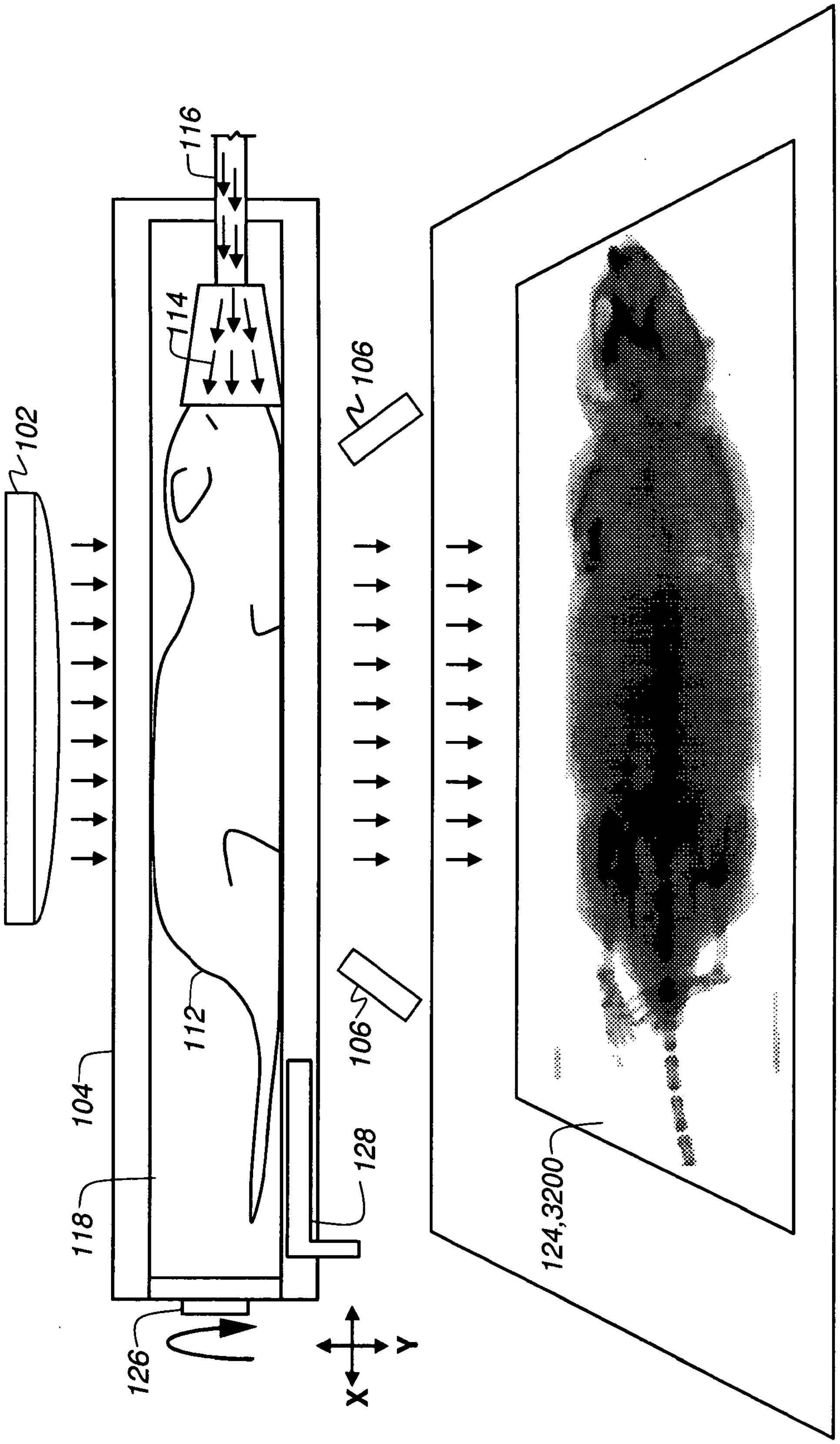


NEXT-TIME TEST X-RAY ANATOMICAL IMAGE

FIG. 7A

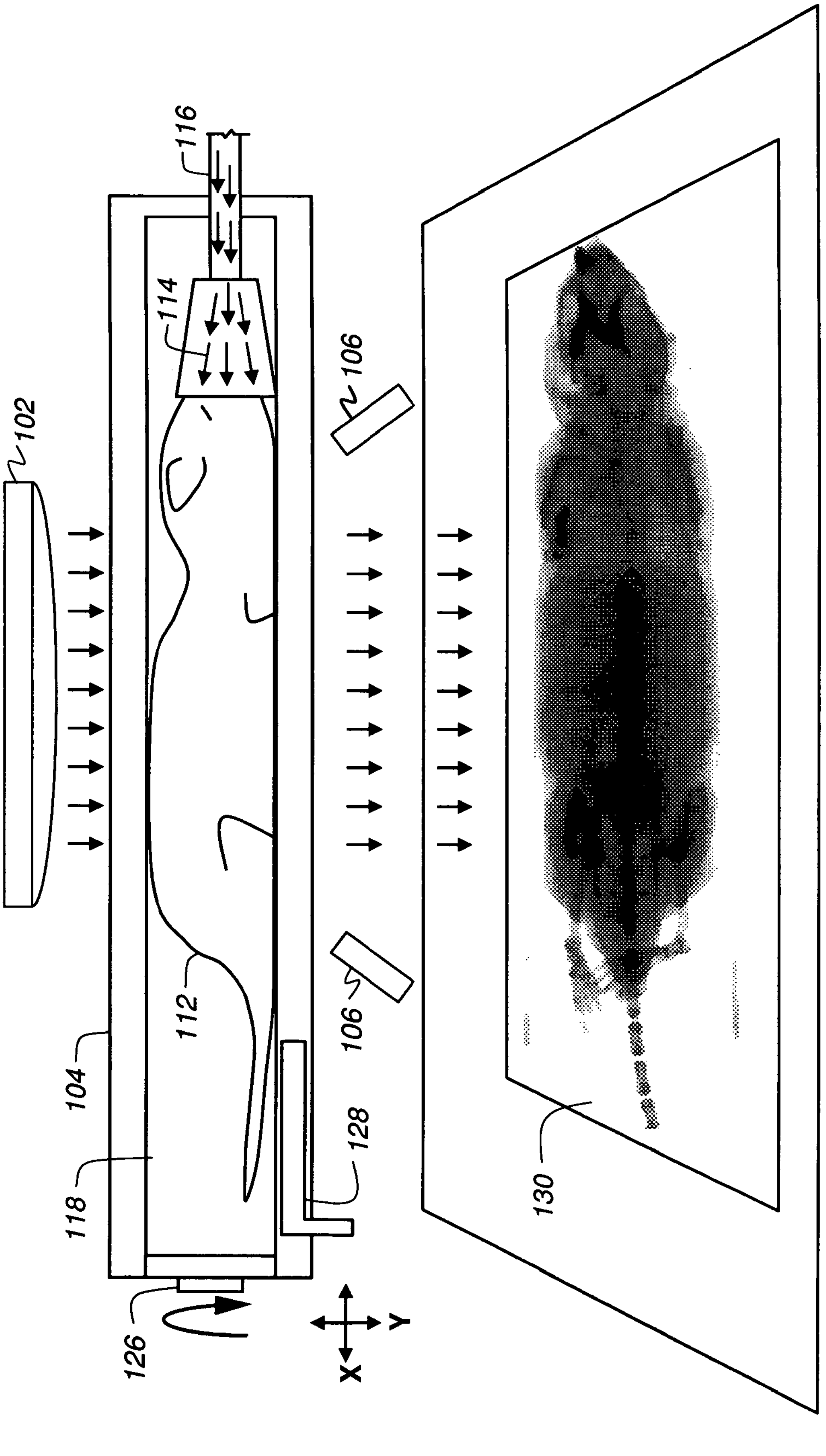


NEXT-TIME X-RAY ANATOMICAL IMAGE (AFTER PHYSICAL, SPATIAL REORIENTATION)

*FIG. 7B*

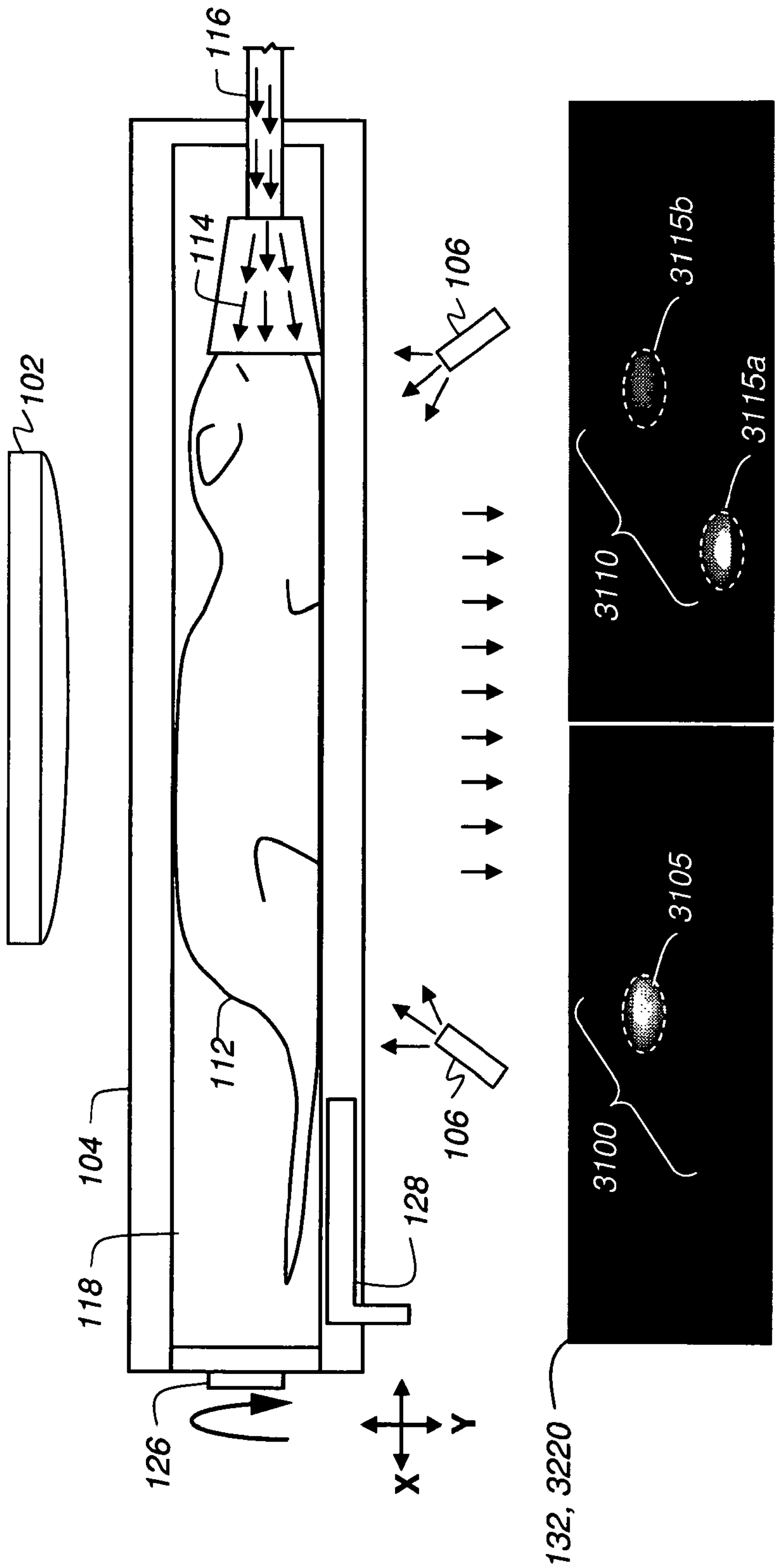


NEXT-TIME SET OF MULTIMODAL MOLECULAR IMAGES; OR VIRTUALLY, SPATIALLY REORIENTED NEXT-TIME SET OF MULTIMODAL MOLECULAR IMAGES (ACHIEVED BY USING SAME VIRTUAL, SPATIAL REORIENTATION AS THE VIRTUALLY, SPATIALLY REORIENTED NEXT-TIME X-RAY ANATOMICAL IMAGE 3210)

*FIG. 7C*

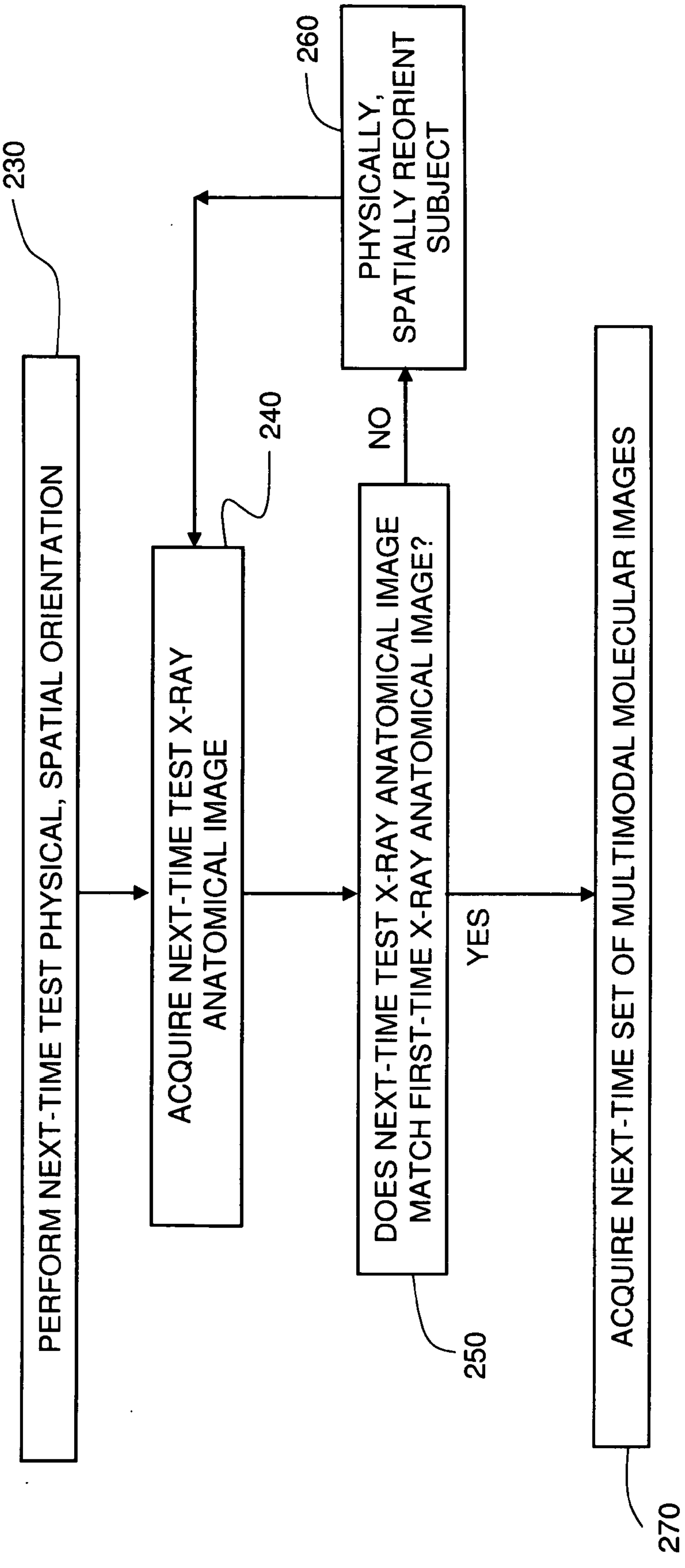
PERFORM NEXT-TIME TEST PHYSICAL, SPATIAL ORIENTATION
230
ACQUIRE NEXT-TIME TEST X-RAY ANATOMICAL IMAGE
240
DOES NEXT-TIME TEST X-RAY ANATOMICAL IMAGE MATCH FIRST-TIME X-RAY ANATOMICAL IMAGE?
250
NO
PHYSICALLY, SPATIALLY REORIENT SUBJECT
260
YES
ACQUIRE NEXT-TIME SET OF MULTIMODAL MOLECULAR IMAGES
270

FIG. 8

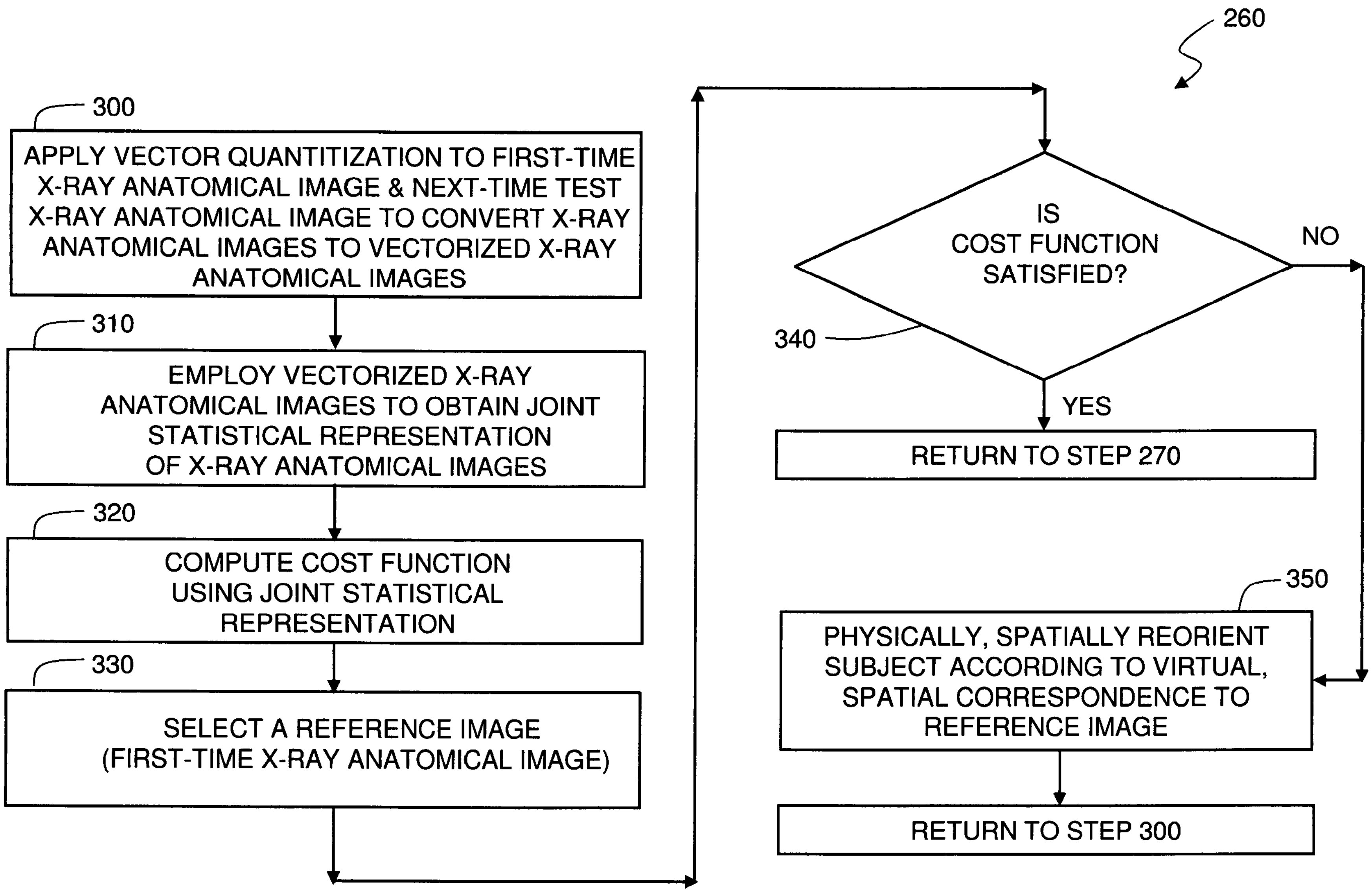
260
300
APPLY VECTOR QUANTITIZATION TO FIRST-TIME X-RAY ANATOMICAL IMAGE & NEXT-TIME TEST X-RAY ANATOMICAL IMAGE TO CONVERT X-RAY ANATOMICAL IMAGES TO VECTORIZED X-RAY ANATOMICAL IMAGES
310
EMPLOY VECTORIZED X-RAY ANATOMICAL IMAGES TO OBTAIN JOINT STATISTICAL REPRESENTATION OF X-RAY ANATOMICAL IMAGES
320
COMPUTE COST FUNCTION USING JOINT STATISTICAL REPRESENTATION
330
SELECT A REFERENCE IMAGE (FIRST-TIME X-RAY ANATOMICAL IMAGE)
340
IS COST FUNCTION SATISFIED?
NO
YES
RETURN TO STEP 270
350
PHYSICALLY, SPATIALLY REORIENT SUBJECT ACCORDING TO VIRTUAL, SPATIAL CORRESPONDENCE TO REFERENCE IMAGE
RETURN TO STEP 300

FIG. 9

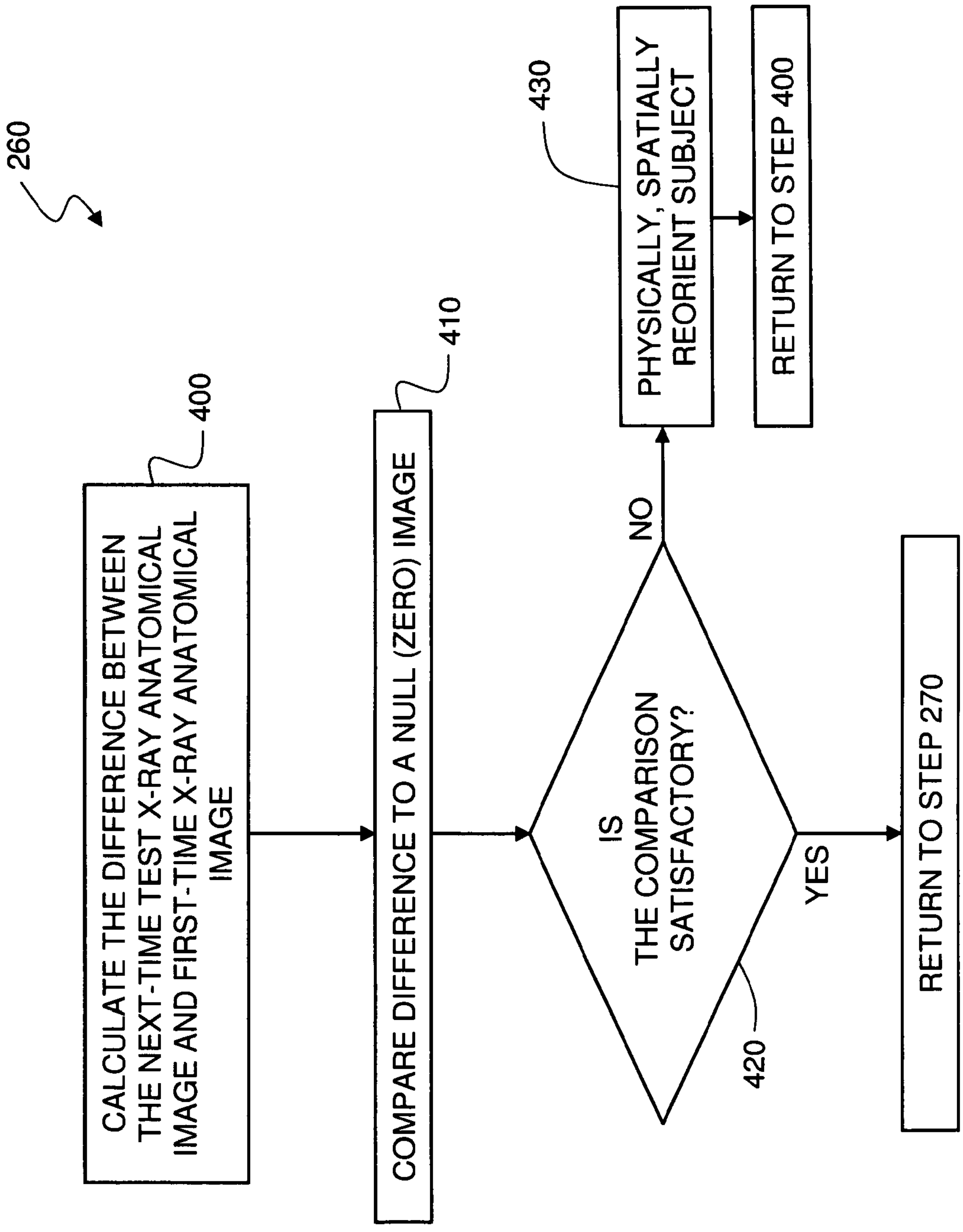
260
CALCULATE THE DIFFERENCE BETWEEN THE NEXT-TIME TEST X-RAY ANATOMICAL IMAGE AND FIRST-TIME X-RAY ANATOMICAL IMAGE
400
COMPARE DIFFERENCE TO A NULL (ZERO) IMAGE
410
IS THE COMPARISON SATISFACTORY?
420
NO
YES
PHYSICALLY, SPATIALLY REORIENT SUBJECT
430
RETURN TO STEP 400
RETURN TO STEP 270

FIG. 10

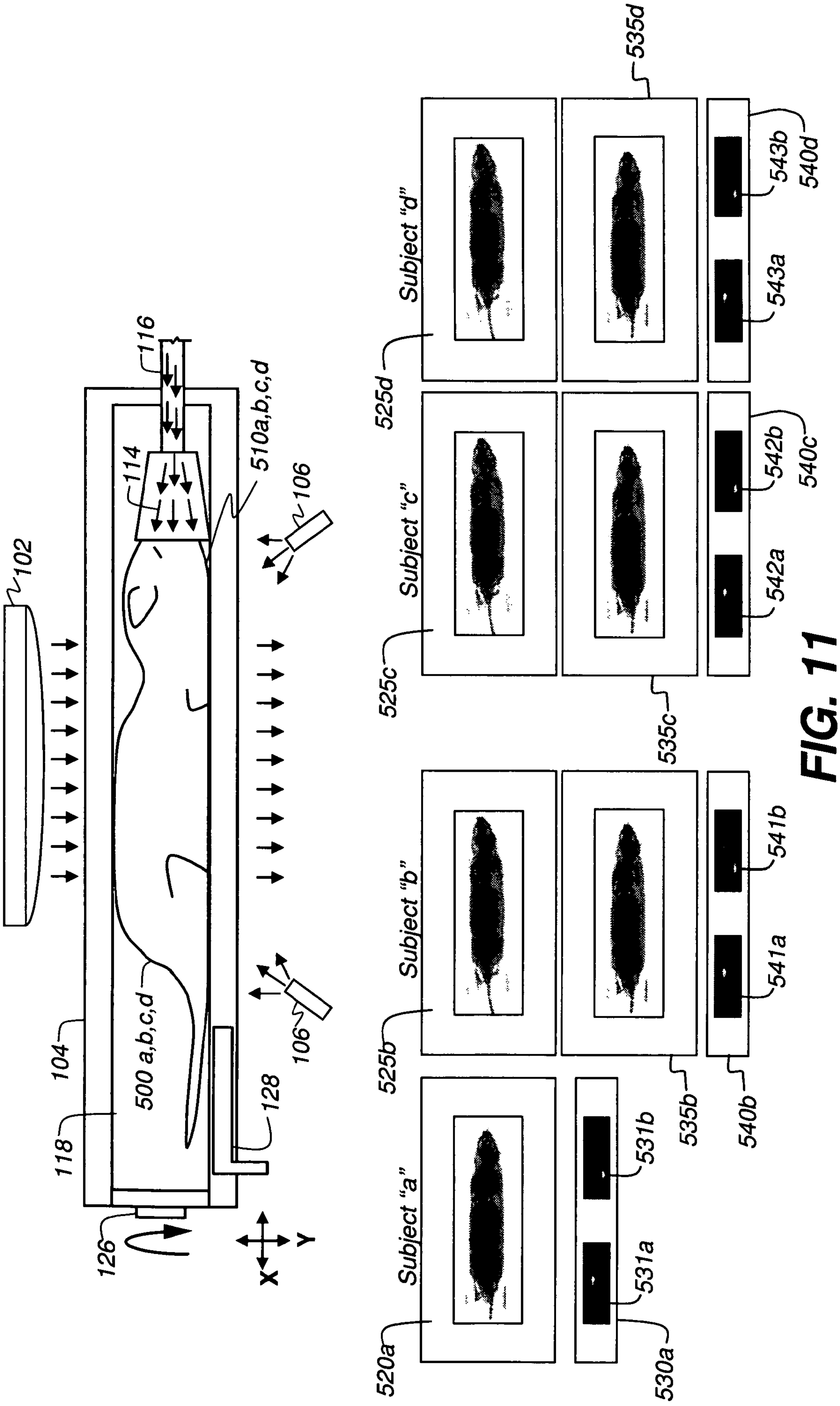
102
104
106
106
114
116
118
126
128
X
Y
500 a,b,c,d
510a,b,c,d
Subject "a"
Subject "b"
Subject "c"
Subject "d"
520a
525b
525c
525d
530a
531a
531b
535b
535c
535d
540b
540c
540d
541a
541b
542a
542b
543a
543b

FIG. 11

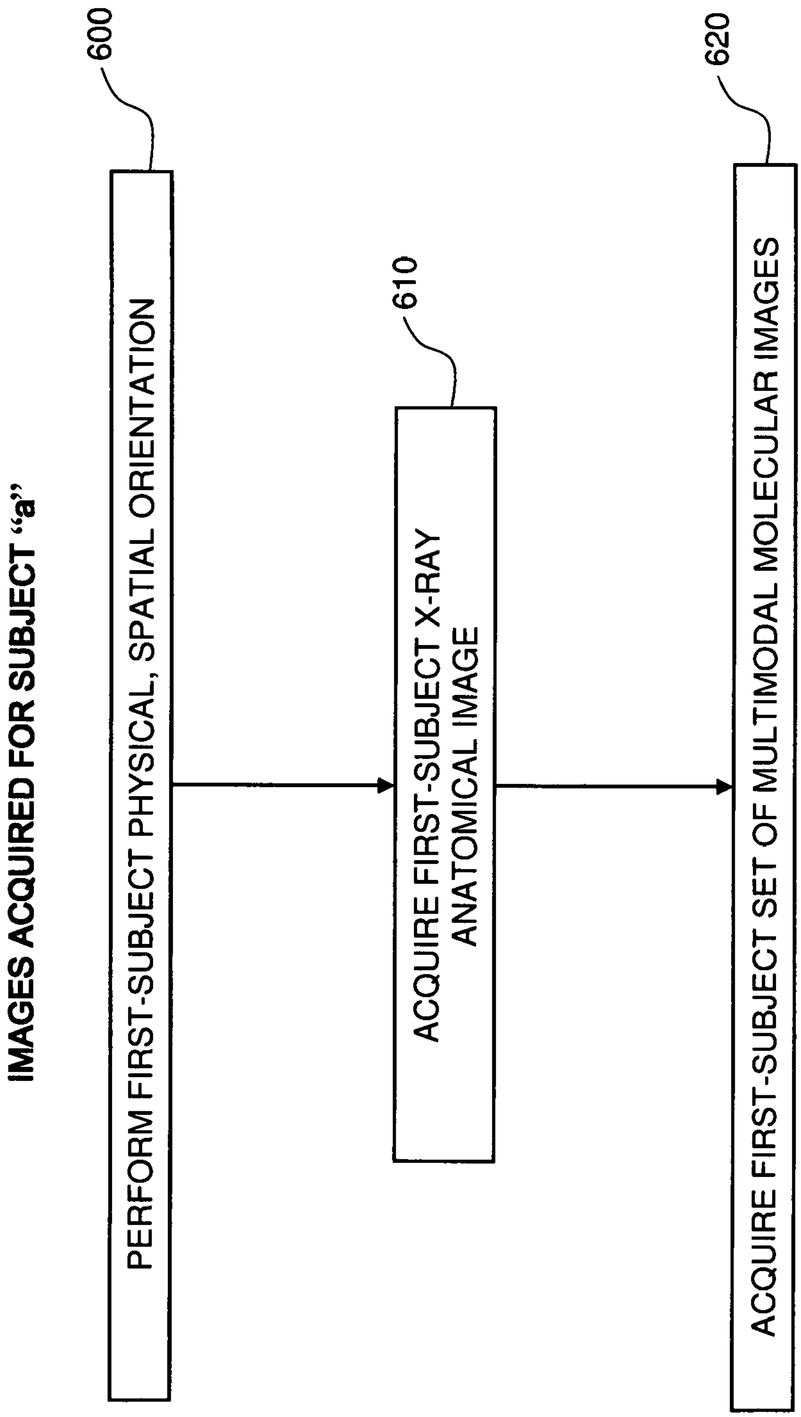
IMAGES ACQUIRED FOR SUBJECT "a"
600
PERFORM FIRST-SUBJECT PHYSICAL, SPATIAL ORIENTATION
610
ACQUIRE FIRST-SUBJECT X-RAY ANATOMICAL IMAGE
620
ACQUIRE FIRST-SUBJECT SET OF MULTIMODAL MOLECULAR IMAGES

FIG. 12

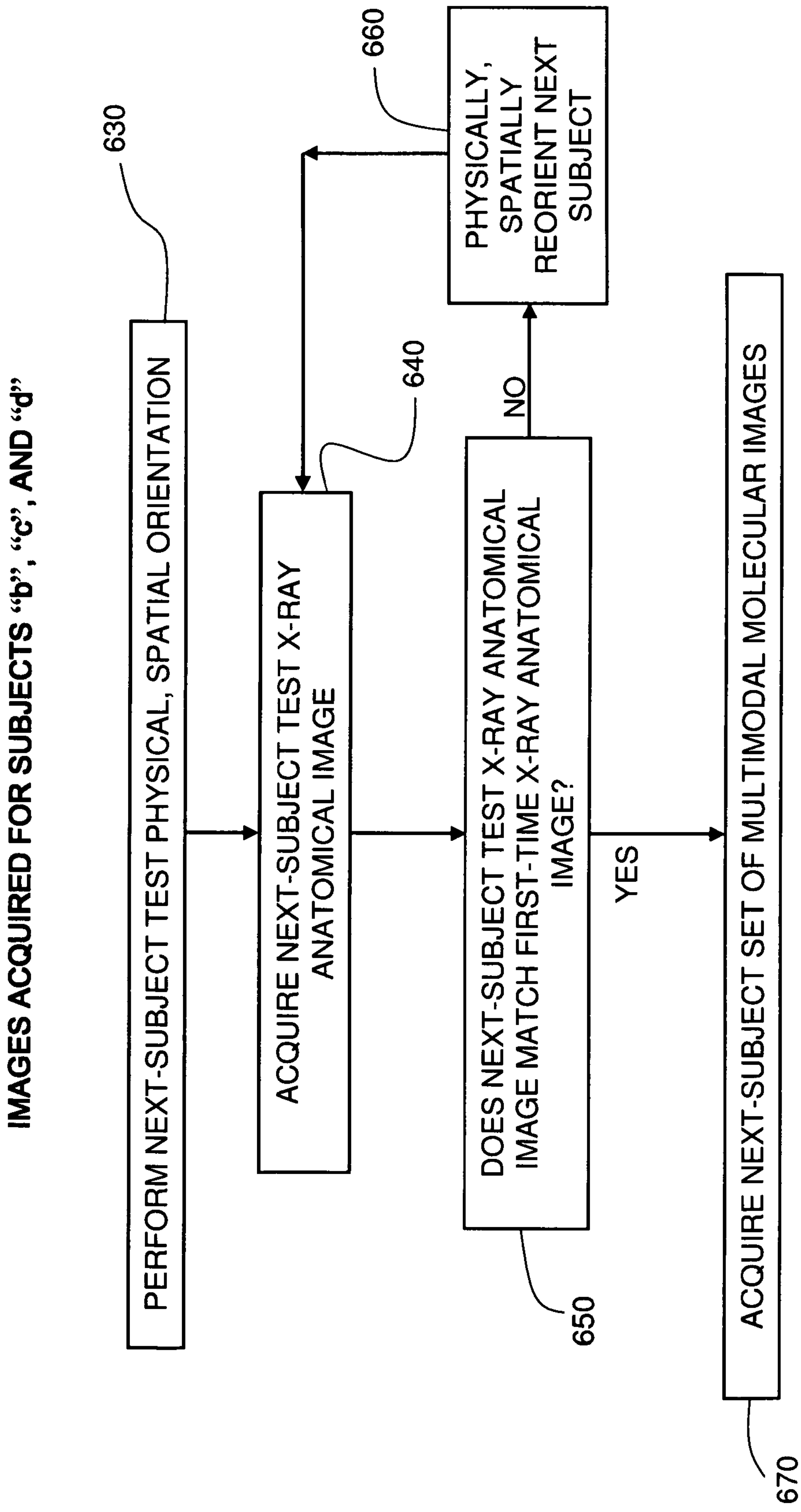
IMAGES ACQUIRED FOR SUBJECTS “b”, “c”, AND “d”
PERFORM NEXT-SUBJECT TEST PHYSICAL, SPATIAL ORIENTATION
630
ACQUIRE NEXT-SUBJECT TEST X-RAY ANATOMICAL IMAGE
640
DOES NEXT-SUBJECT TEST X-RAY ANATOMICAL IMAGE MATCH FIRST-TIME X-RAY ANATOMICAL IMAGE?
650
NO
YES
PHYSICALLY, SPATIALLY REORIENT NEXT SUBJECT
660
ACQUIRE NEXT-SUBJECT SET OF MULTIMODAL MOLECULAR IMAGES
670

FIG. 13

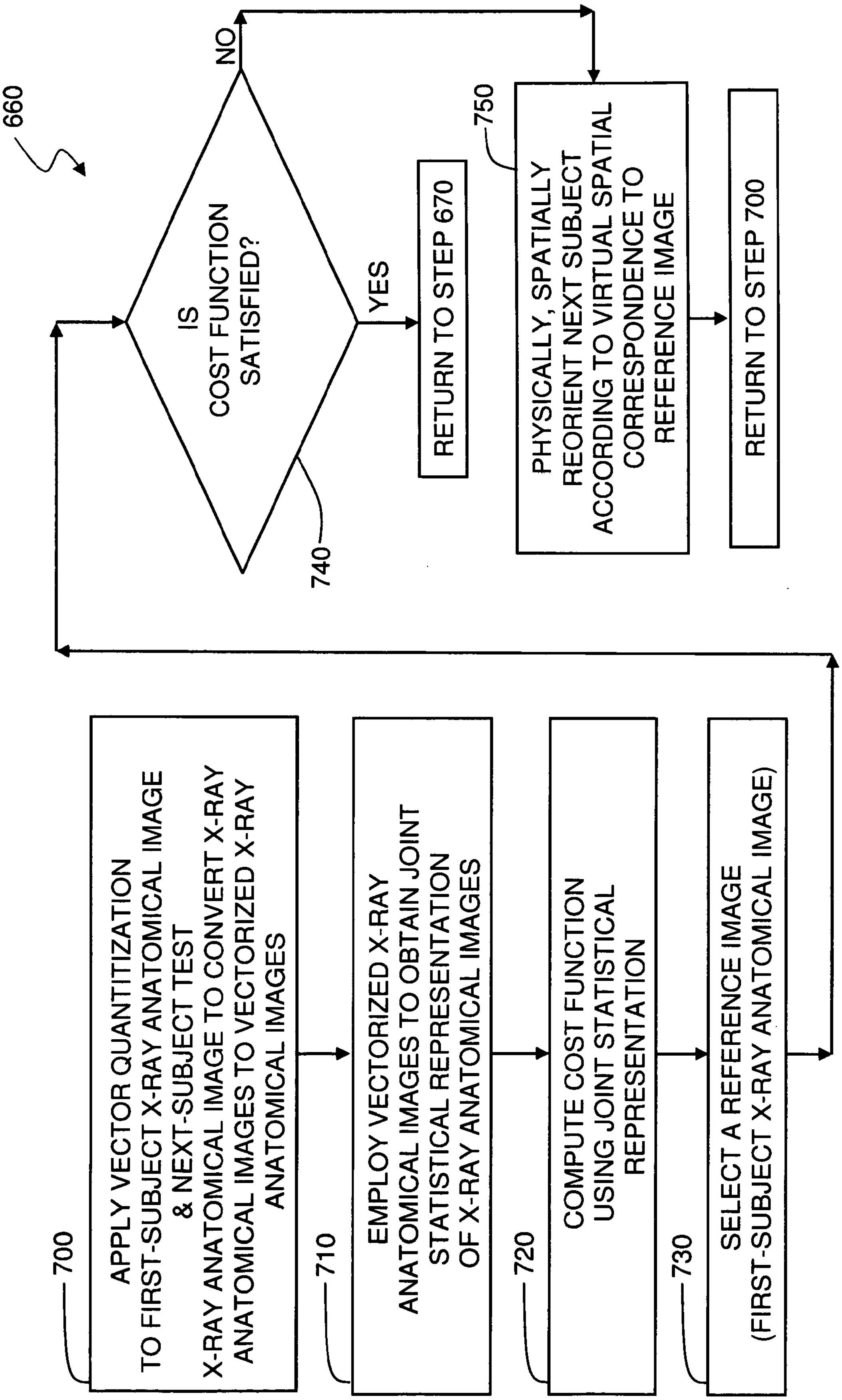
660
700
APPLY VECTOR QUANTITIZATION TO FIRST-SUBJECT X-RAY ANATOMICAL IMAGE & NEXT-SUBJECT TEST X-RAY ANATOMICAL IMAGE TO CONVERT X-RAY ANATOMICAL IMAGES TO VECTORIZED X-RAY ANATOMICAL IMAGES
710
EMPLOY VECTORIZED X-RAY ANATOMICAL IMAGES TO OBTAIN JOINT STATISTICAL REPRESENTATION OF X-RAY ANATOMICAL IMAGES
720
COMPUTE COST FUNCTION USING JOINT STATISTICAL REPRESENTATION
730
SELECT A REFERENCE IMAGE (FIRST-SUBJECT X-RAY ANATOMICAL IMAGE)
740
IS COST FUNCTION SATISFIED?
YES
NO
RETURN TO STEP 670
750
PHYSICALLY, SPATIALLY REORIENT NEXT SUBJECT ACCORDING TO VIRTUAL SPATIAL CORRESPONDENCE TO REFERENCE IMAGE
RETURN TO STEP 700

FIG. 14

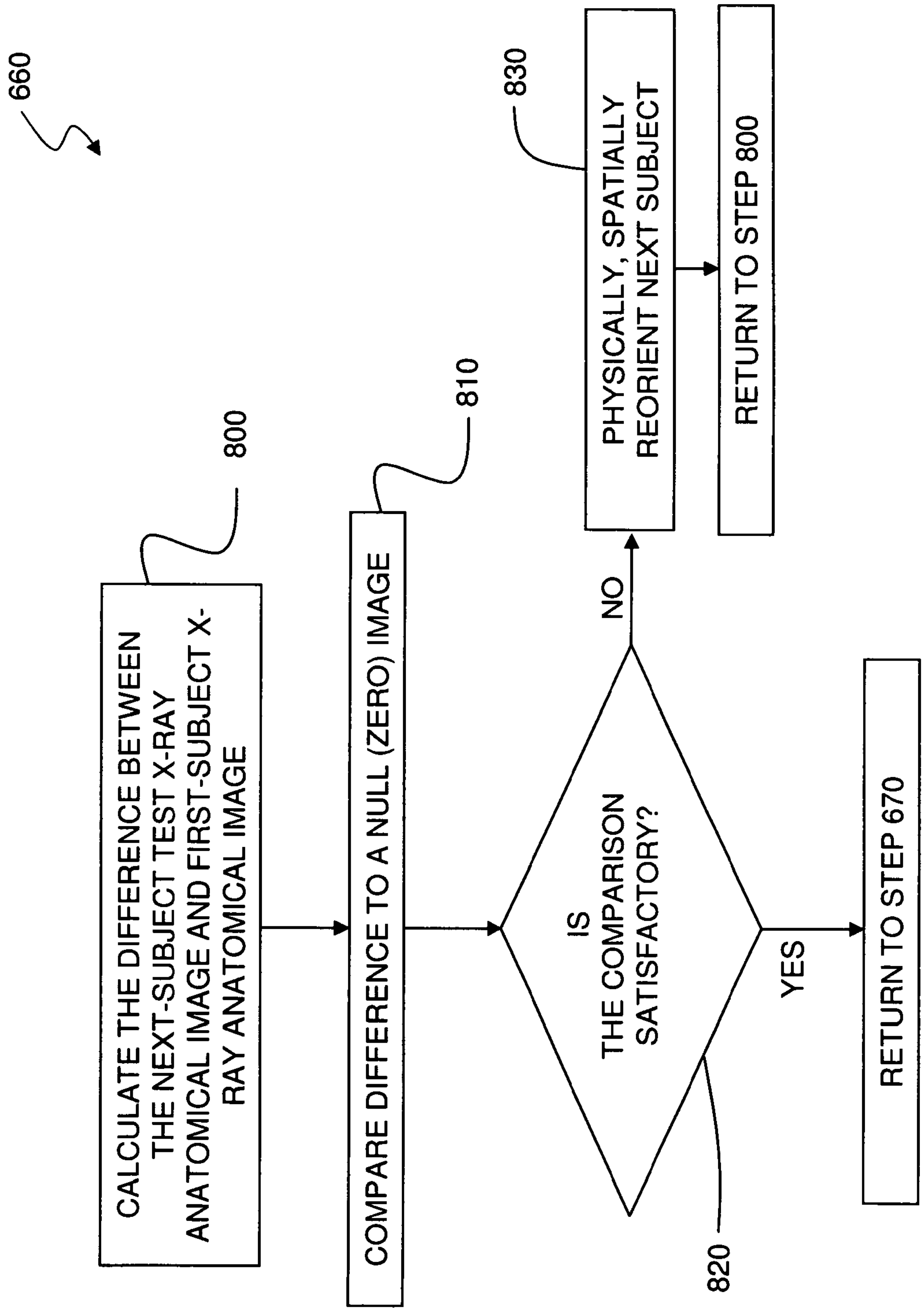
660
CALCULATE THE DIFFERENCE BETWEEN THE NEXT-SUBJECT TEST X-RAY ANATOMICAL IMAGE AND FIRST-SUBJECT X-RAY ANATOMICAL IMAGE
800
COMPARE DIFFERENCE TO A NULL (ZERO) IMAGE
810
IS THE COMPARISON SATISFACTORY?
820
NO
YES
PHYSICALLY, SPATIALLY REORIENT NEXT SUBJECT
830
RETURN TO STEP 800
RETURN TO STEP 670

FIG. 15

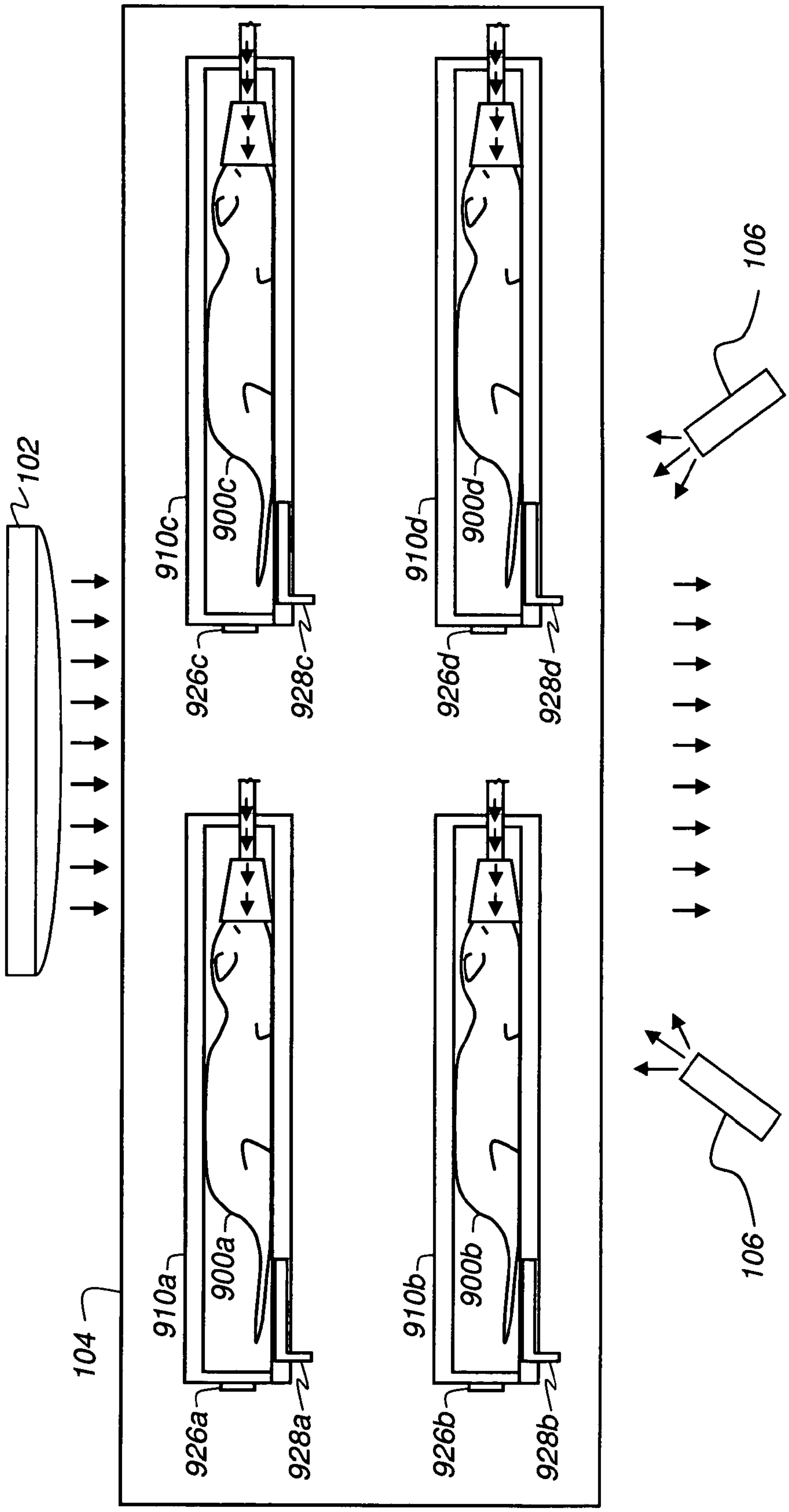
102
104
106
106
910a
900a
926a
928a
910b
900b
926b
928b
910c
900c
926c
928c
910d
900d
926d
928d

FIG. 16

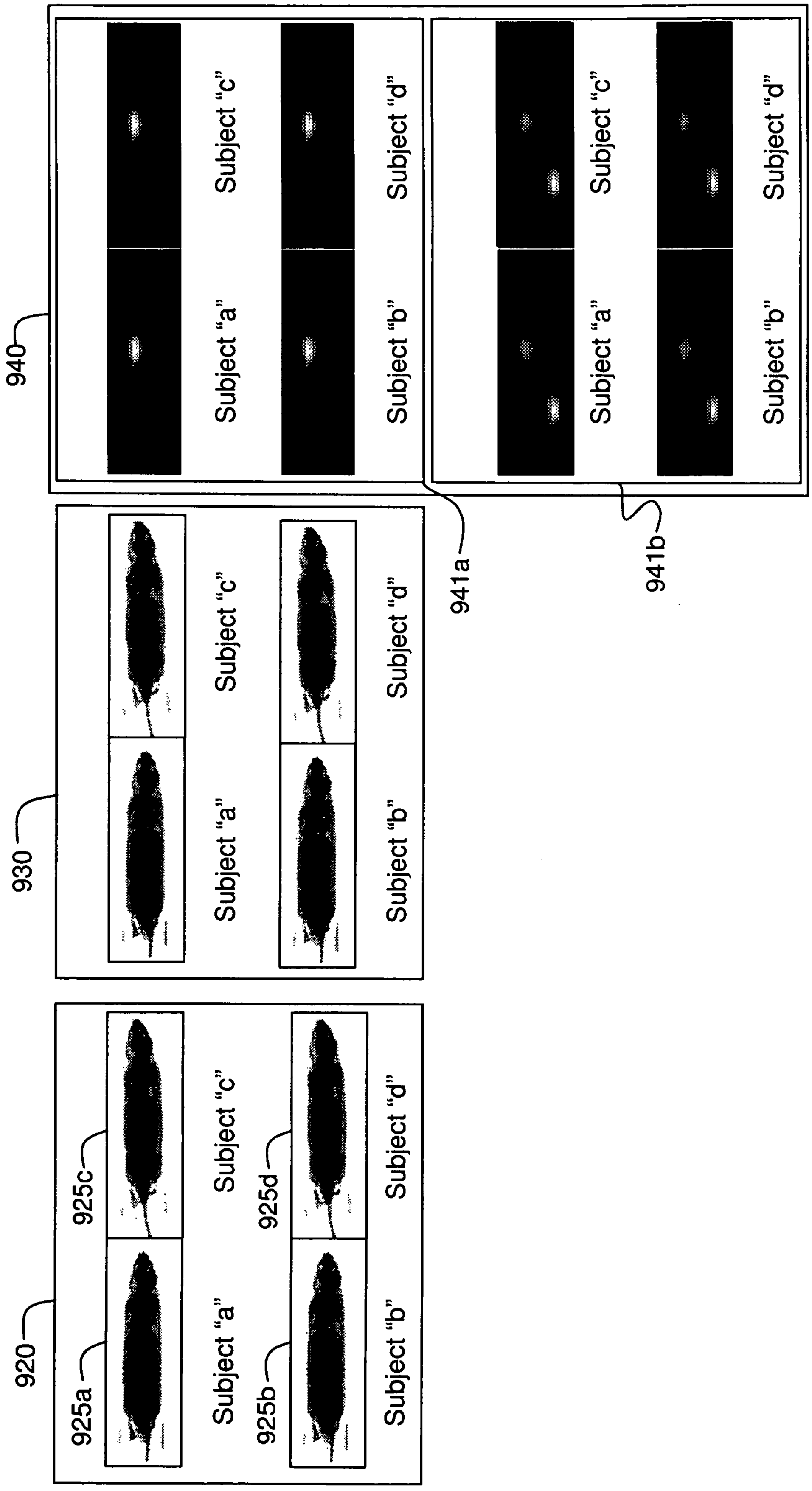
920
925a
925c
925b
925d
Subject “a”
Subject “c”
Subject “b”
Subject “d”
930
Subject “a”
Subject “c”
Subject “b”
Subject “d”
940
Subject “a”
Subject “c”
Subject “b”
Subject “d”
941a
Subject “a”
Subject “c”
Subject “b”
Subject “d”
941b

FIG. 17

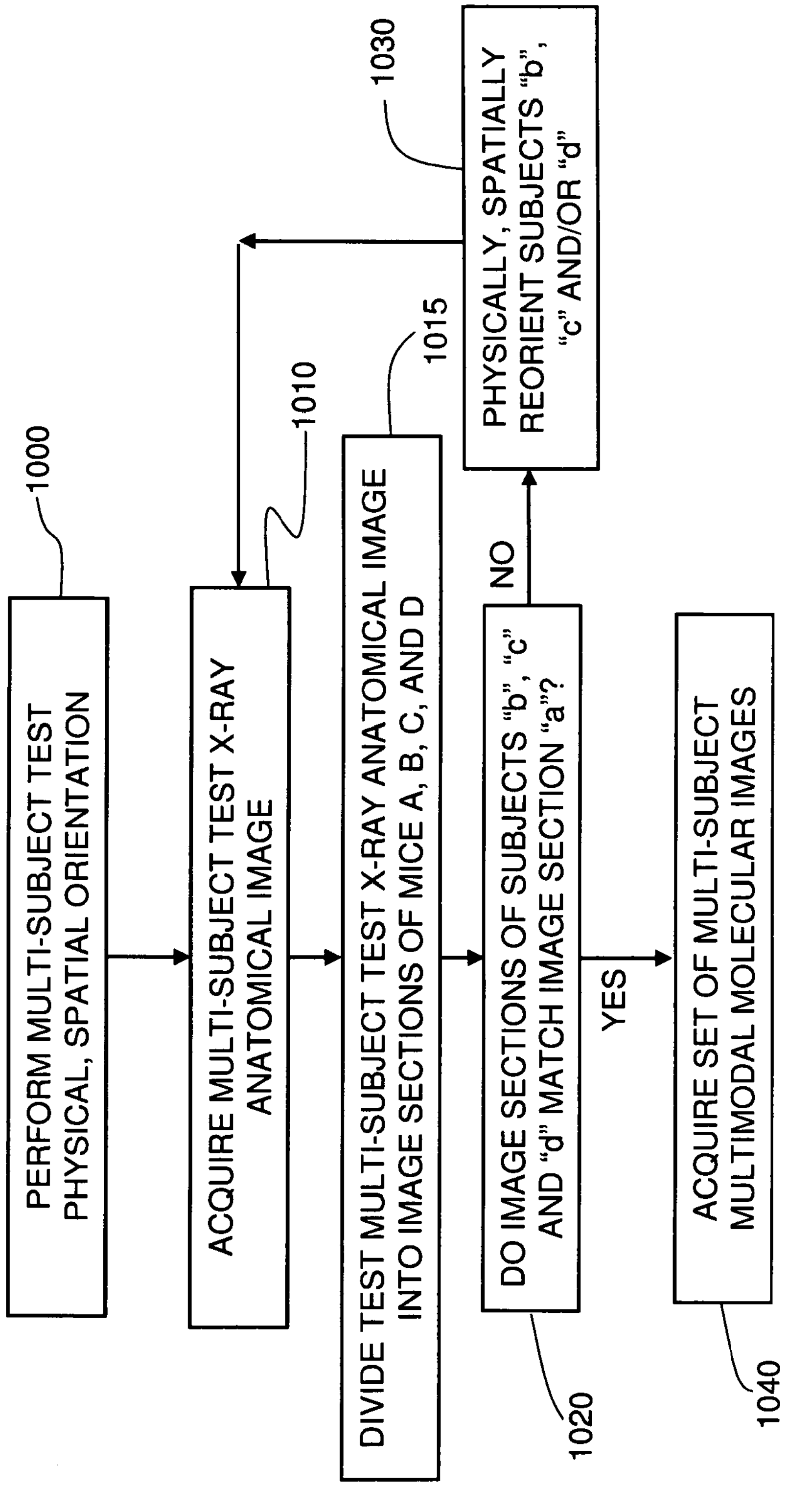
PERFORM MULTI-SUBJECT TEST PHYSICAL, SPATIAL ORIENTATION
1000
ACQUIRE MULTI-SUBJECT TEST X-RAY ANATOMICAL IMAGE
1010
DIVIDE TEST MULTI-SUBJECT TEST X-RAY ANATOMICAL IMAGE INTO IMAGE SECTIONS OF MICE A, B, C, AND D
1015
DO IMAGE SECTIONS OF SUBJECTS "b", "c" AND "d" MATCH IMAGE SECTION "a"?
1020
NO
PHYSICALLY, SPATIALLY REORIENT SUBJECTS "b", "c" AND/OR "d"
1030
YES
ACQUIRE SET OF MULTI-SUBJECT MULTIMODAL MOLECULAR IMAGES
1040

FIG. 18

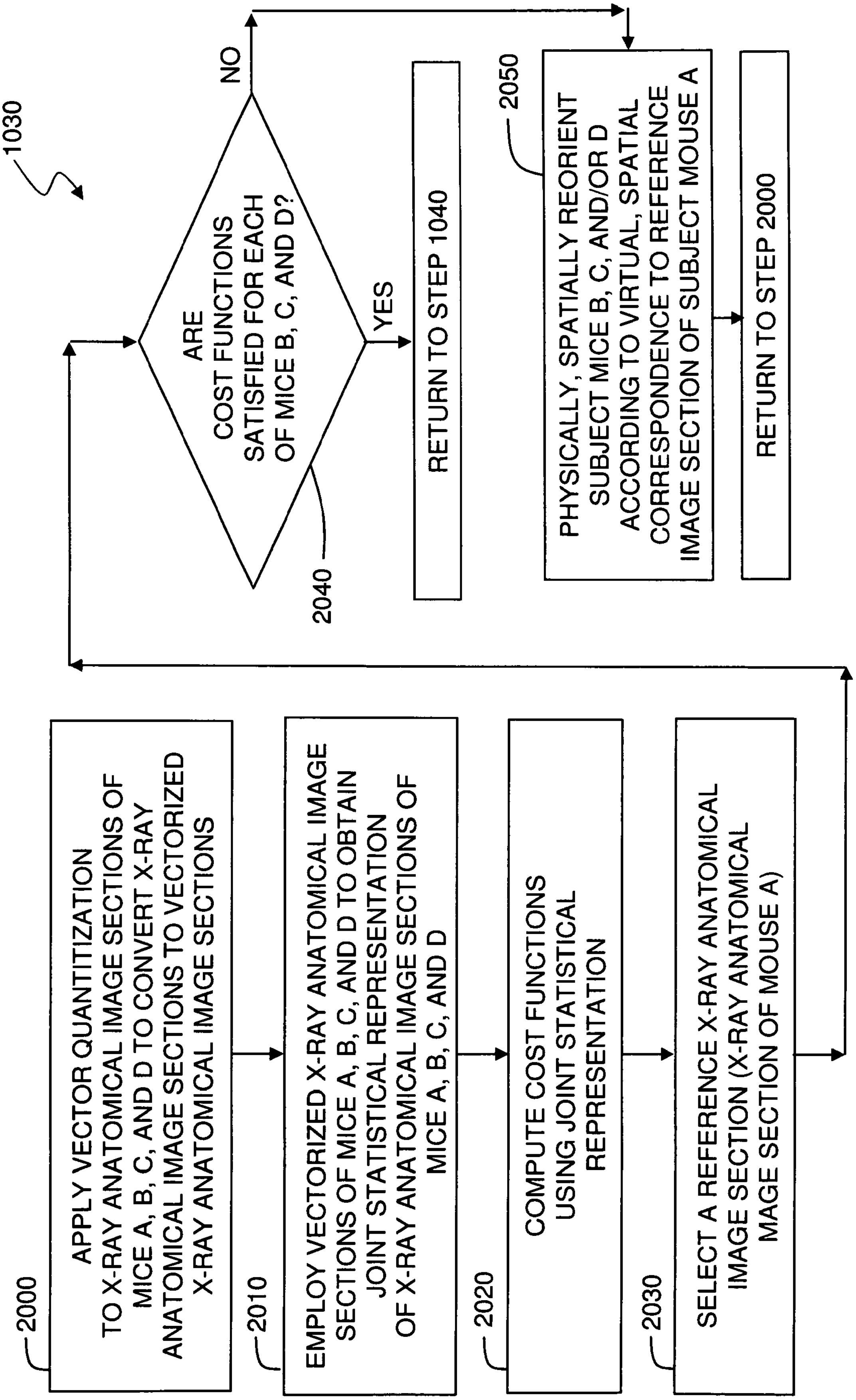
1030
2000
APPLY VECTOR QUANTITIZATION TO X-RAY ANATOMICAL IMAGE SECTIONS OF MICE A, B, C, AND D TO CONVERT X-RAY ANATOMICAL IMAGE SECTIONS TO VECTORIZED X-RAY ANATOMICAL IMAGE SECTIONS
2010
EMPLOY VECTORIZED X-RAY ANATOMICAL IMAGE SECTIONS OF MICE A, B, C, AND D TO OBTAIN JOINT STATISTICAL REPRESENTATION OF X-RAY ANATOMICAL IMAGE SECTIONS OF MICE A, B, C, AND D
2020
COMPUTE COST FUNCTIONS USING JOINT STATISTICAL REPRESENTATION
2030
SELECT A REFERENCE X-RAY ANATOMICAL IMAGE SECTION (X-RAY ANATOMICAL MAGE SECTION OF MOUSE A)
2040
ARE COST FUNCTIONS SATISFIED FOR EACH OF MICE B, C, AND D?
YES
NO
RETURN TO STEP 1040
2050
PHYSICALLY, SPATIALLY REORIENT SUBJECT MICE B, C, AND/OR D ACCORDING TO VIRTUAL, SPATIAL CORRESPONDENCE TO REFERENCE IMAGE SECTION OF SUBJECT MOUSE A
RETURN TO STEP 2000

FIG. 19

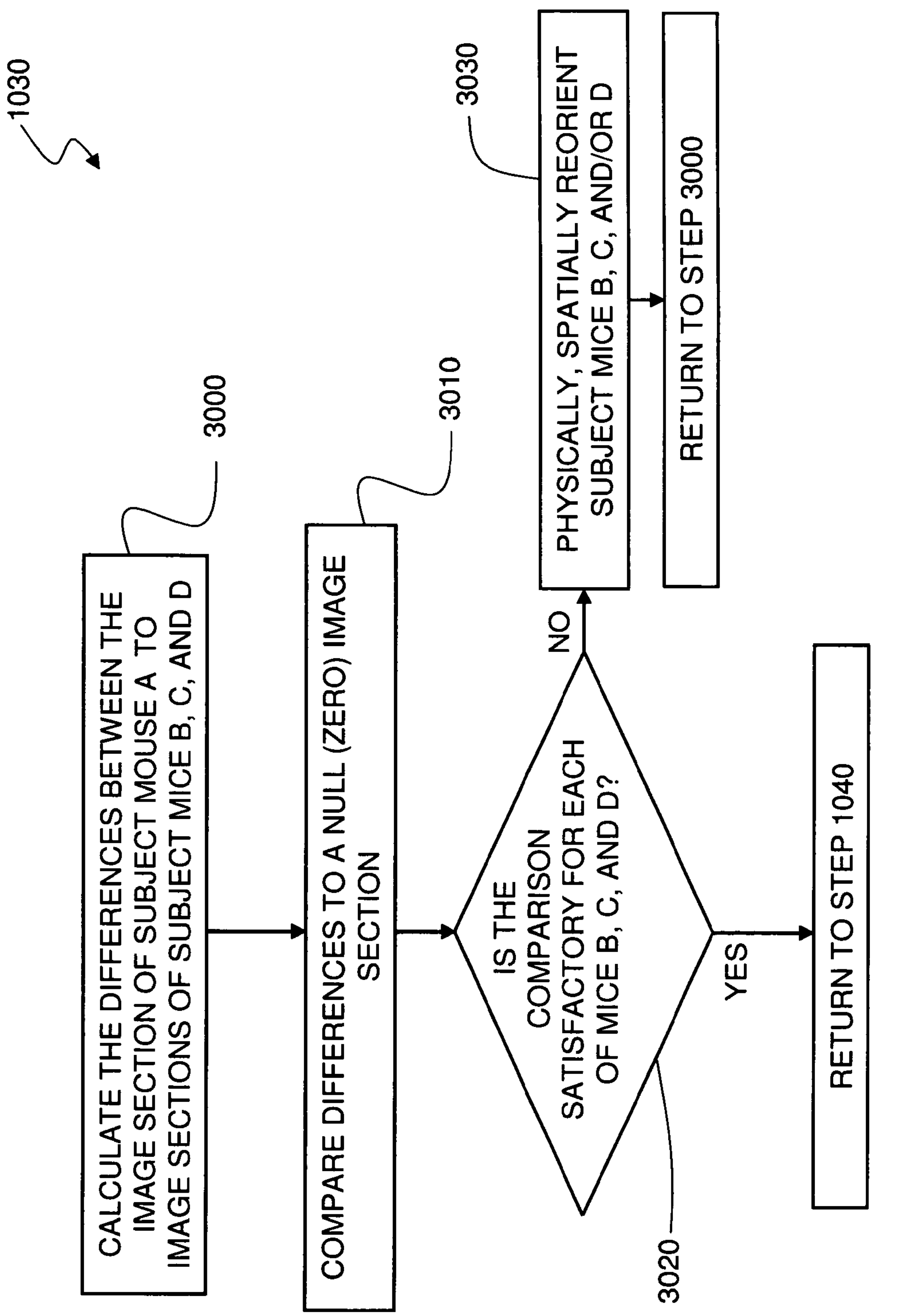
1030
3000
CALCULATE THE DIFFERENCES BETWEEN THE IMAGE SECTION OF SUBJECT MOUSE A TO IMAGE SECTIONS OF SUBJECT MICE B, C, AND D
3010
COMPARE DIFFERENCES TO A NULL (ZERO) IMAGE SECTION
3020
IS THE COMPARISON SATISFACTORY FOR EACH OF MICE B, C, AND D?
YES
RETURN TO STEP 1040
NO
3030
PHYSICALLY, SPATIALLY REORIENT SUBJECT MICE B, C, AND/OR D
RETURN TO STEP 3000

FIG. 20

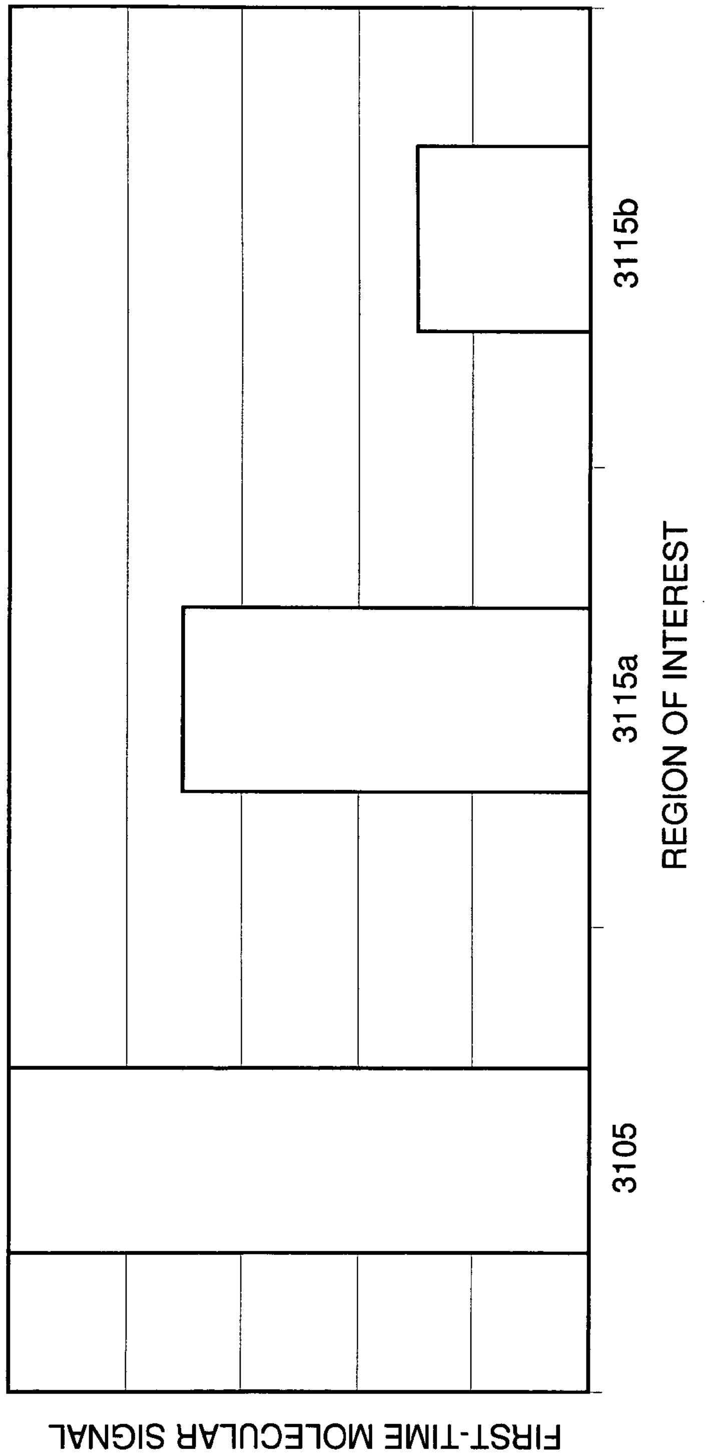
FIRST-TIME MOLECULAR SIGNAL
3105
3115a
3115b
REGION OF INTEREST

FIG. 21

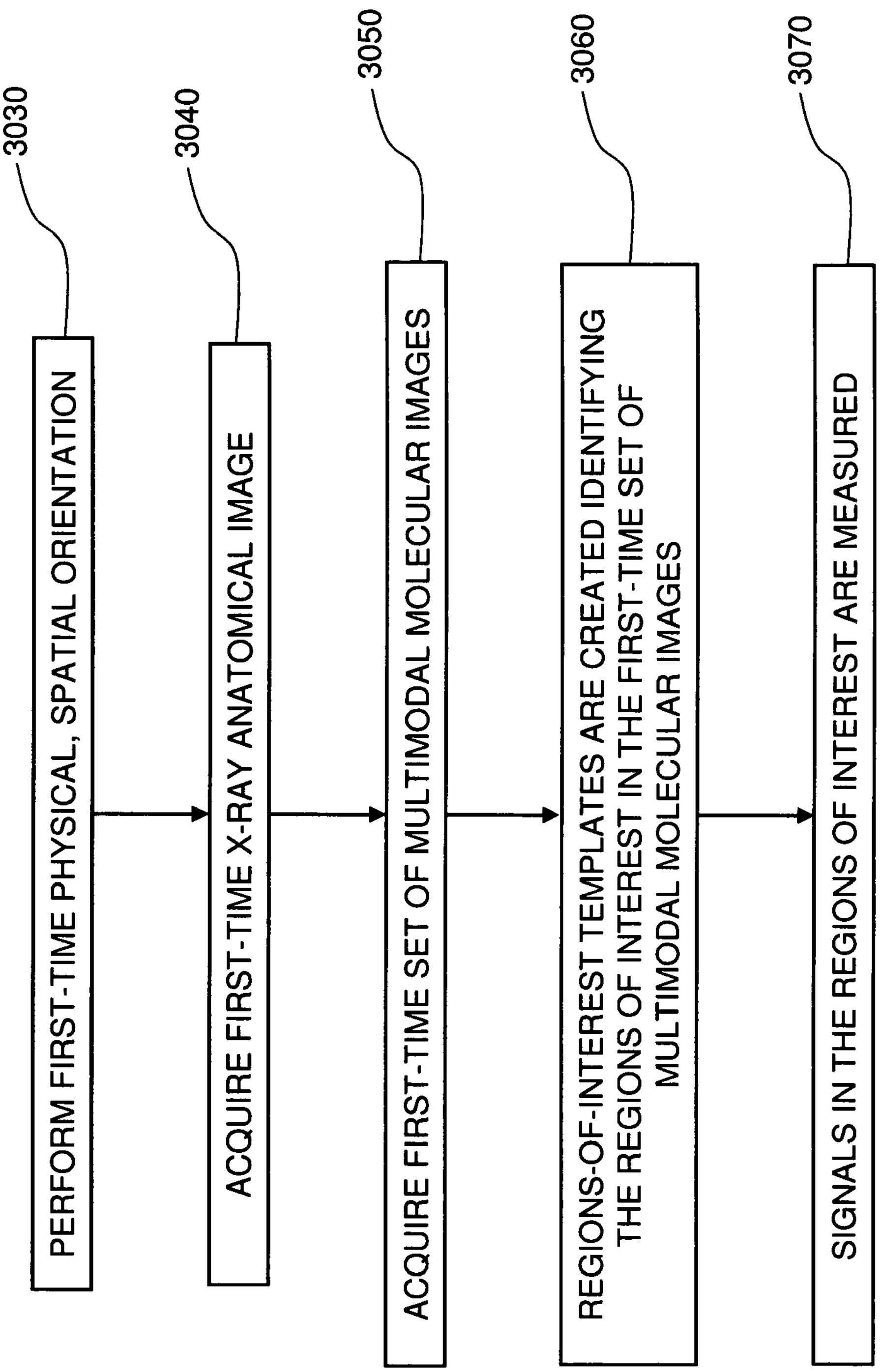
PERFORM FIRST-TIME PHYSICAL, SPATIAL ORIENTATION
3030
ACQUIRE FIRST-TIME X-RAY ANATOMICAL IMAGE
3040
ACQUIRE FIRST-TIME SET OF MULTIMODAL MOLECULAR IMAGES
3050
REGIONS-OF-INTEREST TEMPLATES ARE CREATED IDENTIFYING THE REGIONS OF INTEREST IN THE FIRST-TIME SET OF MULTIMODAL MOLECULAR IMAGES
3060
SIGNALS IN THE REGIONS OF INTEREST ARE MEASURED
3070

FIG. 22

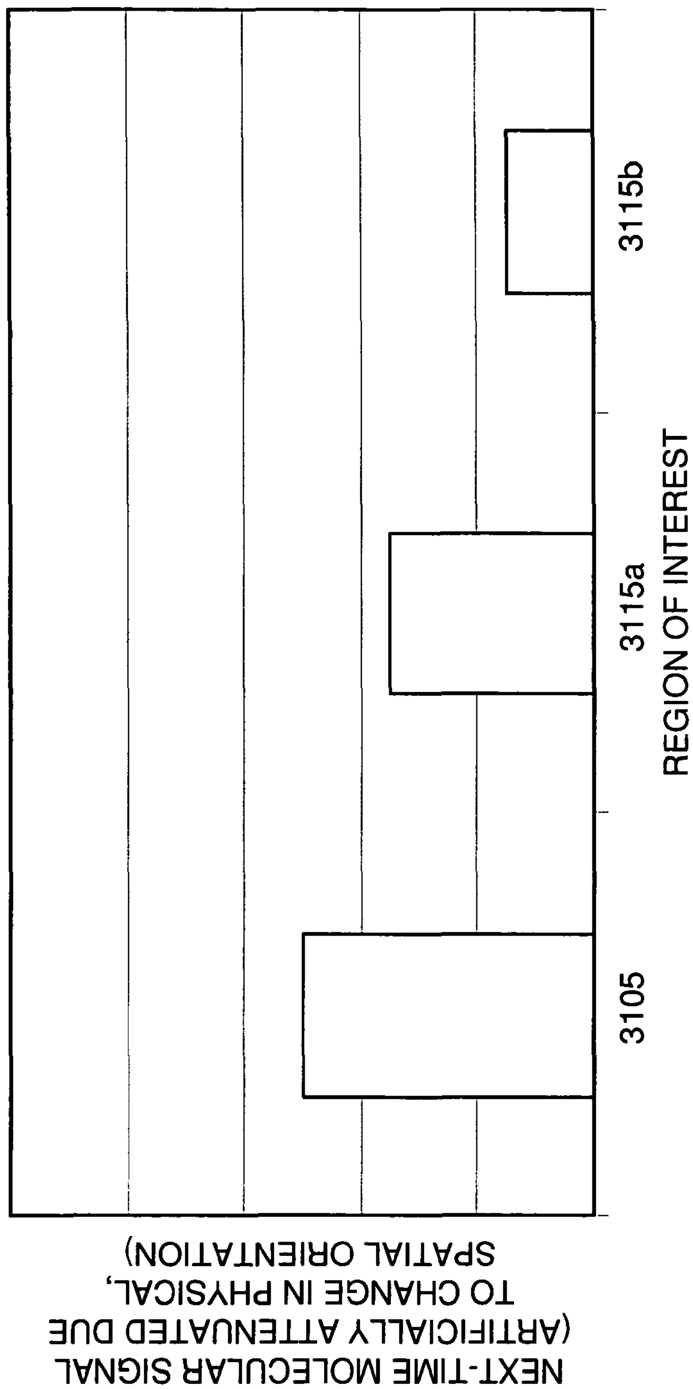
NEXT-TIME MOLECULAR SIGNAL
(ARTIFICIALLY ATTENUATED DUE
TO CHANGE IN PHYSICAL,
SPATIAL ORIENTATION)
3105
3115a
3115b
REGION OF INTEREST

*FIG. 23*

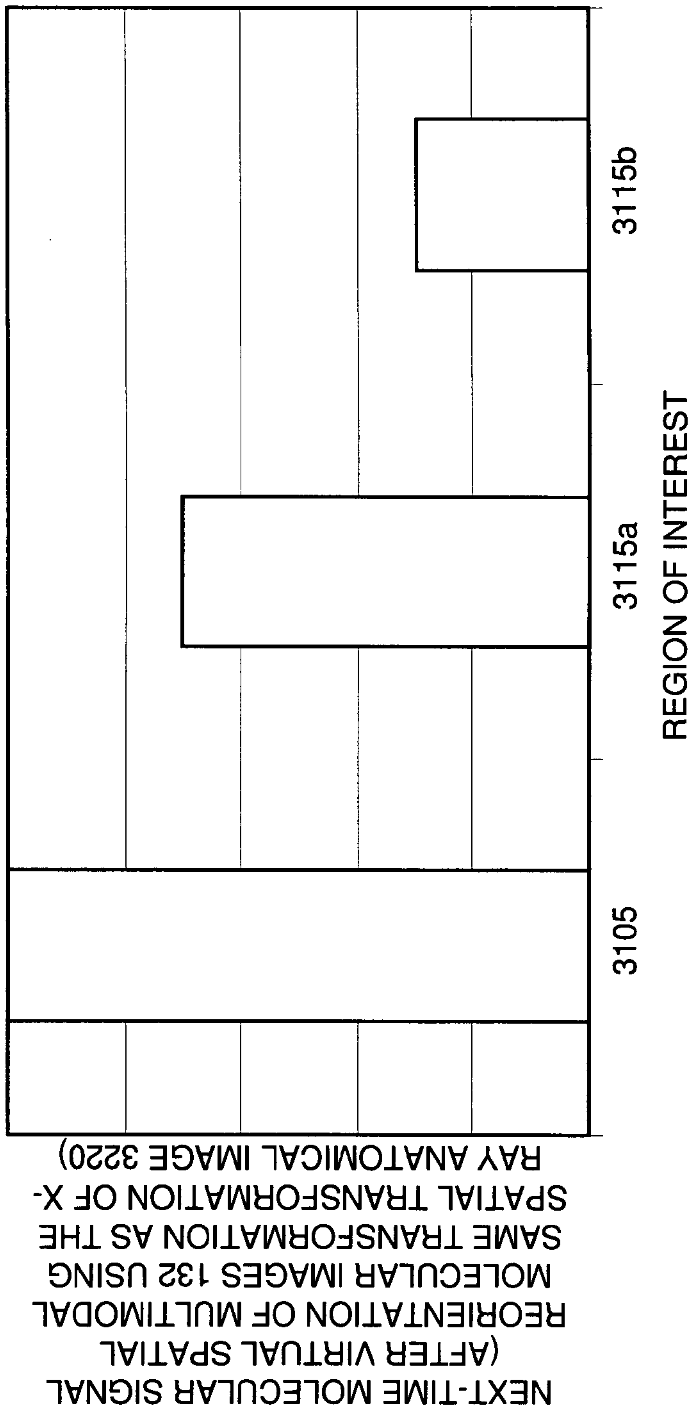
NEXT-TIME MOLECULAR SIGNAL (AFTER VIRTUAL SPATIAL REORIENTATION OF MULTIMODAL MOLECULAR IMAGES 132 USING SAME TRANSFORMATION AS THE SPATIAL TRANSFORMATION OF X-RAY ANATOMICAL IMAGE 3220)
3105
3115a
3115b
REGION OF INTEREST

*FIG. 24*

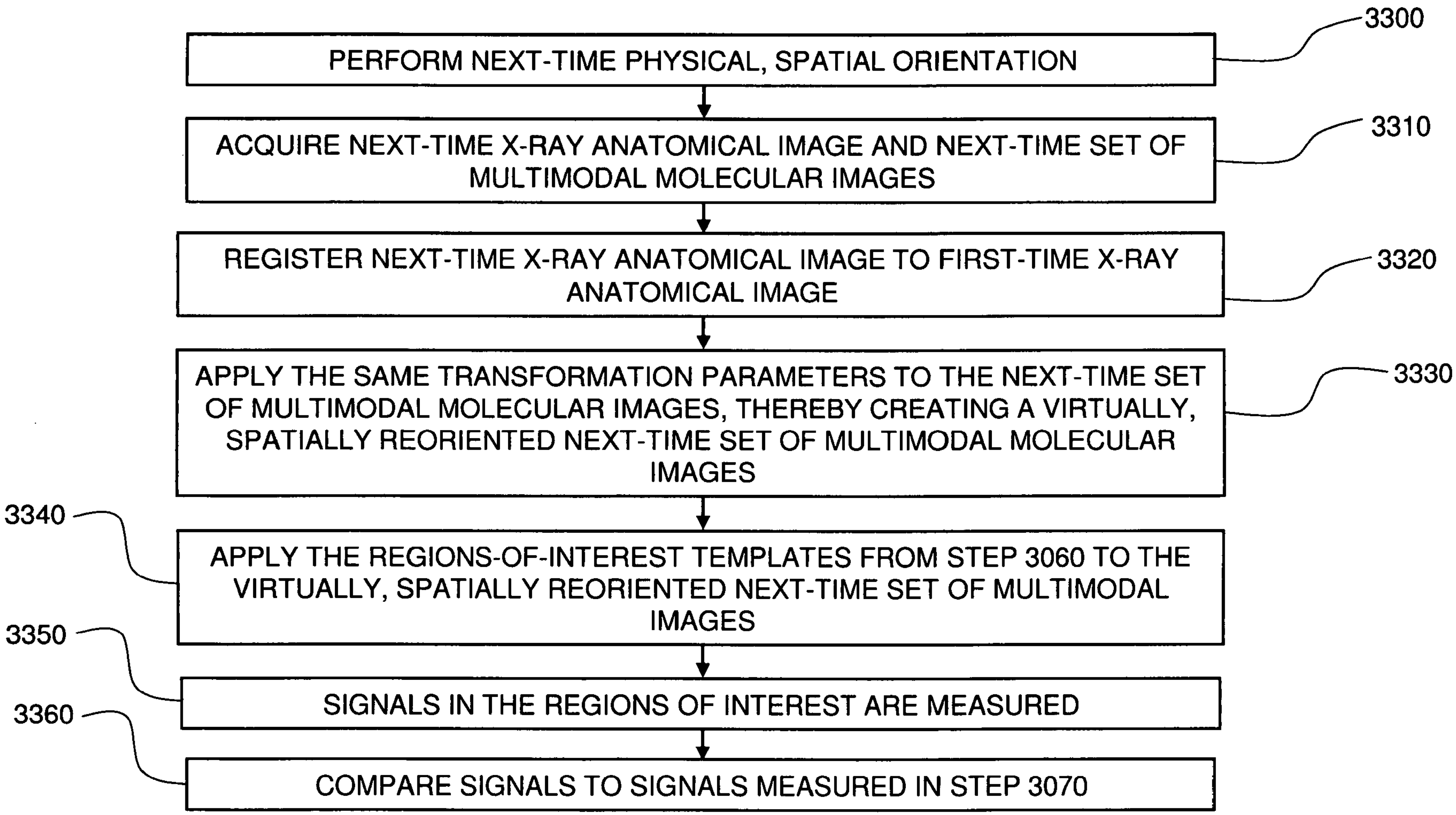
PERFORM NEXT-TIME PHYSICAL, SPATIAL ORIENTATION
3300
ACQUIRE NEXT-TIME X-RAY ANATOMICAL IMAGE AND NEXT-TIME SET OF MULTIMODAL MOLECULAR IMAGES
3310
REGISTER NEXT-TIME X-RAY ANATOMICAL IMAGE TO FIRST-TIME X-RAY ANATOMICAL IMAGE
3320
APPLY THE SAME TRANSFORMATION PARAMETERS TO THE NEXT-TIME SET OF MULTIMODAL MOLECULAR IMAGES, THEREBY CREATING A VIRTUALLY, SPATIALLY REORIENTED NEXT-TIME SET OF MULTIMODAL MOLECULAR IMAGES
3330
APPLY THE REGIONS-OF-INTEREST TEMPLATES FROM STEP 3060 TO THE VIRTUALLY, SPATIALLY REORIENTED NEXT-TIME SET OF MULTIMODAL IMAGES
3340
SIGNALS IN THE REGIONS OF INTEREST ARE MEASURED
3350
COMPARE SIGNALS TO SIGNALS MEASURED IN STEP 3070
3360

FIG. 25

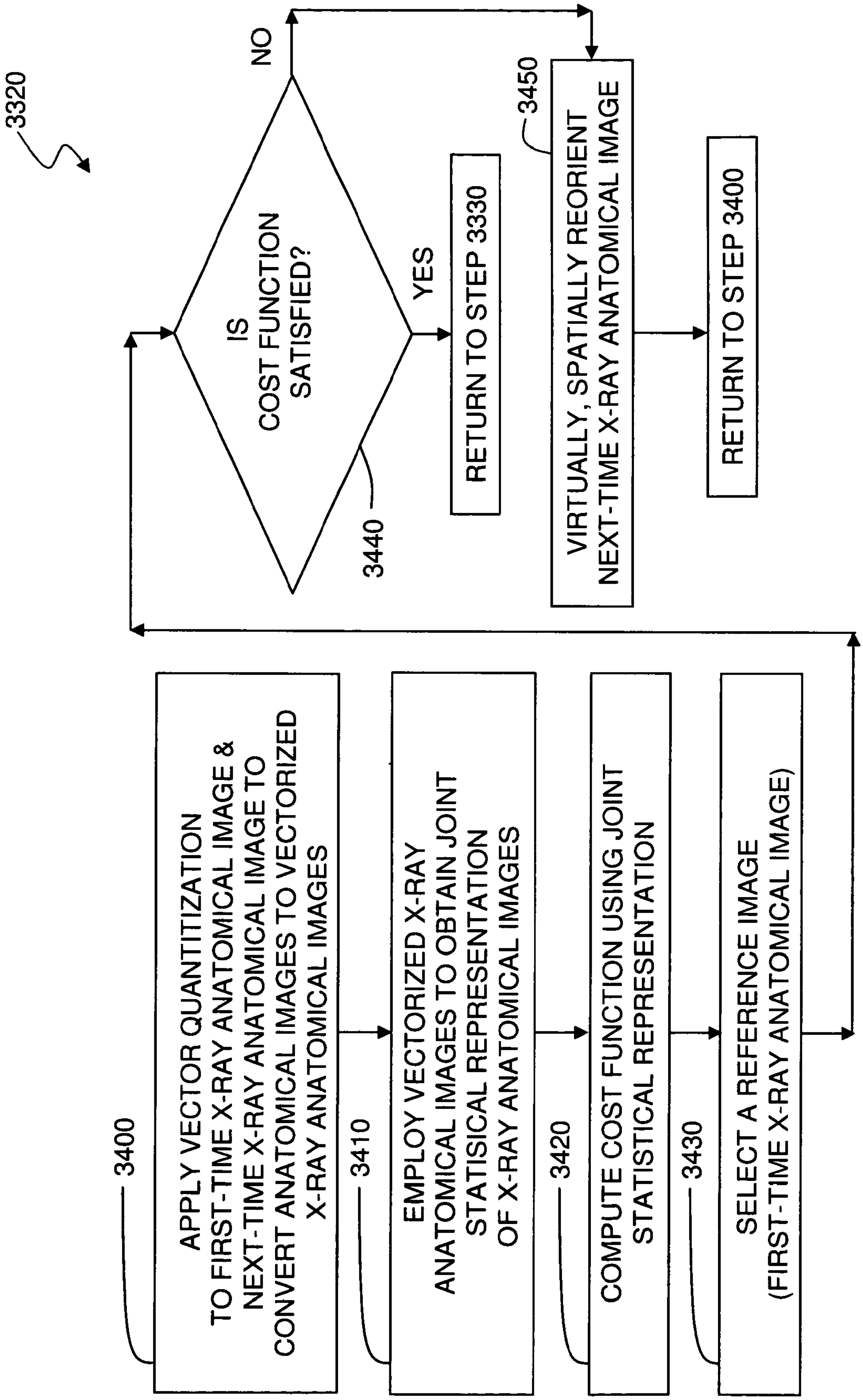
3320
3400
APPLY VECTOR QUANTITIZATION TO FIRST-TIME X-RAY ANATOMICAL IMAGE & NEXT-TIME X-RAY ANATOMICAL IMAGE TO CONVERT ANATOMICAL IMAGES TO VECTORIZED X-RAY ANATOMICAL IMAGES
3410
EMPLOY VECTORIZED X-RAY ANATOMICAL IMAGES TO OBTAIN JOINT STATISICAL REPRESENTATION OF X-RAY ANATOMICAL IMAGES
3420
COMPUTE COST FUNCTION USING JOINT STATISTICAL REPRESENTATION
3430
SELECT A REFERENCE IMAGE (FIRST-TIME X-RAY ANATOMICAL IMAGE)
3440
IS COST FUNCTION SATISFIED?
YES
NO
RETURN TO STEP 3330
3450
VIRTUALLY, SPATIALLY REORIENT NEXT-TIME X-RAY ANATOMICAL IMAGE
RETURN TO STEP 3400

FIG. 26

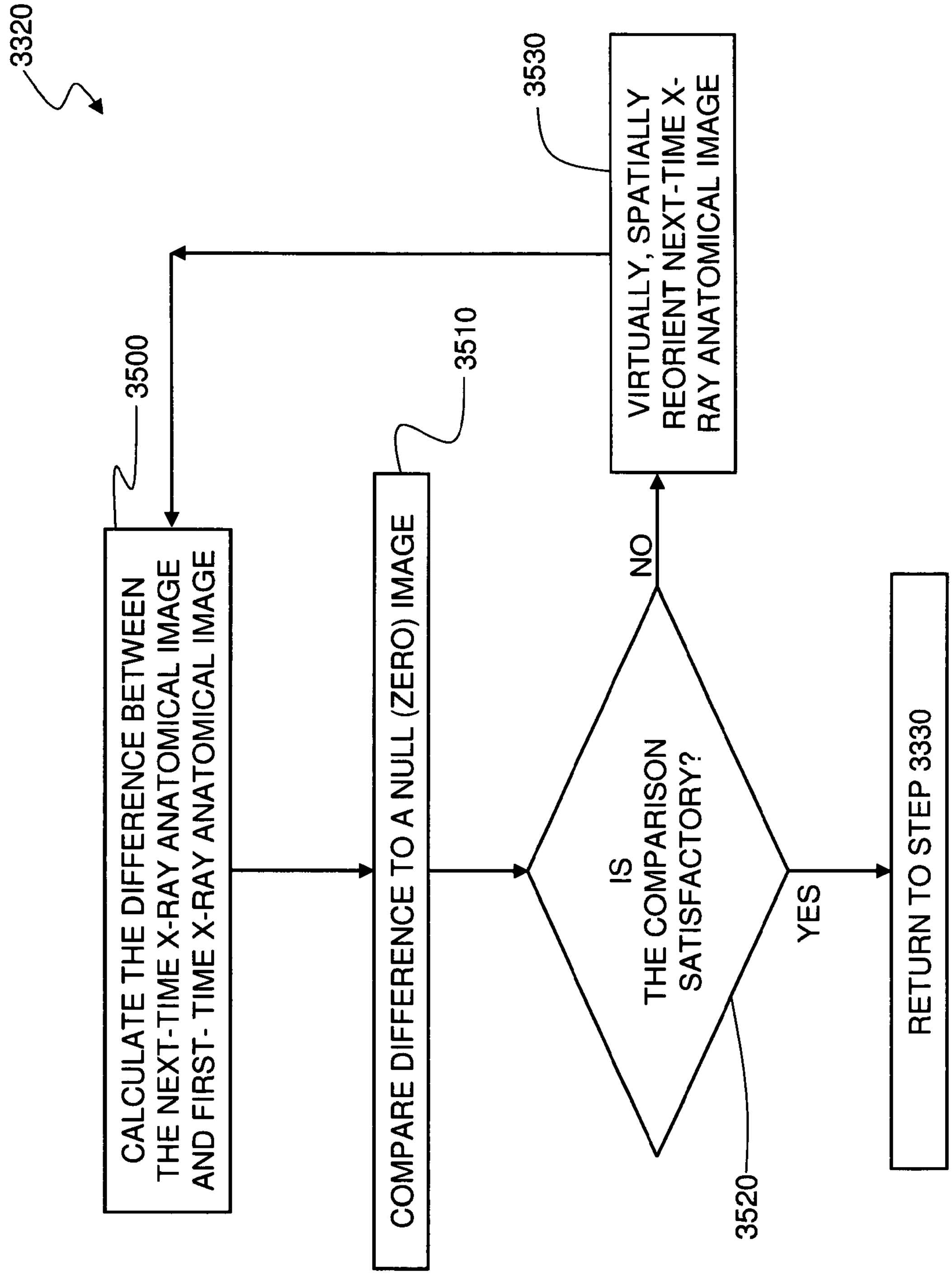
3320
CALCULATE THE DIFFERENCE BETWEEN THE NEXT-TIME X-RAY ANATOMICAL IMAGE AND FIRST- TIME X-RAY ANATOMICAL IMAGE
3500
COMPARE DIFFERENCE TO A NULL (ZERO) IMAGE
3510
IS THE COMPARISON SATISFACTORY?
3520
NO
YES
VIRTUALLY, SPATIALLY REORIENT NEXT-TIME X-RAY ANATOMICAL IMAGE
3530
RETURN TO STEP 3330

FIG. 27

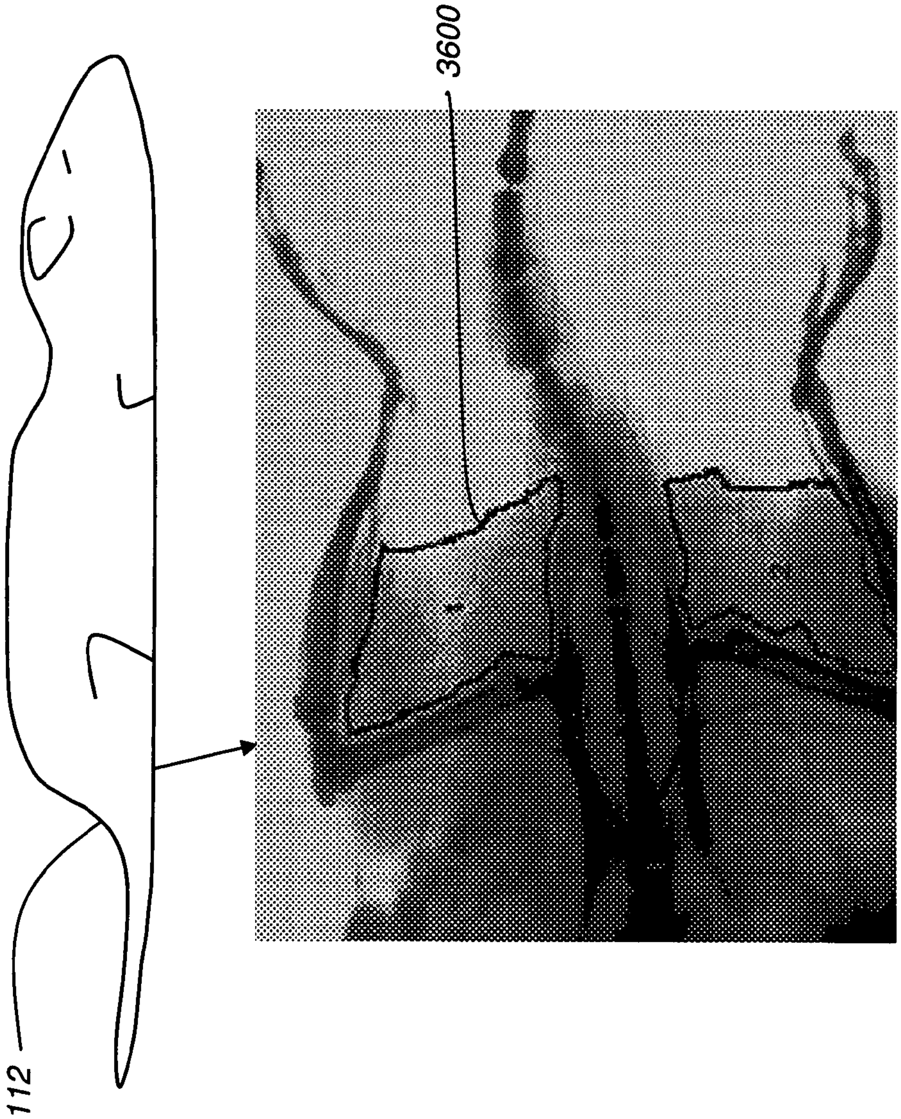


FIG. 28

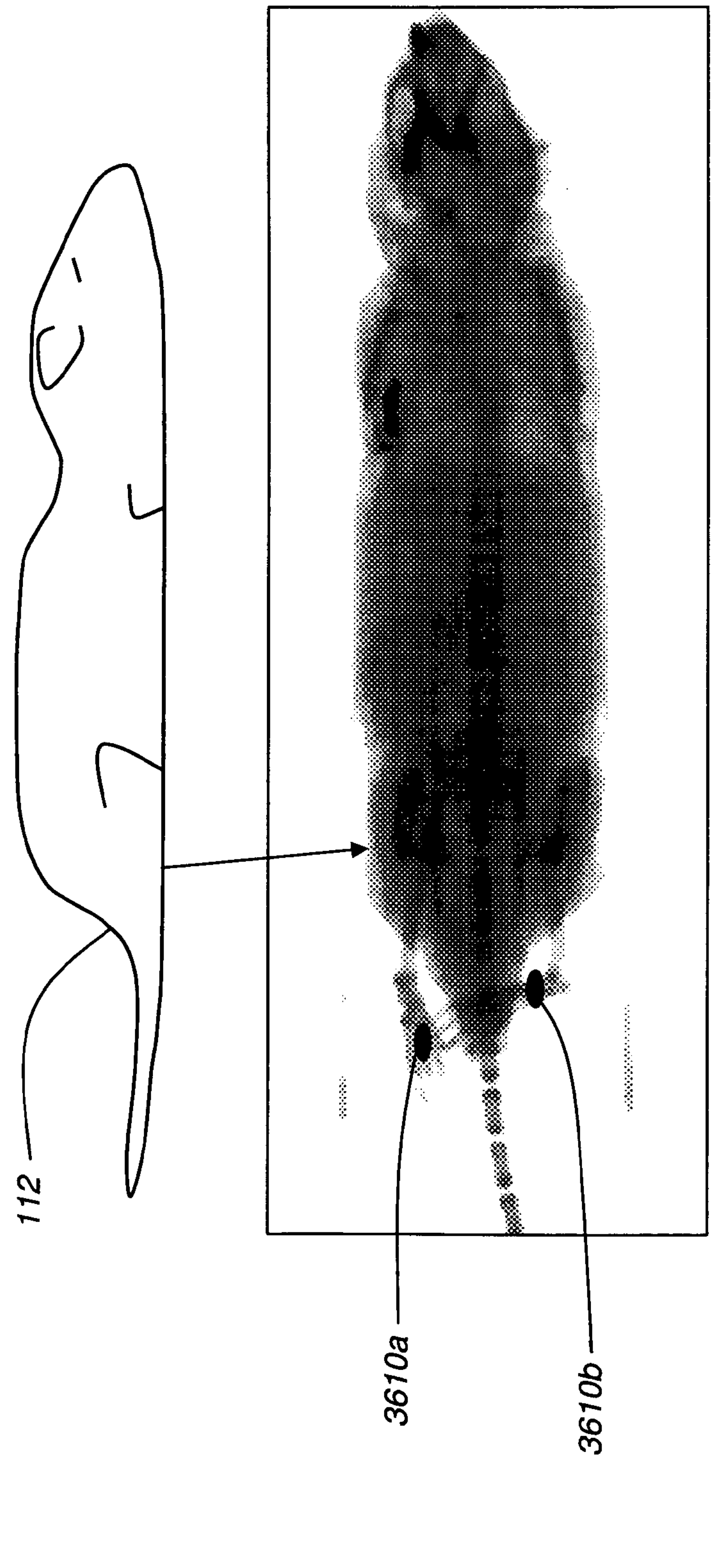


FIG. 29

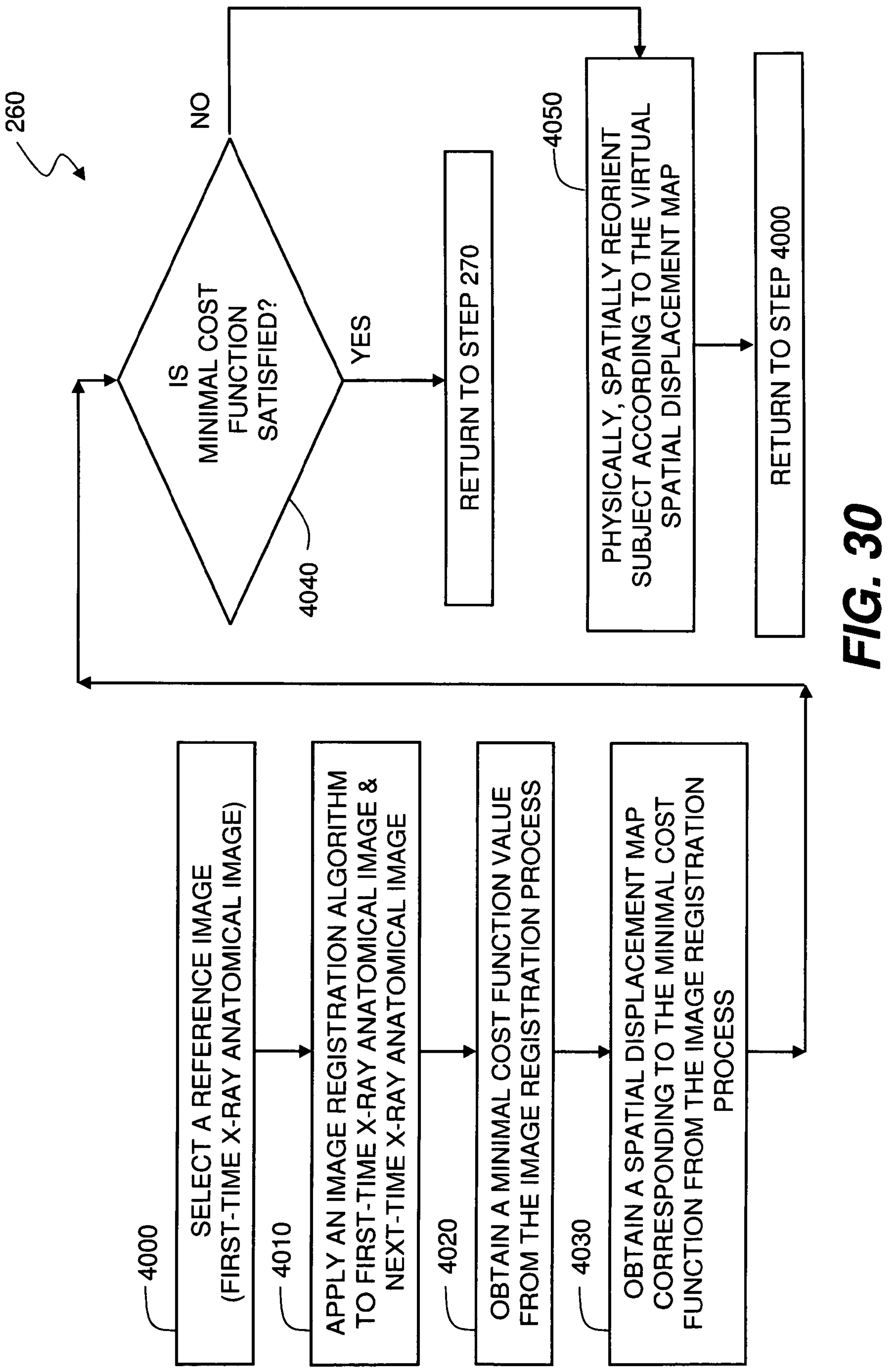
260
4000
SELECT A REFERENCE IMAGE (FIRST-TIME X-RAY ANATOMICAL IMAGE)
4010
APPLY AN IMAGE REGISTRATION ALGORITHM TO FIRST-TIME X-RAY ANATOMICAL IMAGE & NEXT-TIME X-RAY ANATOMICAL IMAGE
4020
OBTAIN A MINIMAL COST FUNCTION VALUE FROM THE IMAGE REGISTRATION PROCESS
4030
OBTAIN A SPATIAL DISPLACEMENT MAP CORRESPONDING TO THE MINIMAL COST FUNCTION FROM THE IMAGE REGISTRATION PROCESS
4040
IS MINIMAL COST FUNCTION SATISFIED?
YES
NO
RETURN TO STEP 270
4050
PHYSICALLY, SPATIALLY REORIENT SUBJECT ACCORDING TO THE VIRTUAL SPATIAL DISPLACEMENT MAP
RETURN TO STEP 4000

FIG. 30

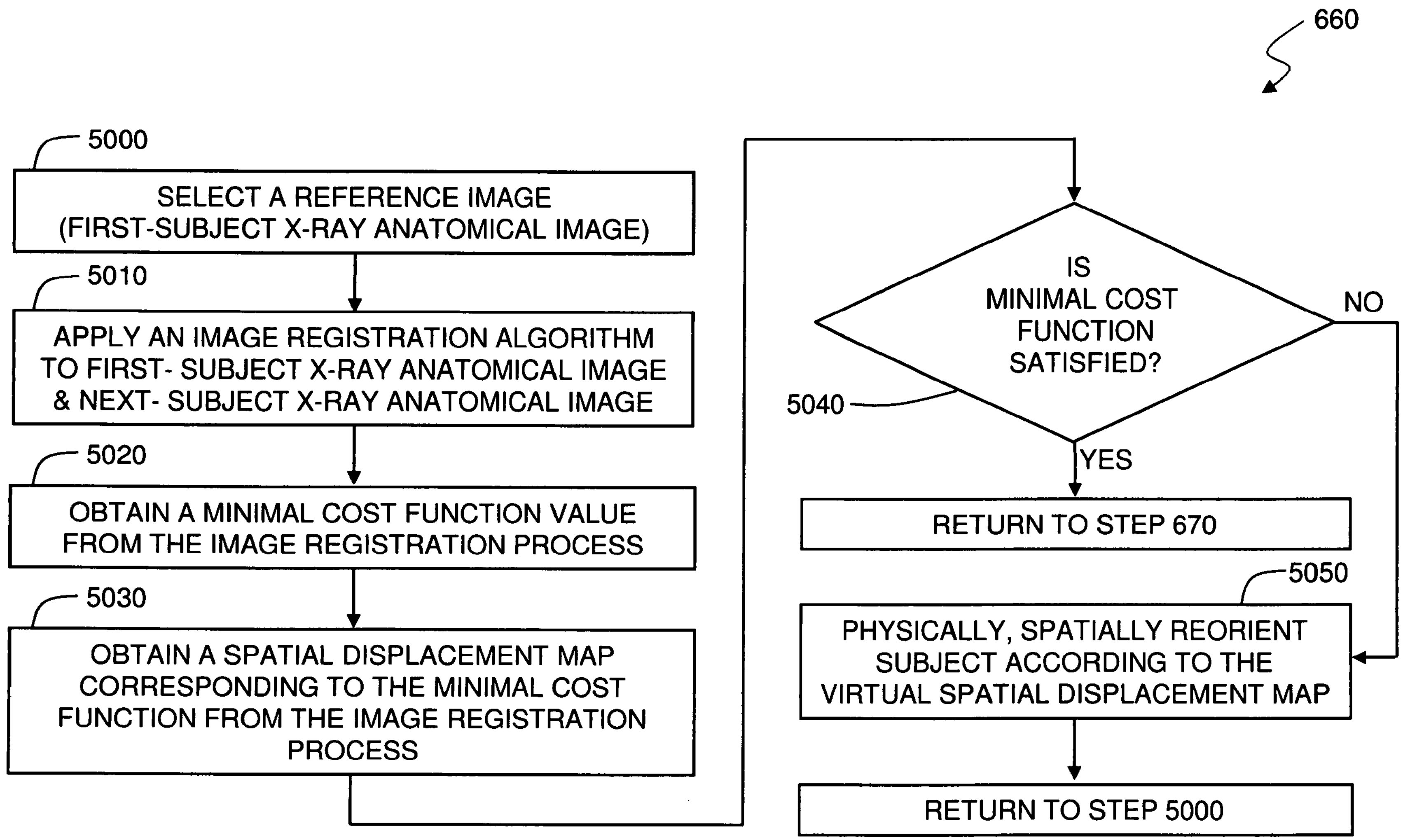
660
5000
SELECT A REFERENCE IMAGE (FIRST-SUBJECT X-RAY ANATOMICAL IMAGE)
5010
APPLY AN IMAGE REGISTRATION ALGORITHM TO FIRST- SUBJECT X-RAY ANATOMICAL IMAGE & NEXT- SUBJECT X-RAY ANATOMICAL IMAGE
5020
OBTAIN A MINIMAL COST FUNCTION VALUE FROM THE IMAGE REGISTRATION PROCESS
5030
OBTAIN A SPATIAL DISPLACEMENT MAP CORRESPONDING TO THE MINIMAL COST FUNCTION FROM THE IMAGE REGISTRATION PROCESS
5040
IS MINIMAL COST FUNCTION SATISFIED?
YES
NO
RETURN TO STEP 670
5050
PHYSICALLY, SPATIALLY REORIENT SUBJECT ACCORDING TO THE VIRTUAL SPATIAL DISPLACEMENT MAP
RETURN TO STEP 5000

FIG. 31

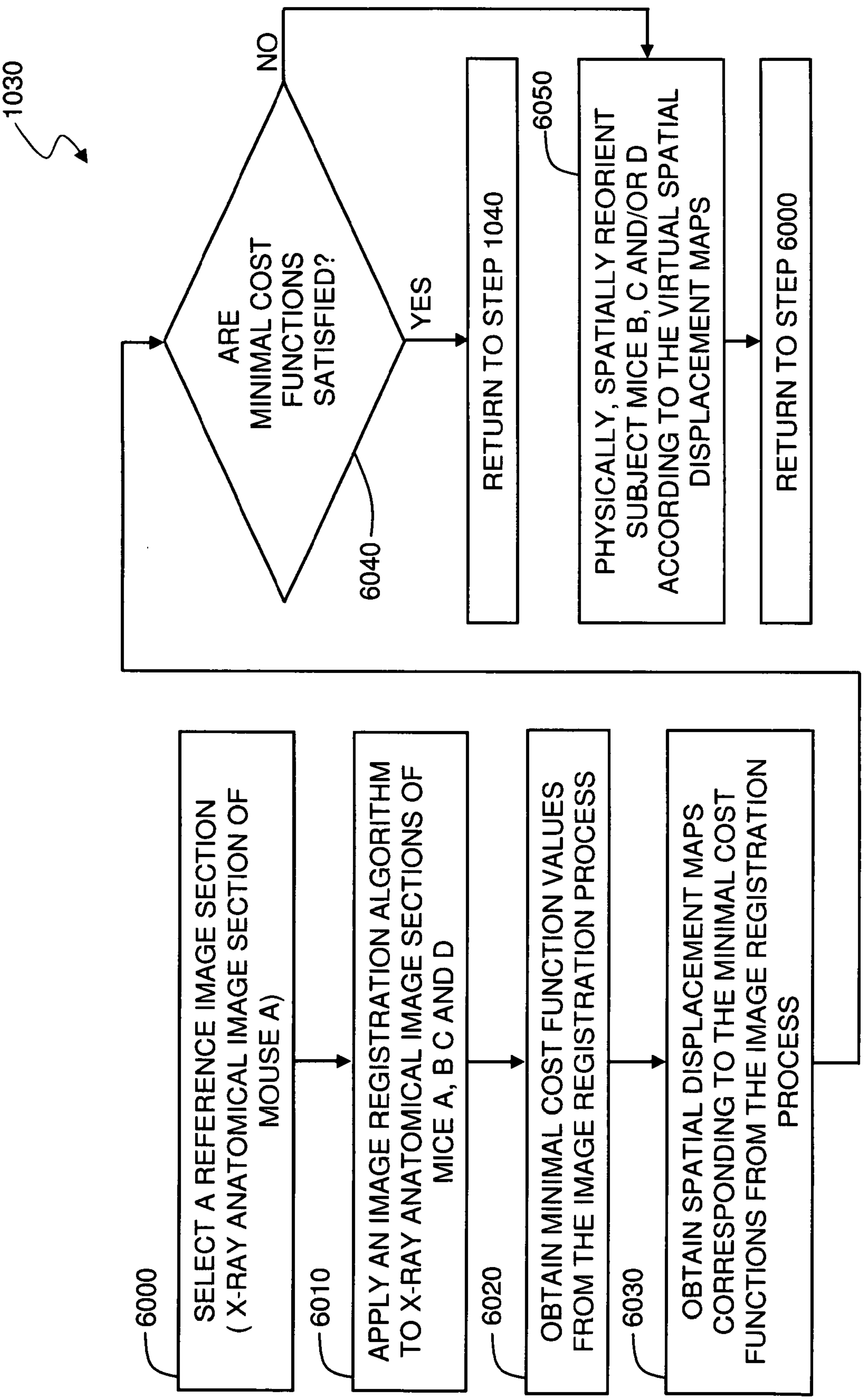
1030
6000
SELECT A REFERENCE IMAGE SECTION ( X-RAY ANATOMICAL IMAGE SECTION OF MOUSE A)
6010
APPLY AN IMAGE REGISTRATION ALGORITHM TO X-RAY ANATOMICAL IMAGE SECTIONS OF MICE A, B C AND D
6020
OBTAIN MINIMAL COST FUNCTION VALUES FROM THE IMAGE REGISTRATION PROCESS
6030
OBTAIN SPATIAL DISPLACEMENT MAPS CORRESPONDING TO THE MINIMAL COST FUNCTIONS FROM THE IMAGE REGISTRATION PROCESS
6040
ARE MINIMAL COST FUNCTIONS SATISFIED?
YES
NO
RETURN TO STEP 1040
6050
PHYSICALLY, SPATIALLY REORIENT SUBJECT MICE B, C AND/OR D ACCORDING TO THE VIRTUAL SPATIAL DISPLACEMENT MAPS
RETURN TO STEP 6000

FIG. 32

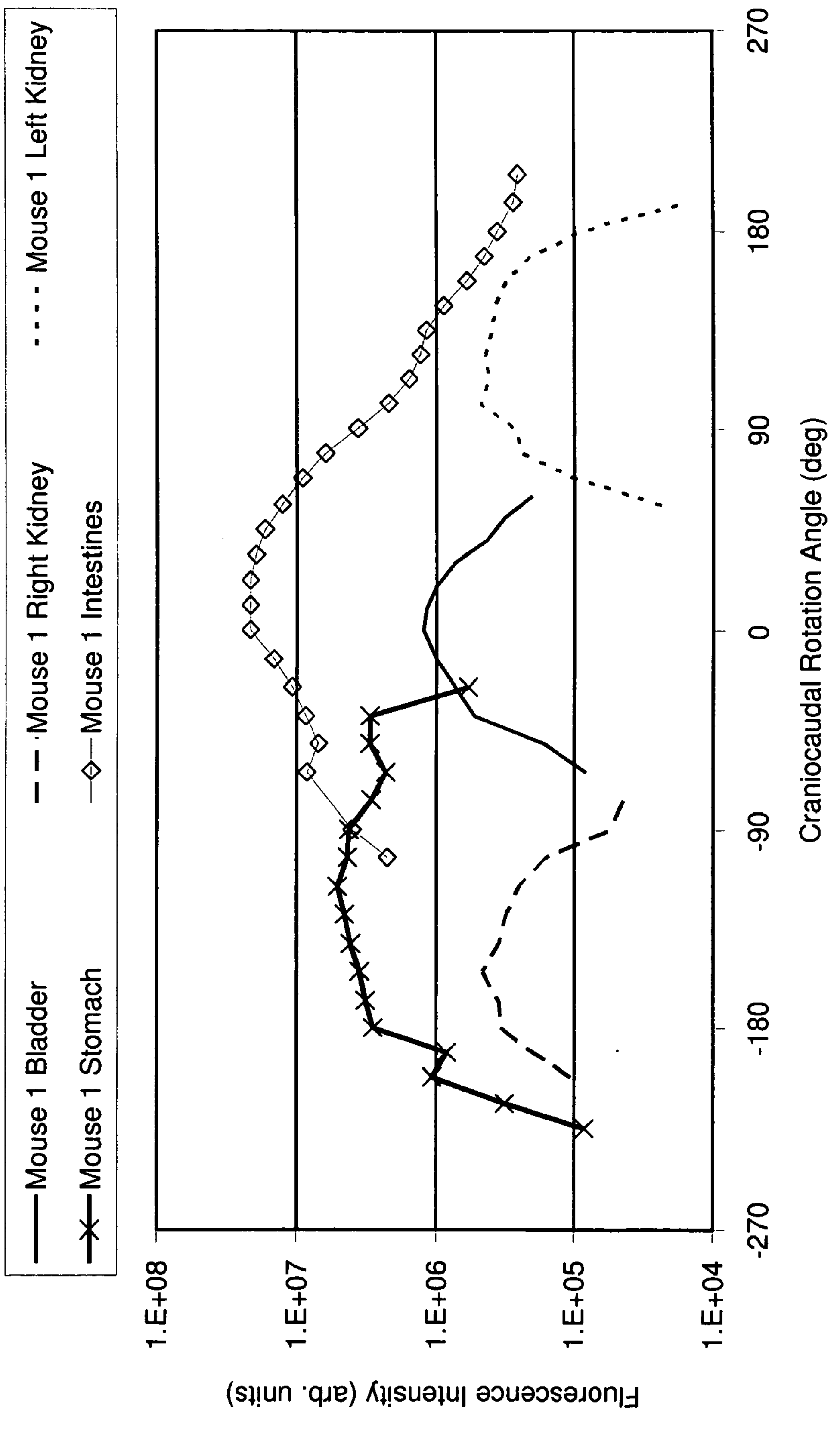
Mouse 1 Bladder
Mouse 1 Right Kidney
Mouse 1 Left Kidney
Mouse 1 Stomach
Mouse 1 Intestines
1.E+08
1.E+07
1.E+06
1.E+05
1.E+04
Fluorescence Intensity (arb. units)
-270
-180
-90
0
90
180
270
Craniocaudal Rotation Angle (deg)

FIG. 33A

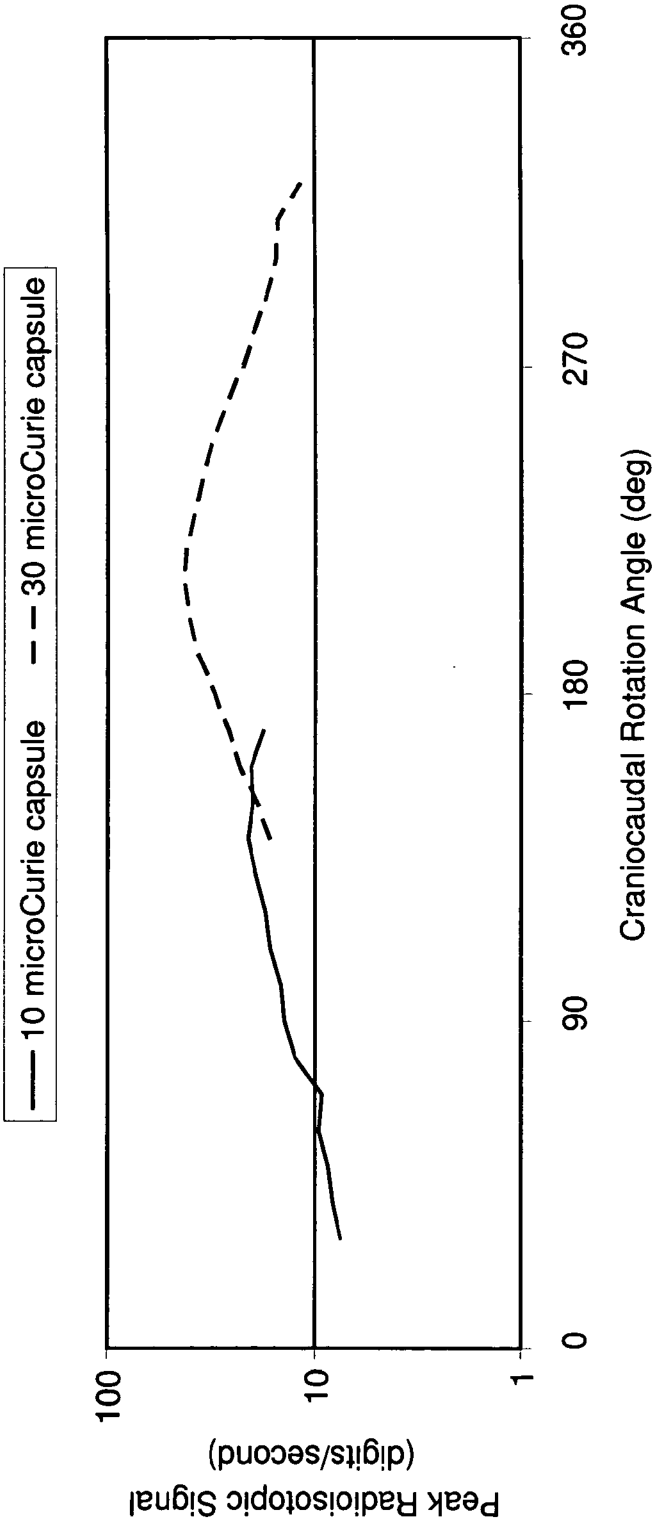
10 microCurie capsule
30 microCurie capsule
Peak Radioisotopic Signal (digits/second)
100
10
1
0
90
180
270
360
Craniocaudal Rotation Angle (deg)

*FIG. 33B*

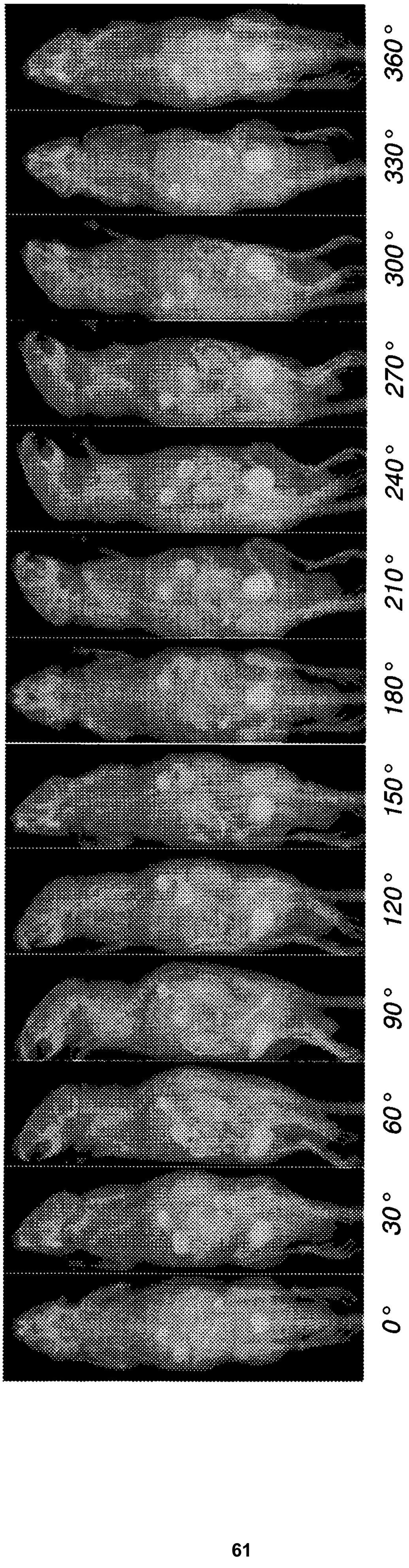


FIG. 34

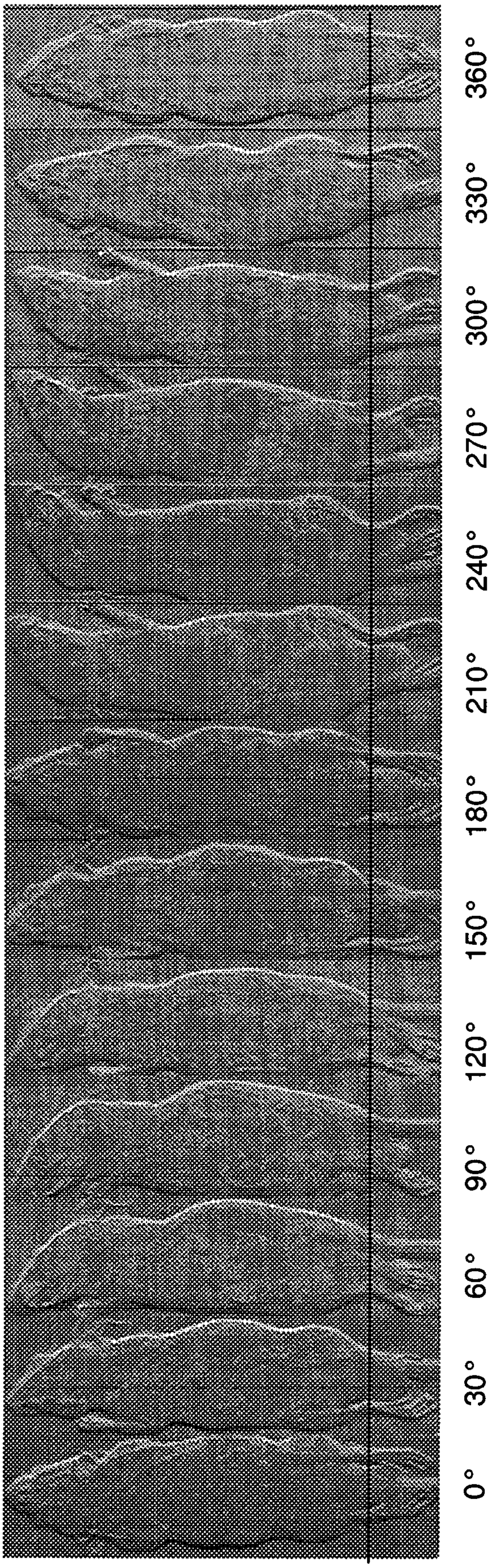


FIG. 35A

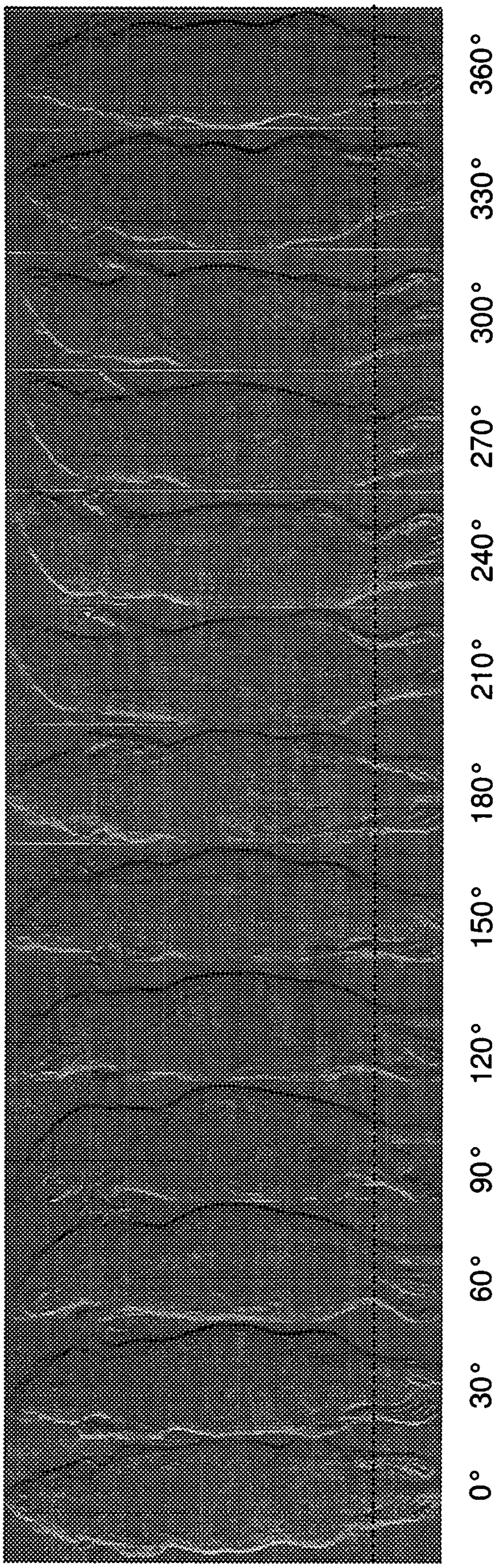


FIG. 35B

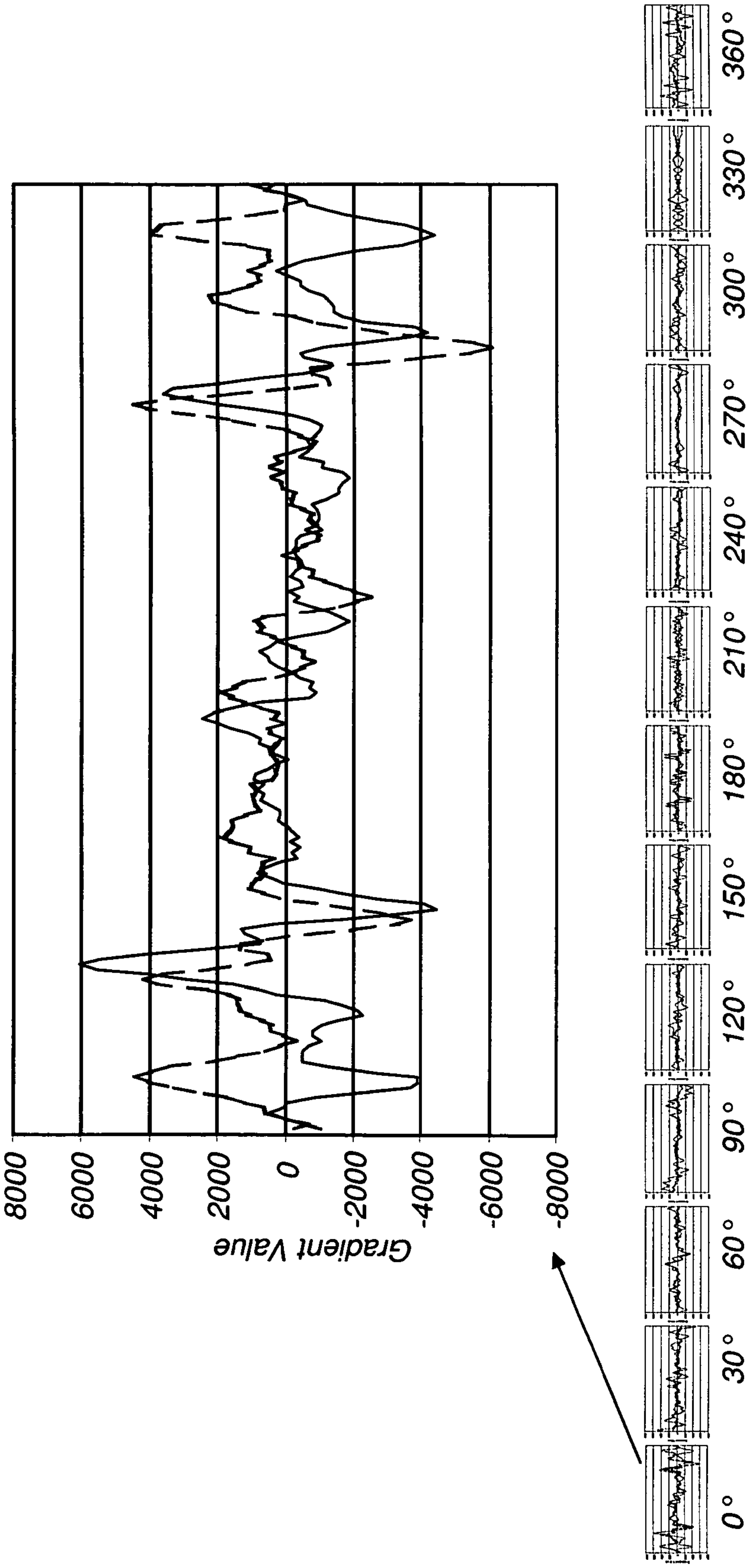
8000
6000
4000
2000
0
-2000
-4000
-6000
-8000
Gradient Value
0°
30°
60°
90°
120°
150°
180°
210°
240°
270°
300°
330°
360°

FIG. 36

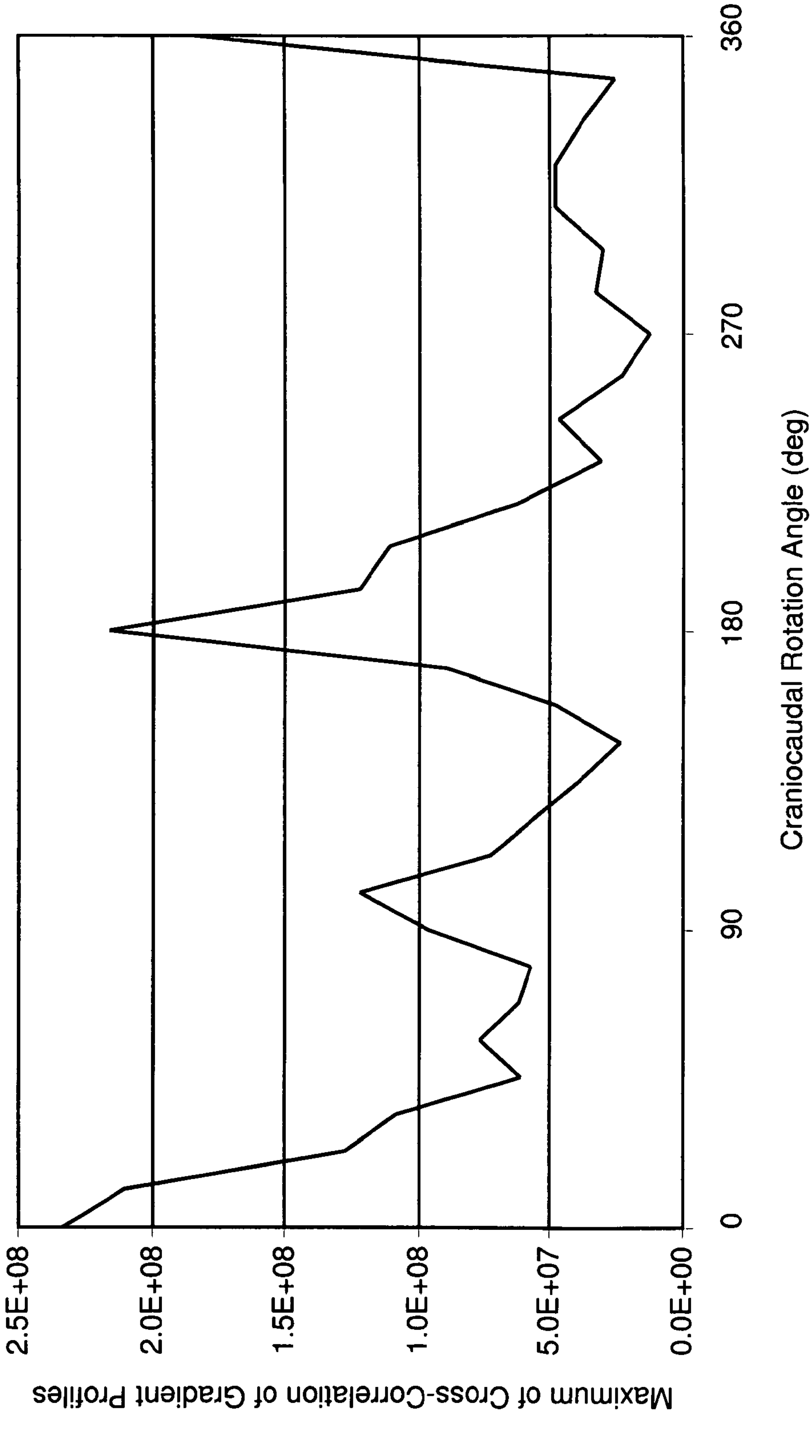
Maximum of Cross-Correlation of Gradient Profiles
2.5E+08
2.0E+08
1.5E+08
1.0E+08
5.0E+07
0.0E+00
0
90
180
270
360
Craniocaudal Rotation Angle (deg)

FIG. 37

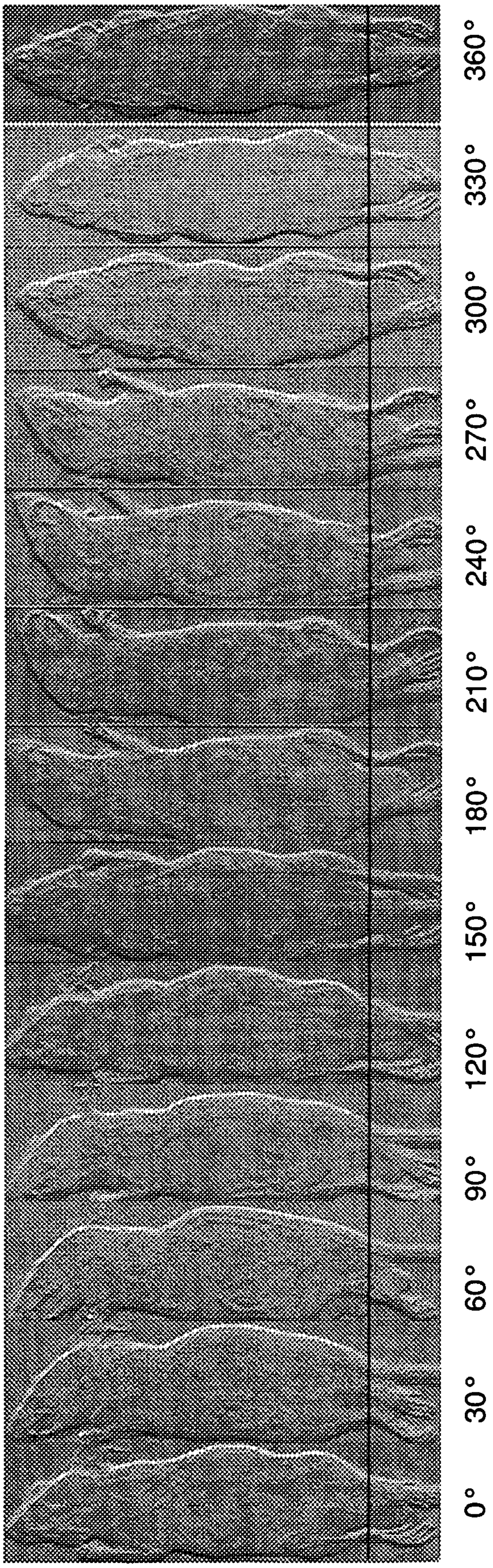


FIG. 38A

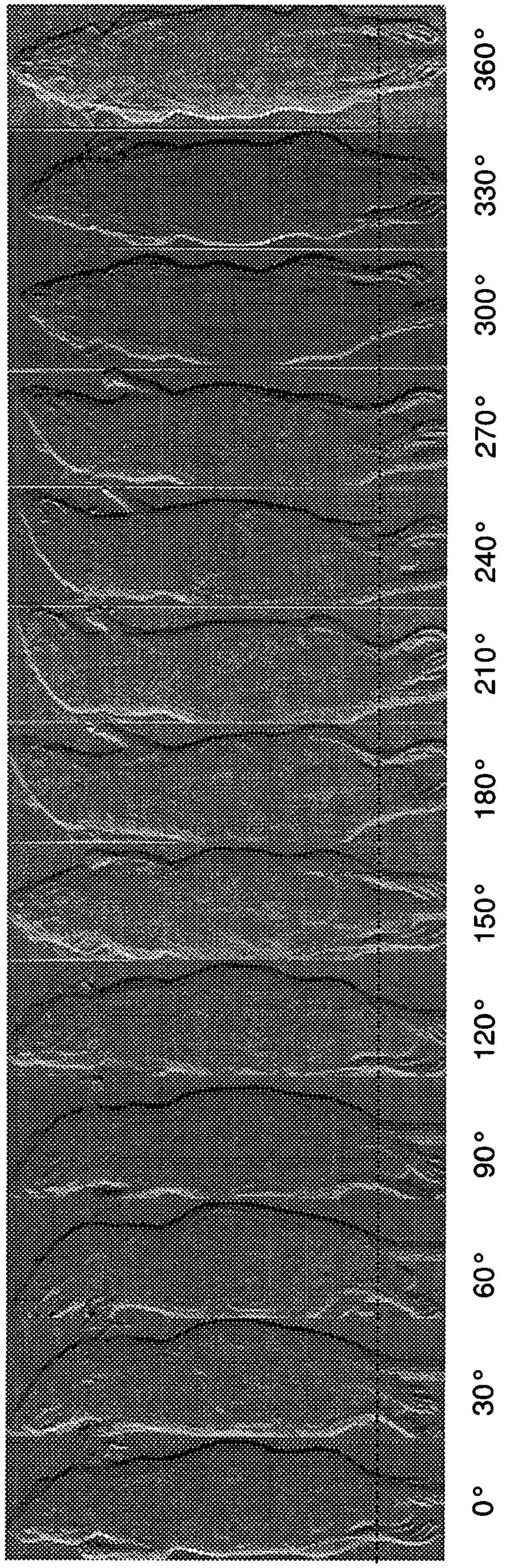


FIG. 38B

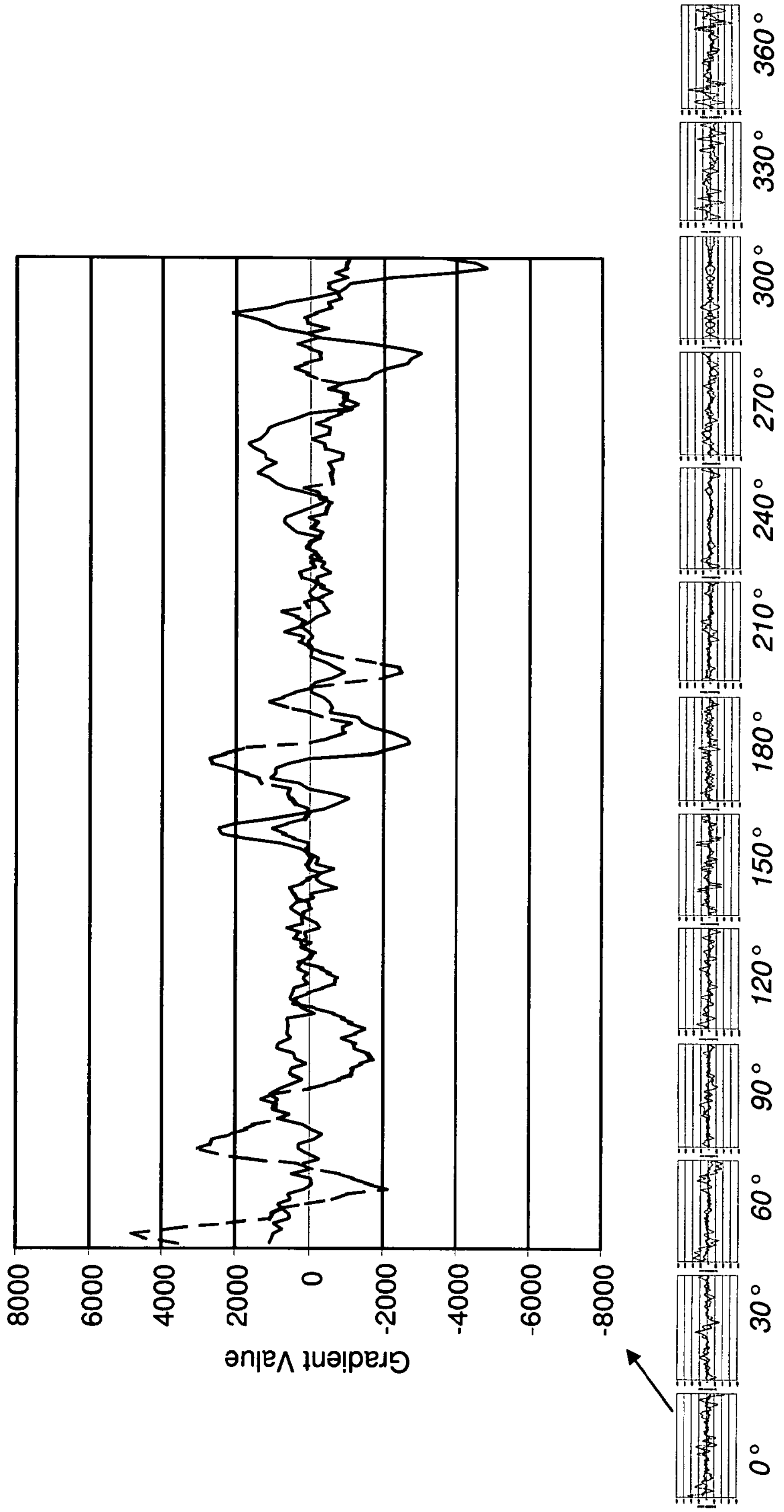
8000
6000
4000
2000
0
-2000
-4000
-6000
-8000
Gradient Value
0°
30°
60°
90°
120°
150°
180°
210°
240°
270°
300°
330°
360°

FIG. 39

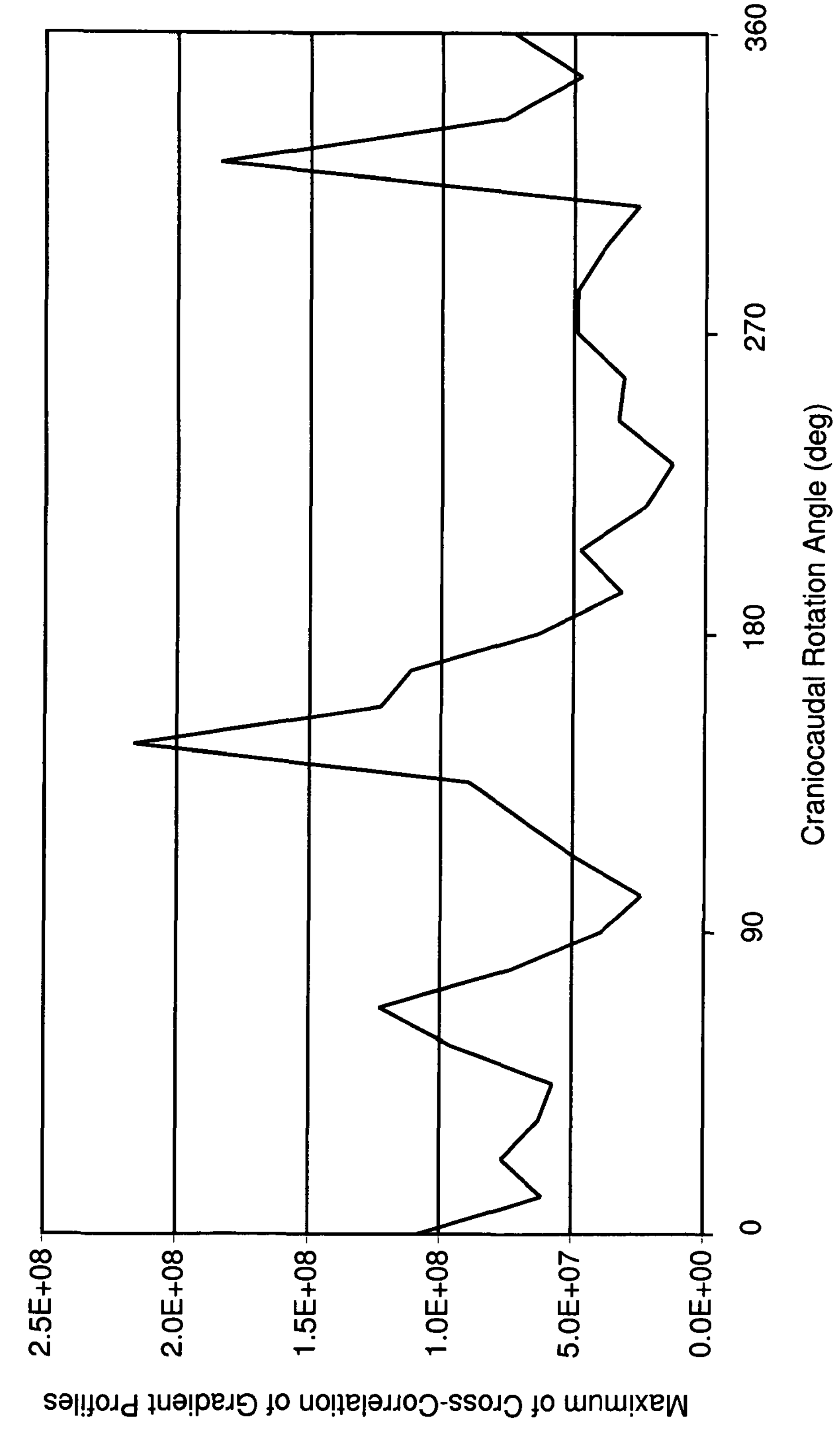
Maximum of Cross-Correlation of Gradient Profiles
2.5E+08
2.0E+08
1.5E+08
1.0E+08
5.0E+07
0.0E+00
0
90
180
270
360
Craniocaudal Rotation Angle (deg)

FIG. 40

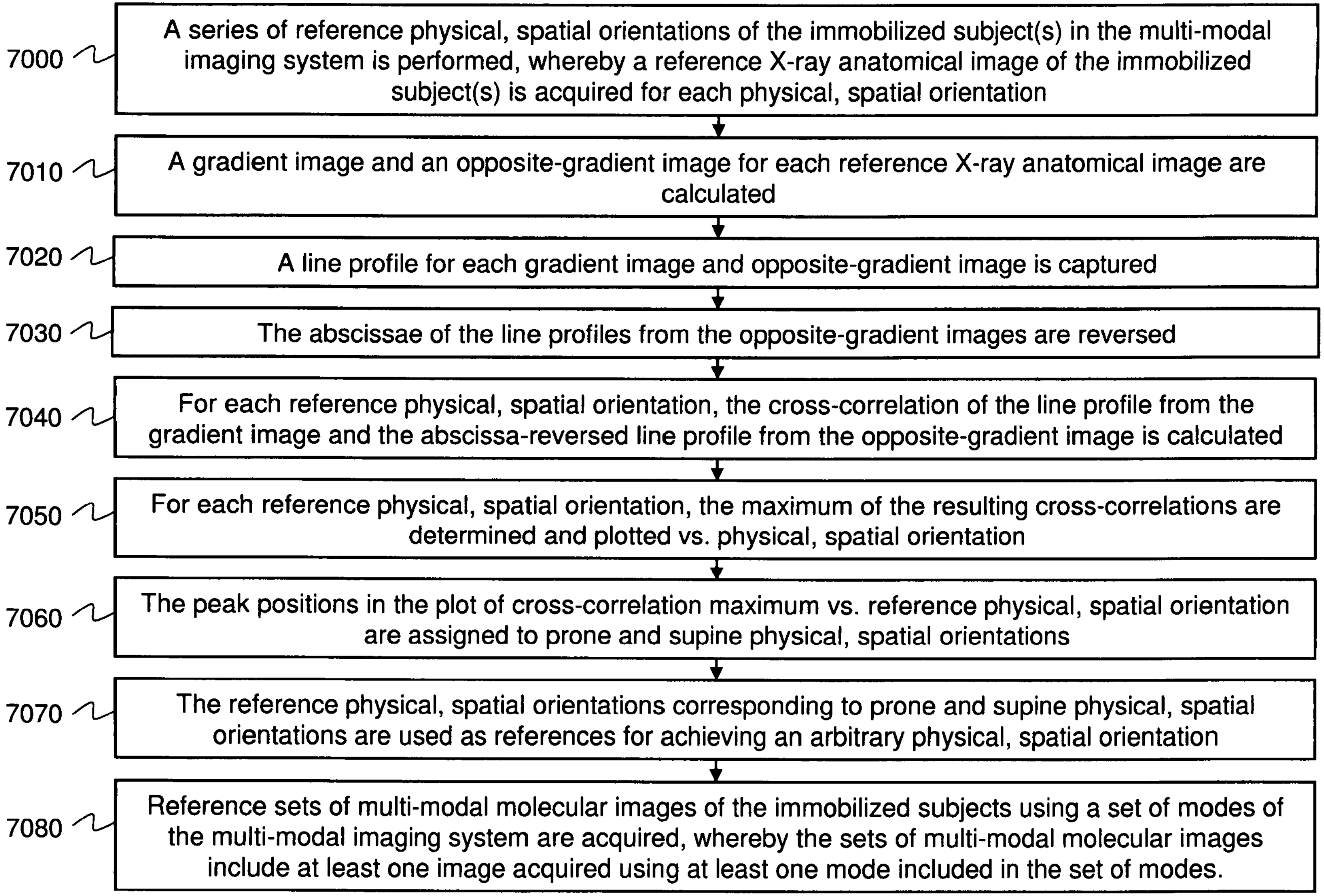


Continued on FIG. 41B

FIG. 41A

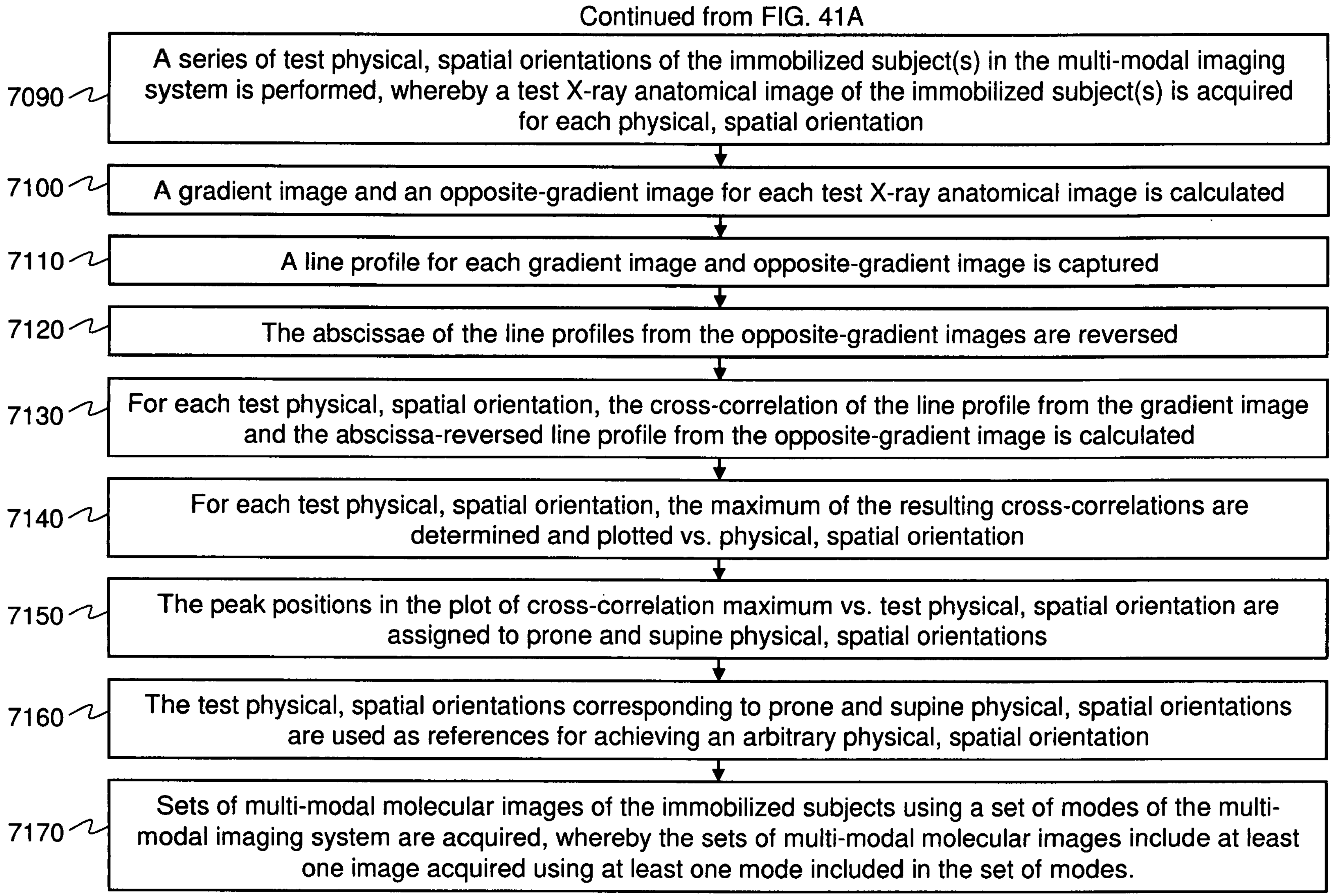
Continued from FIG. 41A
7090
A series of test physical, spatial orientations of the immobilized subject(s) in the multi-modal imaging system is performed, whereby a test X-ray anatomical image of the immobilized subject(s) is acquired for each physical, spatial orientation
7100
A gradient image and an opposite-gradient image for each test X-ray anatomical image is calculated
7110
A line profile for each gradient image and opposite-gradient image is captured
7120
The abscissae of the line profiles from the opposite-gradient images are reversed
7130
For each test physical, spatial orientation, the cross-correlation of the line profile from the gradient image and the abscissa-reversed line profile from the opposite-gradient image is calculated
7140
For each test physical, spatial orientation, the maximum of the resulting cross-correlations are determined and plotted vs. physical, spatial orientation
7150
The peak positions in the plot of cross-correlation maximum vs. test physical, spatial orientation are assigned to prone and supine physical, spatial orientations
7160
The test physical, spatial orientations corresponding to prone and supine physical, spatial orientations are used as references for achieving an arbitrary physical, spatial orientation
7170
Sets of multi-modal molecular images of the immobilized subjects using a set of modes of the multi-modal imaging system are acquired, whereby the sets of multi-modal molecular images include at least one image acquired using at least one mode included in the set of modes.

*FIG. 41B*

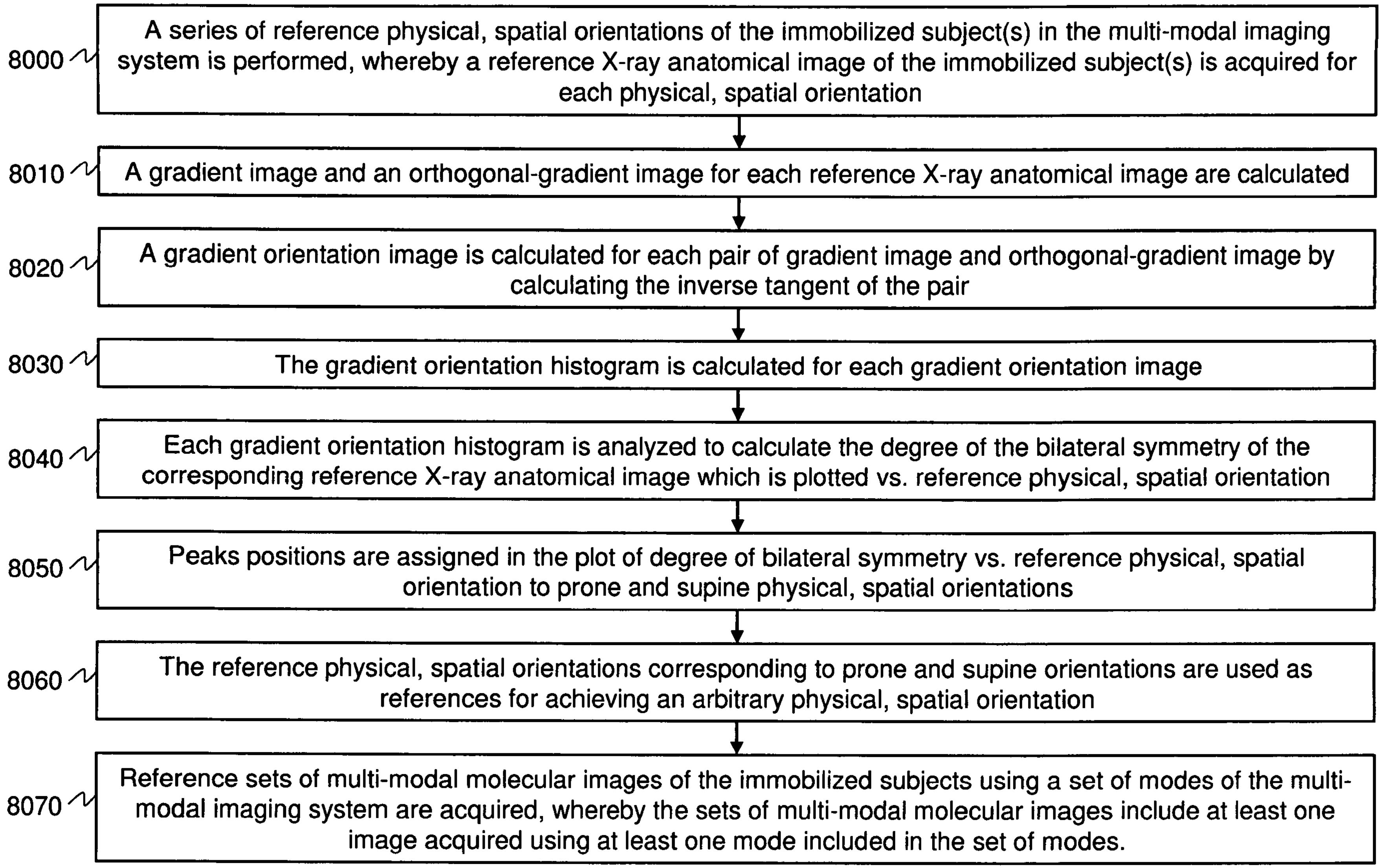


Continued on FIG. 42B

*FIG. 42A*

Continued from FIG. 42A

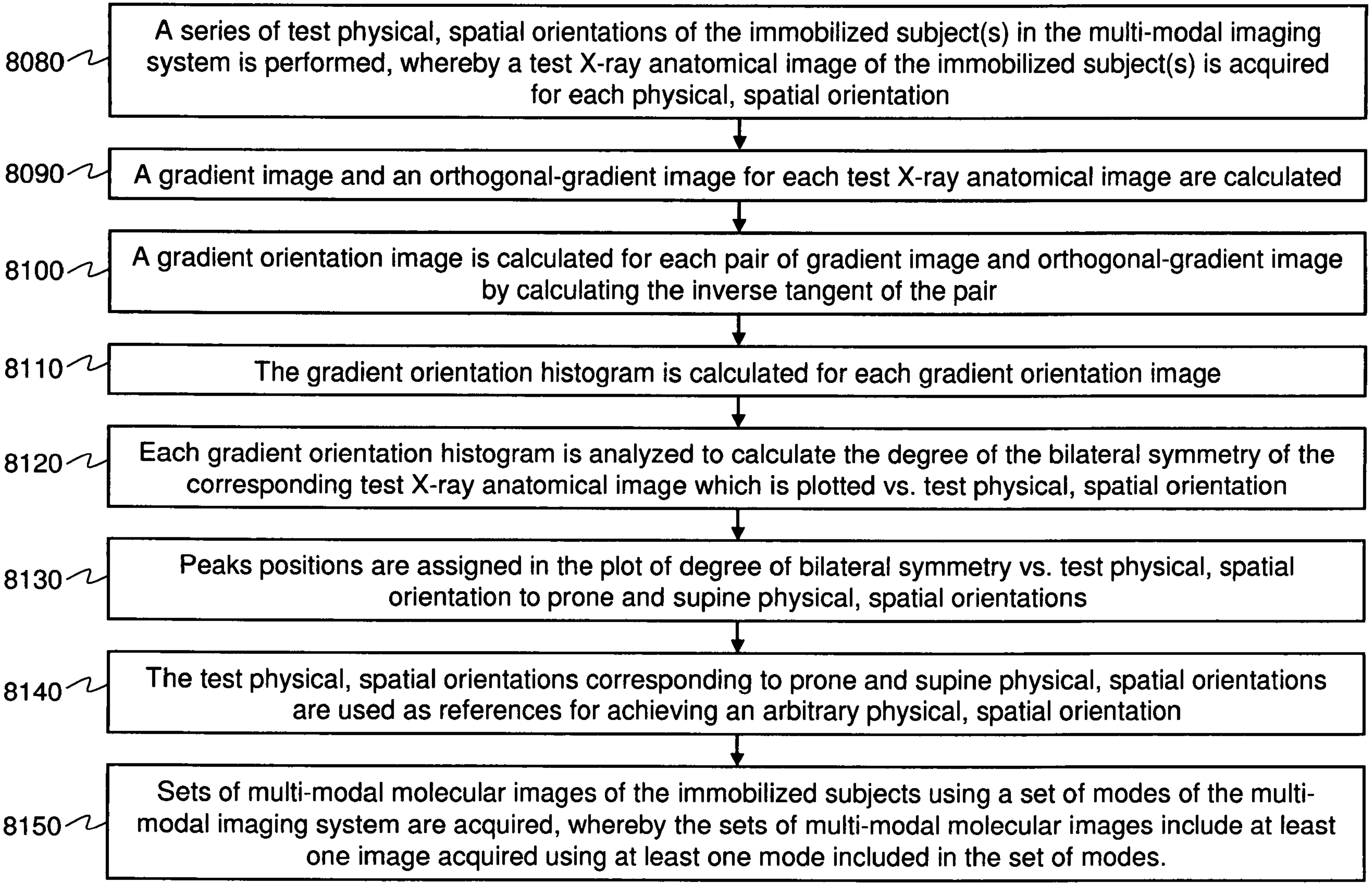


*FIG. 42B*

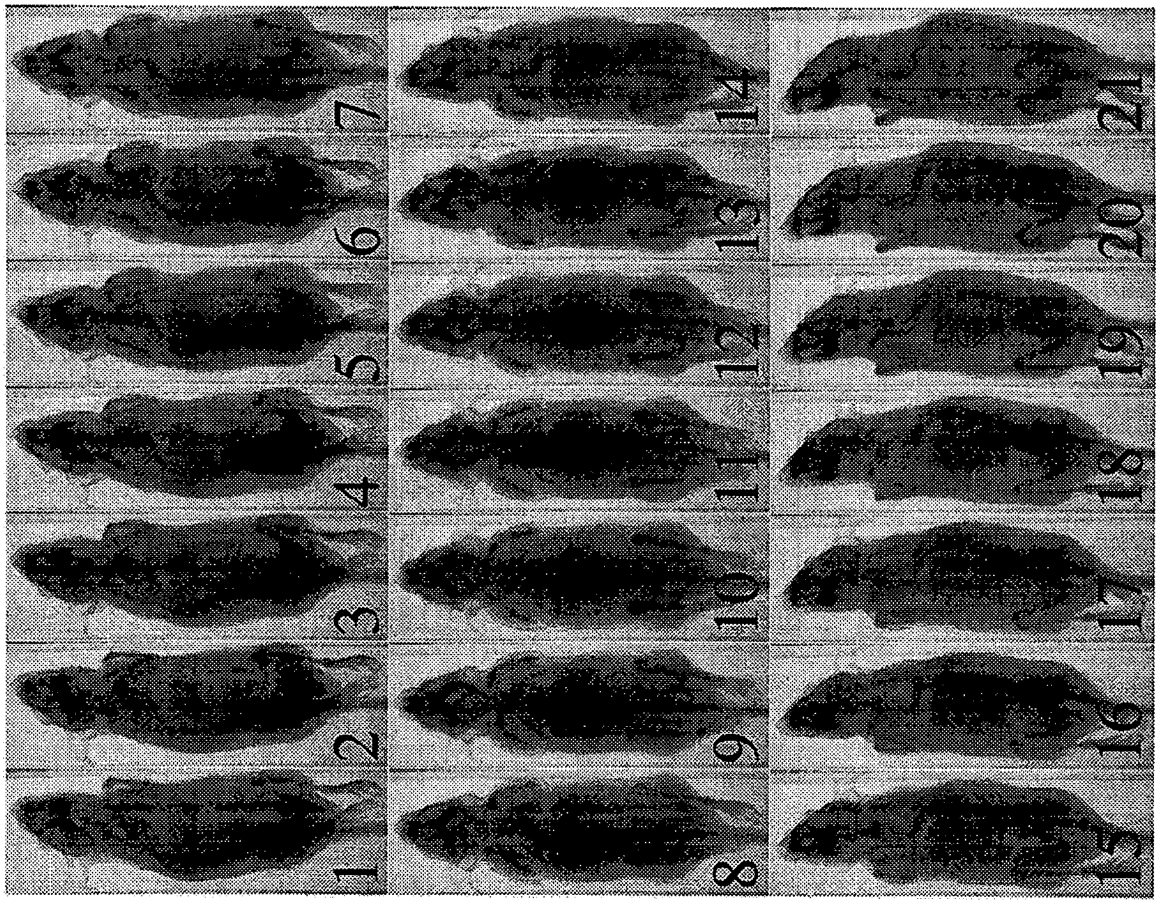

FIG. 43

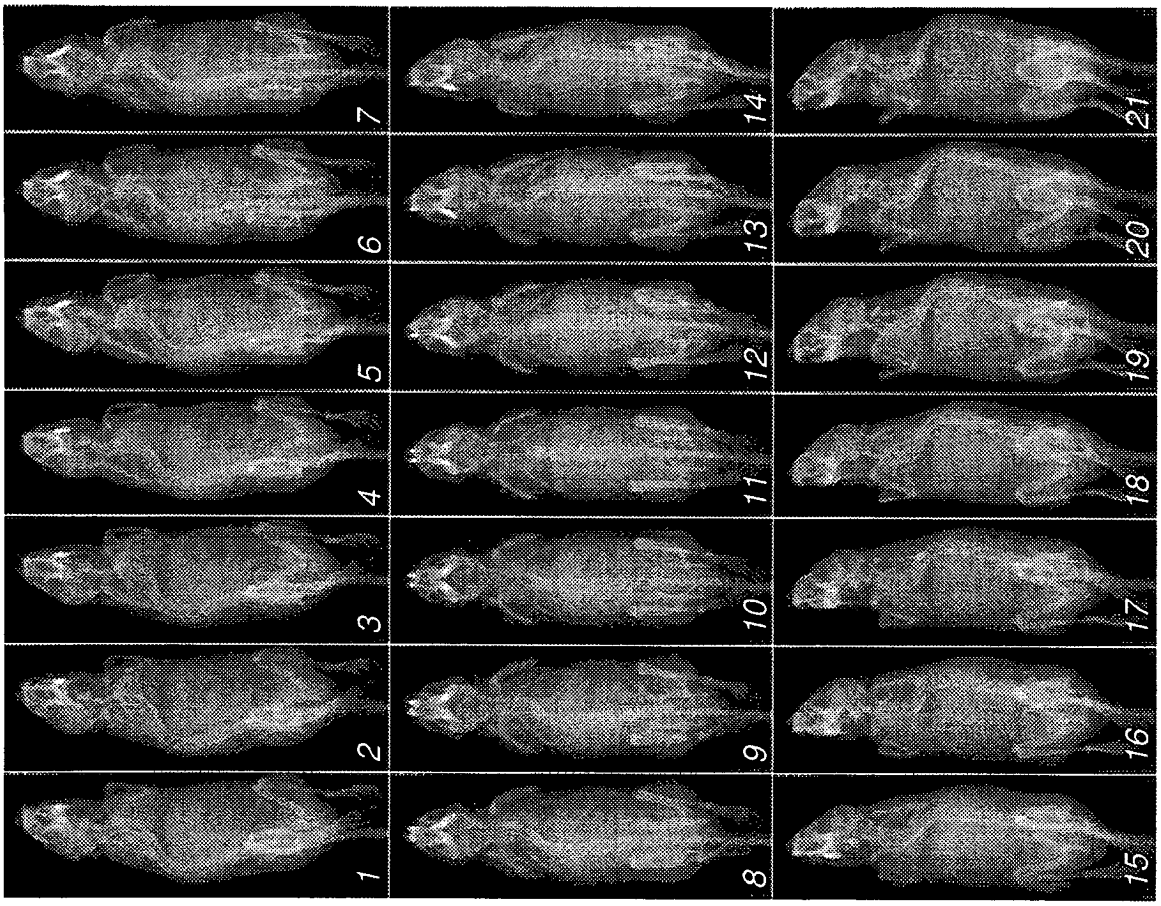


FIG. 44

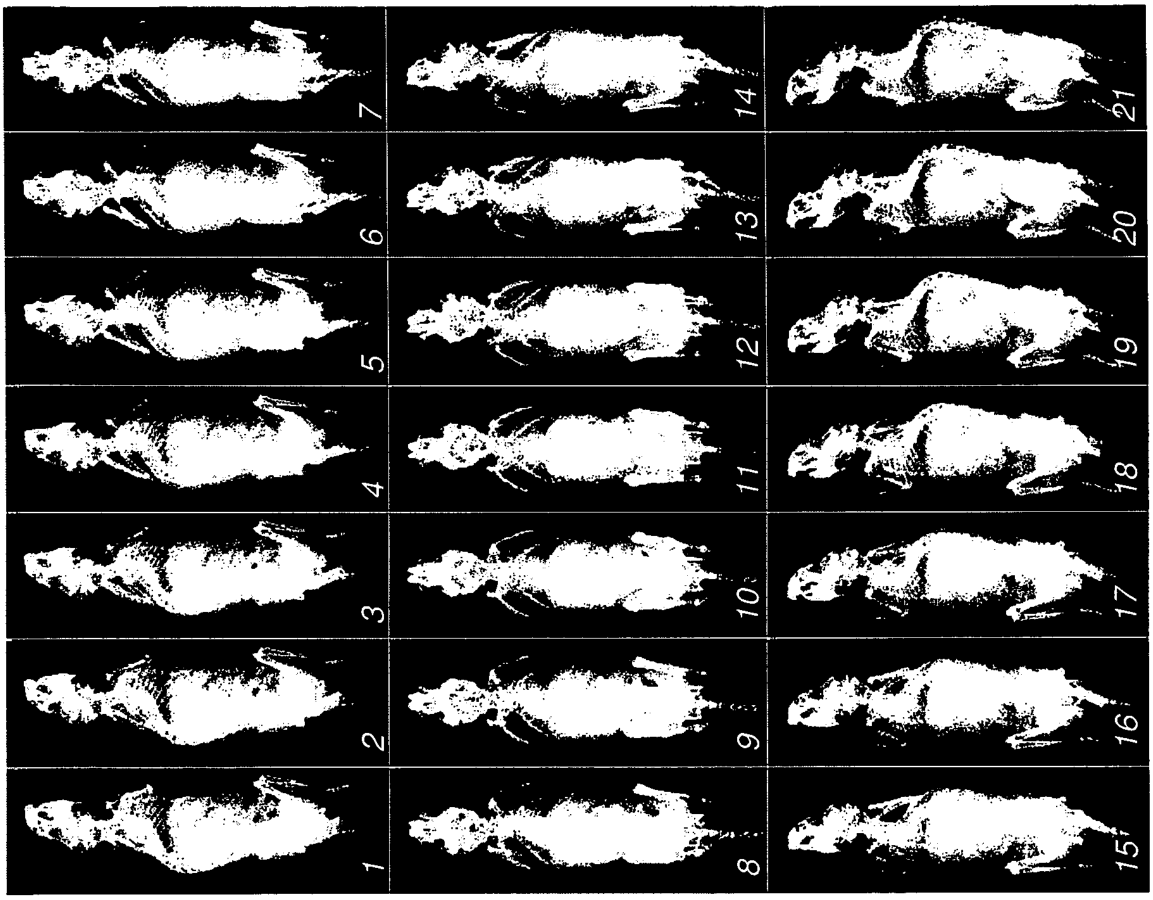


FIG. 45

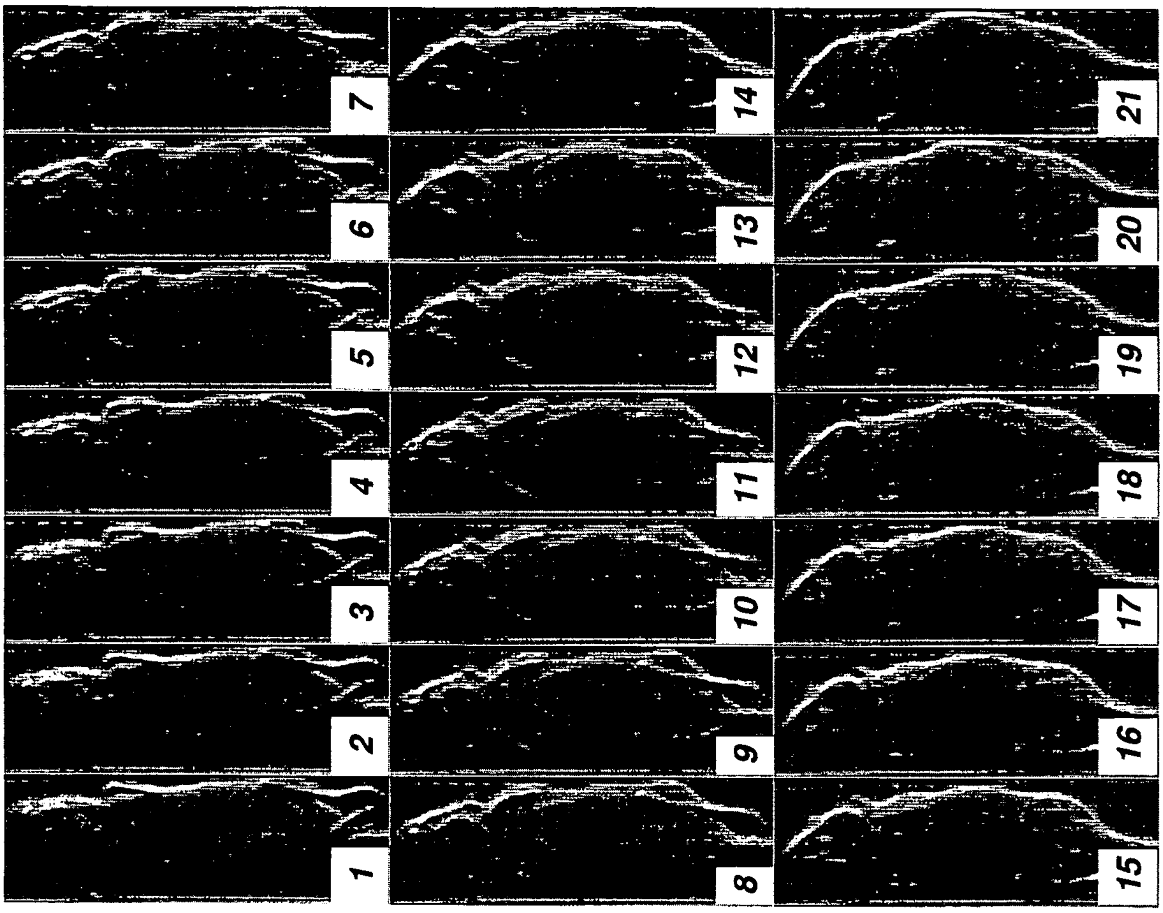


FIG. 46

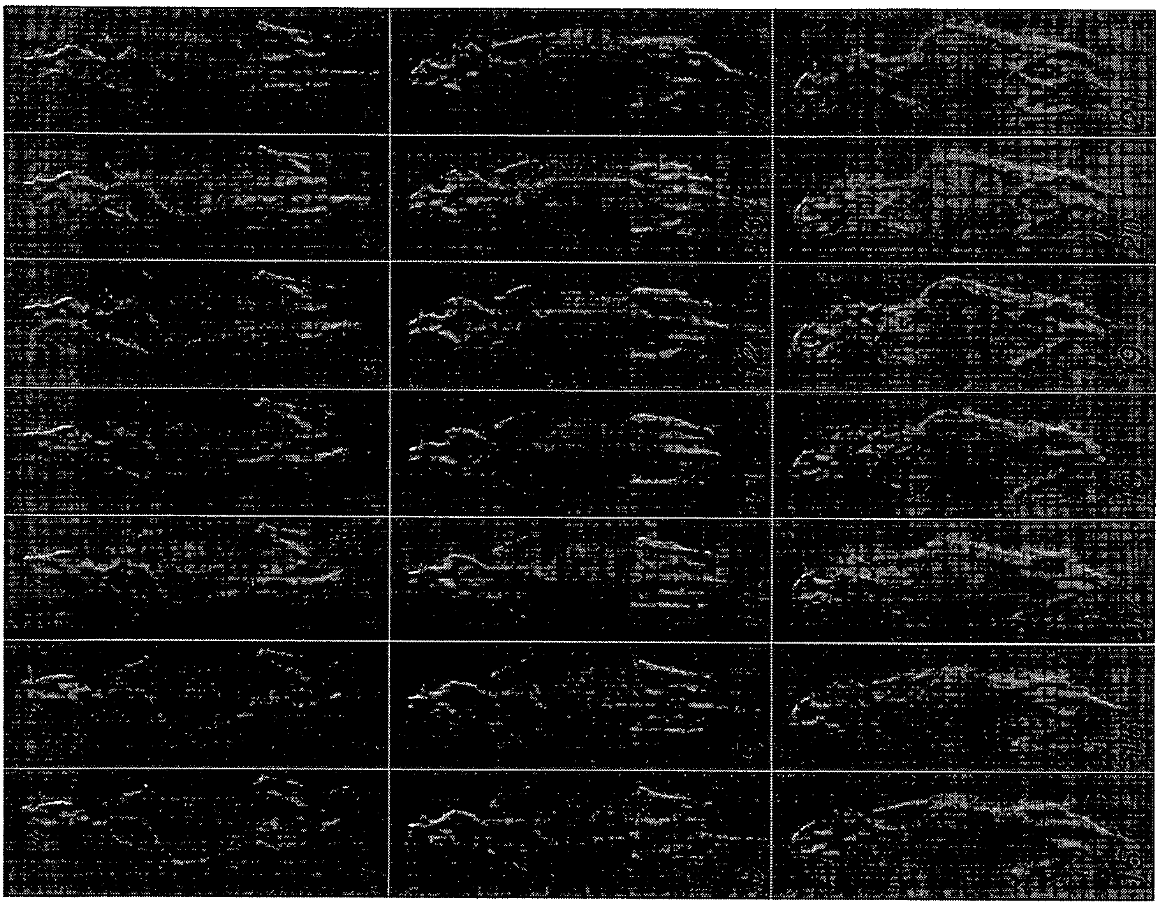

FIG. 47

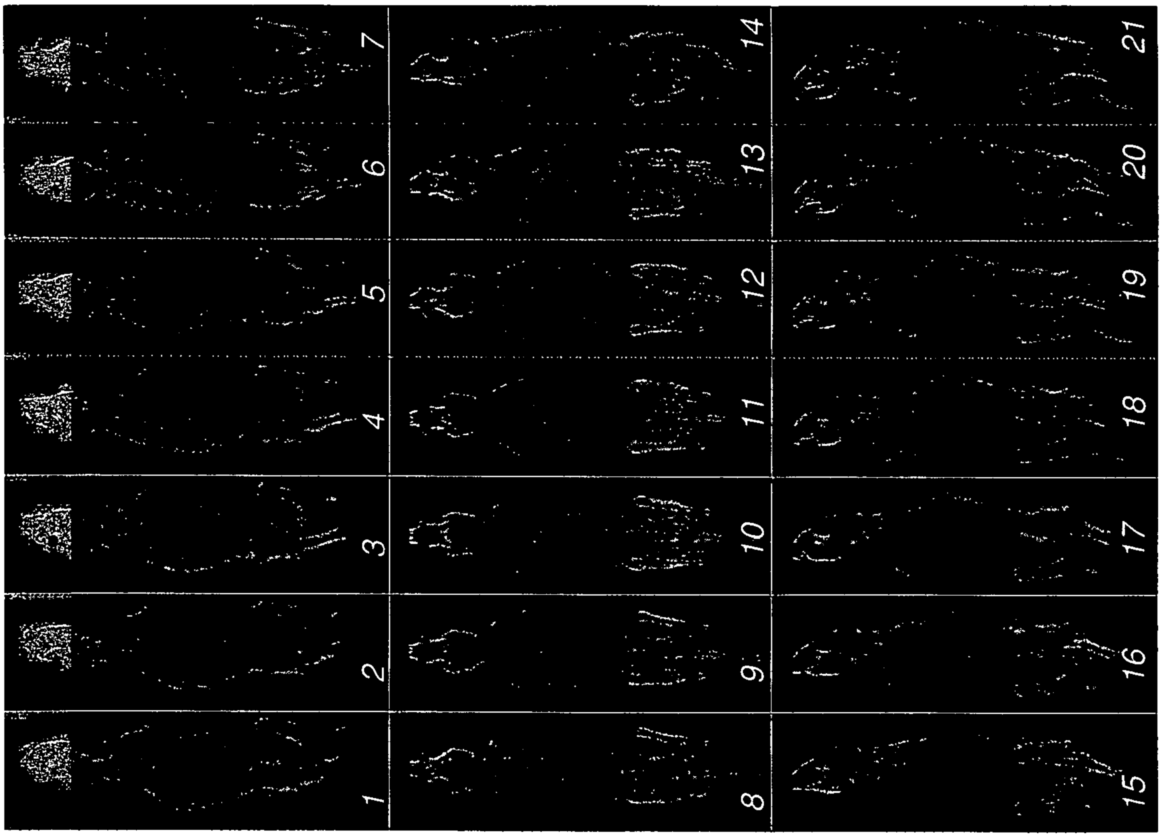


FIG. 48

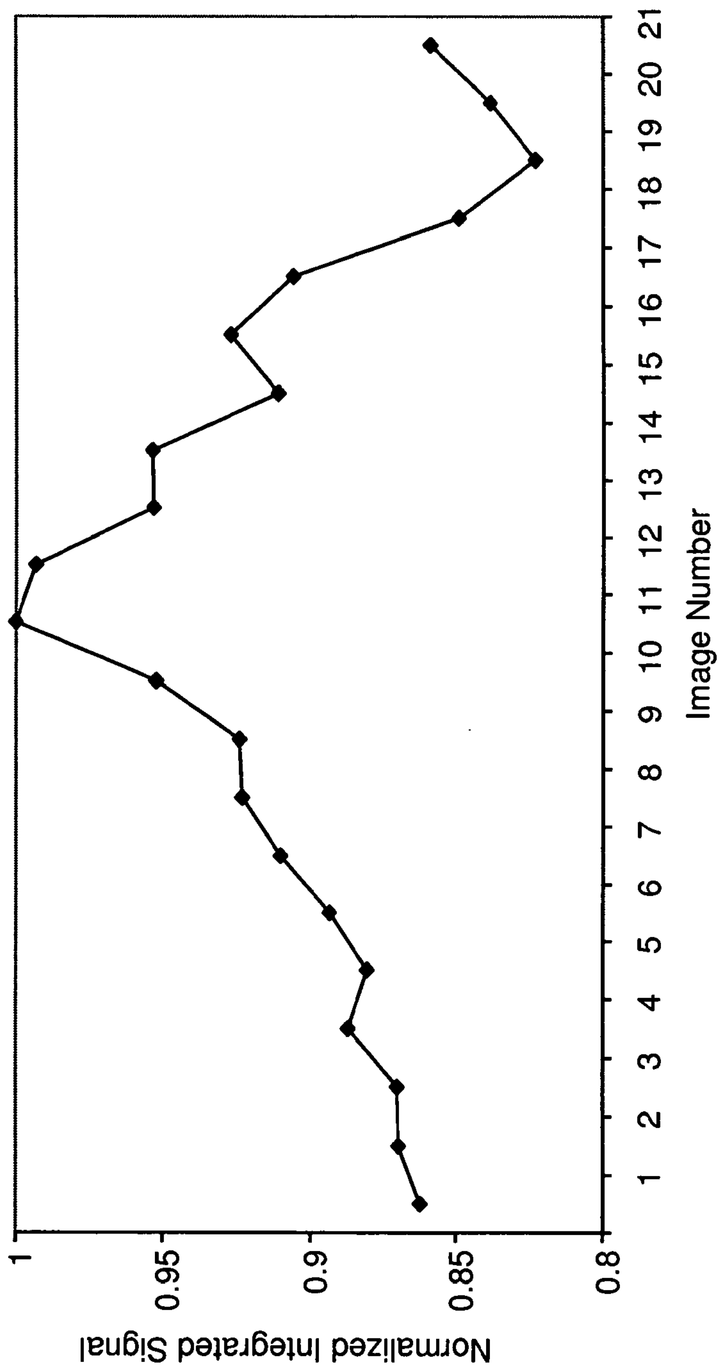
Normalized Integrated Signal
1
0.95
0.9
0.85
0.8
Image Number
1 2 3 4 5 6 7 8 9 10 11 12 13 14 15 16 17 18 19 20 21

FIG. 49

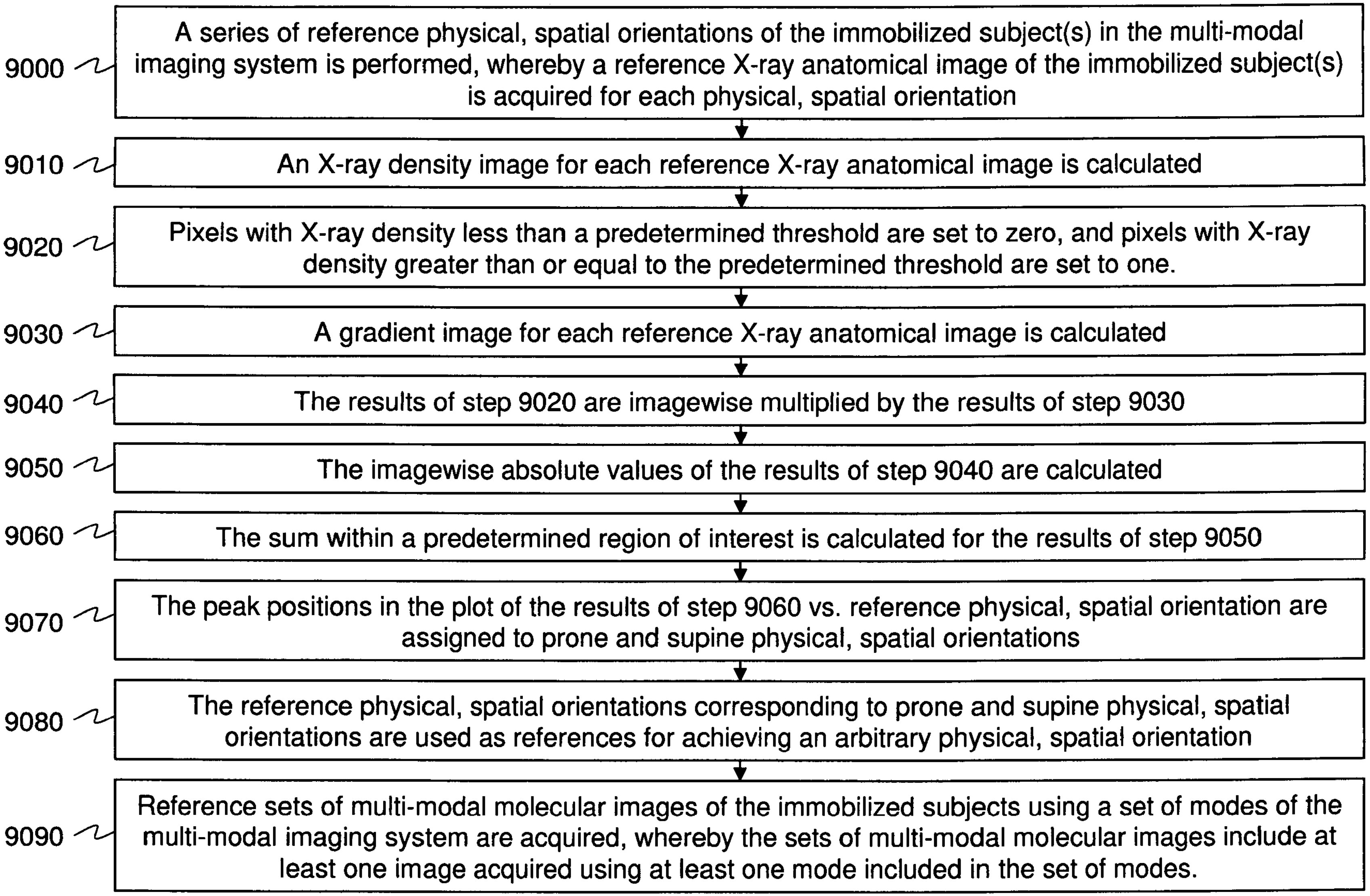


Continued on FIG. 50B

FIG. 50A

Continued from FIG. 50A

9100 A series of test physical, spatial orientations of the immobilized subject(s) in the multi-modal imaging system is performed, whereby a test X-ray anatomical image of the immobilized subject(s) is acquired for each physical, spatial orientation

9110 An X-ray density image for each test X-ray anatomical image is calculated

9120 Pixels with X-ray density less than a predetermined threshold are set to zero, and pixels with X-ray density greater than or equal to the predetermined threshold are set to one.

9130 A gradient image for each test X-ray anatomical image is calculated

9140 The results of step 9120 are imagewise multiplied by the results of step 9130

9150 The imagewise absolute values of the results of step 9140 are calculated

9160 The sum within a predetermined region of interest is calculated for the results of step 9150

9170 The peak positions in the plot of the results of step 9160 vs. test physical, spatial orientation are assigned to prone and supine physical, spatial orientations

9180 The test physical, spatial orientations corresponding to prone and supine physical, spatial orientations are used as references for achieving an arbitrary physical, spatial orientation

9190 Sets of multi-modal molecular images of the immobilized subjects using a set of modes of the multi-modal imaging system are acquired, whereby the sets of multi-modal molecular images include at least one image acquired using at least one mode included in the set of modes.

***FIG. 50B***

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20070238957 A, Yared
- US 6868172 B, Boland
- US 61131948 A, Feke
- US 6444988 B, Vizard
- US 221530 A
- US 12354830 B
- US 7263243 B, Chen


### Non-patent literature cited in the description


- **C. SUN.** Symmetry detection using gradient information. *Pattern Recognition Letters,* 1995, vol. 16, 987-996
- **C. SUN ; D. SI.** Fast Reflectional Symmetry Detection Using Orientation Histograms. *Real-Time Imaging,* 1999, vol. 5, 63-74
- Optimal Detection of Symmetry Axis in Digital Chest X-ray Images. IbPRIA 2003. LNCS, 2003, vol. 2652, 1082-1089